# EUROPEAN PATENT SPECIFICATION

(11) **EP 1 709 063 B1**
(45) Date of publication and mention of the grant of the patent: **23.01.2008**
(21) Application number: 04820440.8
(22) Date of filing: 17.12.2004
(51) Int. Cl.: C07J 7/00, A61K 31/57, A61P 35/00

(54) **GLYCOSYLATED STEROID DERIVATIVES WITH ANTI-MIGRATORY ACTIVITY**
GLYCOSYLIERTE STEROIDDERIVATE MIT ANTIMIGRATORISCHER WIRKUNG
DERIVES STEROIDES GLYCOSYLES A ACTIVITE ANTI-MIGRATOIRE

(30) Priority: 18.12.2003 WO PCT/EP03/14567; 16.06.2004 WO PCT/EP2004/006486
(43) Date of publication of application: 11.10.2006
(73) Proprietor: Unibioscreen S.A., 1070 Bruxelles (BE); UNIVERSITE LIBRE DE BRUXELLES, 1050 Bruxelles (BE)
(72) Inventor: BRAEKMAN, Jean-Claude, B-1640 Rhôde-St-Genèse (BE); INGRASSIA, Laurent, B-1420 Brain L´Alleud (BE); NSHIMYUMUKIZA, Prosper, B-1731 Zellik (BE); VAN QUAQUEBEKE, Eric, B-1200 Woluwe St-Lambert (BE); DE WELLE, Janique, B-6230 Buzet (BE); VAN DEN HOVE, Laurent, B-1190 Forest (BE); KISS, Robert, B-1600 Sint-Pieters-Leeuw (BE); DARRO, Francis, B-1180 Uccle (BE)
(74) Representative: Brants, Johan P.E.
(86) International application number: PCT/EP2004/014408
(87) International publication number: WO 2005/058934

(56) References cited:
- PATENT ABSTRACTS OF JAPAN vol. 2000, no. 16, 8 May 2001 (2001-05-08) & JP 2001 002698 A (KANEGAFUCHI CHEM IND CO LTD), 9 January 2001 (2001-01-09)
- PATENT ABSTRACTS OF JAPAN vol. 1997, no. 06, 30 June 1997 (1997-06-30) & JP 09 040690 A (SASHITA YUTAKA; OKA KITAROU; POLA CHEM IND INC), 10 February 1997 (1997-02-10)

## Description

### Field of the invention

The present invention relates to the medical field. In a first aspect, the present invention relates to novel glycosylated steroid compounds having a pharmacological activity, in particular an anti-migratory activity. In a second aspect, the present invention relates to a method for the preparation of said glycosylated steroid derivatives. The invention further relates in a third aspect to a pharmaceutical composition comprising an effective amount of said glycosylated steroid compounds. In a fourth aspect, the present invention concerns the use of said glycosylated steroid derivatives as a medicament and the use of said glycosylated steroid compounds for the preparation of a medicament for the treatment of diseases associated with cell migration, and even in particular in the treatment of cancer. In a fifth aspect, the present invention relates to the use of a glycosylated steroid compound or a pharmaceutical composition comprising said glycosylated steroid compounds according to the invention in the treatment of diseases associated with cell migration, and even in particular in the treatment of cancer.

### Background of the invention

Cancer develops in a given tissue when some genomic mutation perturbs cell cycle kinetics by increasing cell proliferation or decreasing cell death, or both. This perturbation leads to unrestrained growth of a genomically transformed cell population. Some cells from this transformed cell population may switch to the angiogenic phenotype, enabling them to recruit endothelial cells from the healthy tissue and leading to the sustained growth of the developing neoplastic tumor tissue. Subsequently, some cells migrate from the neoplastic tumor tissue and colonize new tissues, using blood or lymphatic vessels as major routes of migration. This process is also known as the metastatic process.

In practice, most of the agents used today in hospitals to treat cancer patients are drugs, which more or less directly target the cell kinetics, i.e. cell proliferation, of the cancer to be combated. The working mechanism of such anti-cancer drugs essentially relates to the disruption of the development of malignant cells by acting on cell kinetics. These drugs include alkylating agents, intercalating agents, antimetabolites, etc... most of which target DNA or enzymes regulating the DNA duplication and elongation process. These drugs attack the DNA.

A major drawback of these drugs involves that the drugs do not work in a selective manner, i.e. they do not select between normal and neoplastic cells. They are used in accordance with the fact that the DNA of rapidly proliferating cells, i.e, cancer cells, is more sensitive to this type of agents than the DNA of less rapidly proliferating cells, i.e. normal cells. However, rapidly growing tumors are not always tumors exhibiting high levels of cell proliferation. Rapidly growing tumors may also include tumors which exhibit low levels of cell death compared to the normal cell population from which these tumor cells issue. For these types of rapidly growing tumors, the above-described, non-selective anti-cancer drugs are not effective.

In addition, the great majority of the drugs used in the standard treatment of cancer using the cell kinetics approach have the drawback of being toxic or even highly toxic, i.e. involving many detrimental side-effects on healthy cells, tissues and organs, and this limits their clinical use to a relatively low number of administrations per patient. In addition, several of these compounds must be combined into a poly-chemotherapeutic regimen in order to have any observable effect against cancer. By way of evidence such anti-cancer drug combinations increase detrimentally the toxicity of the treatment and also limit the number of administrations that can be applied.

Some anti-cancer drugs from natural origins, such as e.g. anti-tubulin compounds, using a therapeutic approach different from the cell kinetics approach, have been proposed. Said drugs aim to prevent the migration of cancer cells which escape from the primary tumor tissue and first invade neighbouring tissue therefore establishing metastases. However, the compounds of this type known so far also show major toxic side effects, which limits their use over long periods of treatment.

JP 2001002698 discloses an anticancer compound extracted from a plant of the Liliaceae family or its allied species. JP09040690 discloses steroid glycosides, extracted from a plant of the family Liliacea Onityhogalum saundersiae, having anticancer and immunosuppressing actions.

Therefore, there remains an urgent need in the art for finding improved anti-cancer drugs, which overcome at least some of the above-mentioned drawbacks. Consequently, it is a general object of the invention to provide improved anti-cancer drugs. More in particular, it is an object of the present invention to provide novel anti-cancer drugs and methods for synthesizing these. It is still another object of the invention to provide intermediate compounds as a result of the aforementioned synthesis methods, which have a pharmaceutical utility, e.g. in the treatment of cancer.

### Summary of the invention

In a first aspect the present invention relates to glycosylated steroid derivatives of the formula I, stereoisomers, tautomers, racemics, prodrugs, metabolites thereof, or a pharmaceutically acceptable salt and/or solvate thereof, as defined in claim 1

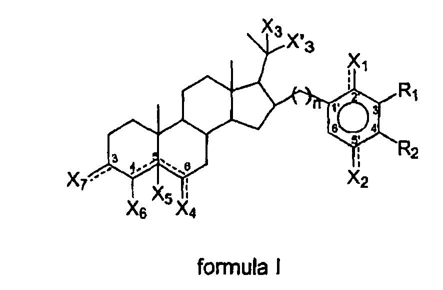

In an embodiment, the present invention relates more in particular to glycosylated steroid derivatives of the formula I, stereoisomers, tautomers, racemics, prodrugs, metabolites thereof, or a pharmaceutically acceptable salt and/or solvate thereof, as defined in claims 2 to 12.

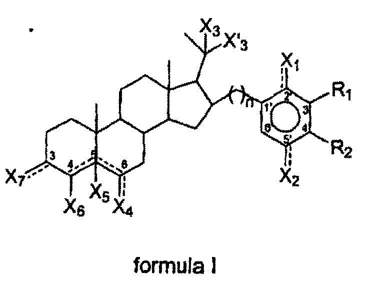

The present invention provides novel glycosylated steroid compounds that have anti-migratory activity and that are consequently very suitable for use in all kind of therapeutic applications as described below.

In a second aspect, the present invention relates to a method for synthesizing said glycosylated steroid compounds.

In addition, the present invention further relates to pharmaceutical compositions comprising the above-described compounds. Furthermore, the present invention relates to glycosylated steroid compounds for use as a medicament and for use in the preparation of a medicament for the treatment of diseases associated with cell proliferation and cell migration, in particular for treatment of cancer. The present invention further relates to the use of the above-described compounds or a pharmaceutical composition comprising said compounds in the treatment of cancer.

### Detailed description of the invention

### Glycosylated steroid compounds according to the invention

A lot of steroid compounds are described in the literature. These compounds have various biological activities. For example, WO 96/10031 and WO 98/14194 describe steroid derivatives as neurochemical stimulators of a specific neuroepithelial receptor to alleviate symptoms of anxiety.

The present invention now relates to novel glycosylated steroid compounds showing an anti-migratory activity. Migration refers to the process whereby cells migrate from the neoplastic tumor tissue and colonize new tissues, using blood or lymphatic vessels as major routes of migration. This process is also known as the metastatic process. According to the present invention the term "anti-migratory", refers to the ability of compounds according to the invention to stop the migration of cells away from the neoplastic tumor tissue and thus reduces the colonization of new tissues by these cells.

The term "steroid" as used herein is intended to mean compounds and their stereochemically isomeric forms having a perhydrogenated cyclopentanophenanthrene nucleus. The compounds according to the invention, represented by the general formula given below, have four rings, represented by the letters A to D.

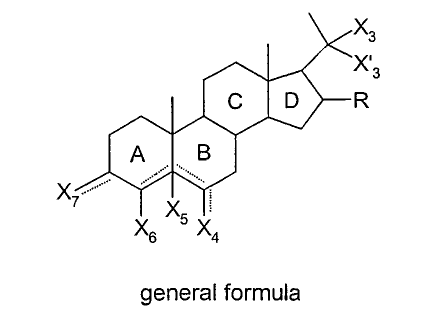

The terms "stereochemically isomeric forms" or "stereoisomeric forms", as used herein, defines all possible compounds made up of the same atoms bonded by the same sequence of bonds but having different three-dimensional structures which are not interchangeable, which the compounds of the present invention may possess. Unless otherwise mentioned or indicated, the chemical designation of a compound herein encompasses the mixture of all possible stereochemically isomeric forms, which said compound may possess. Said mixture may contain all diastereomers and/or enantiomers of the basic molecular structure of said compound. All stereochemically isomeric forms of the compounds of the invention either in pure form or in admixture with each other are intended to fall within the scope of the present invention.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

As used herein the term "glycosylated" or "glycosyl" refers a saccharyl moiety such as a mono-, di-, oligo- or an poly-saccharide moiety, a hydroxy-substituted cyclohexyl moiety, the amino derivatives thereof, the amido derivatives thereof, the thio derivatives thereof, the hydroxyl-protected derivatives thereof such as acetate or benzoyl derivatives thereof, or the carboxy derivatives thereof, and can be optionally substituted by one or more substituents. The term "glycosyl" as used herein encompasses stereoisomers, optical isomers, anomers, and epimers of said glycosyl moiety. Thus, a hexose moeity for example can be either an aldose or a ketose moiety, and can be of D- or L-configuration, can assume either an α or β conformation, and can be a dextro- or levo-rotatory with respect to plane-polarized light.

The term "saccharyl" as used herein refers to a saccharide moiety, which comprises monosaccharides, di-, tri-, oligo- and polysaccharides. Exemplary monosaccharide moiety includes but is not limited to a pentosyl, a hexosyl, or a heptosyl moiety. The glycosyl moiety may also be substituted with various groups. Such substitutions may include lower alkyl, lower alkoxy, acyl, carboxy, carboxyamino, amino, acetamido, halo, thio, nitro, keto, and phosphatyl groups, wherein the substitution may be at one or more positions on the saccharide. Moreover, the glycosyl may also be present as a deoxy glycosyl. The hydroxy-substituted cyclohexyl moiety includes but is not limited to mono-hydroxycyclohexyl group such as 2-, 3- or 4-hydroxycyclohexyl group, a dihydroxycyclohexyl group such as 2,3-, 2,4-, 2,5-, 2,6-, 3,4-, or 3,5 -dihydroxycyclohexyl) group, a tri-hydroxycyclohexyl group such as 2,3,4-, 2,3,5-, 2,3,6-, 3,4,5-, or 3,4,6-trihydroxycyclohexyl group or a tetra-hydroxycyclohexyl group such as 2,3,4,5-, 2,3,4,6-, or 2,3,5,6-tetrahydroxycyclohexyl group, hydroxyl-protected derivatives thereof, thio derivatives thereof, amido derivatives thereof or amino derivatives thereof.

In an embodiment, said glycosyl is a saccharyl moiety, a hydroxy-substituted cyclohexyl moiety, including monosaccharide, L or D isomers thereof, α or β form thereof, pyranuronic or furanuronic form thereof, pyranose or furanose form thereof, combination thereof, deoxy derivatives thereof, carboxy derivatives thereof, hydroxy protected derivatives thereof such as hydroxyl-protected acetate or benzoyl derivatives thereof, amino derivatives thereof optionally substituted, amido derivatives thereof, thio derivatives thereof, di-, tri-, oligo- and polysaccharide thereof optionally substituted by one or more substituents.

As used herein, the term "halo" or "halogen" as a group or part of a group is generic for fluoro, chloro, bromo or iodo.

The term "alkyl", alone or in combination, means straight and branched chained saturated hydrocarbon radicals containing from 1 to 10 carbon atoms, preferably from 1 to 8 carbon atoms, more preferably 1 to 6 carbon atoms. Examples of such radicals include methyl, ethyl, n-propyl, isopropyl, n-butyl, isobutyl, sec-butyl, tert-butyl, 2-methylbutyl, pentyl, isoamyl, hexyl, 3-methylpentyl, octyl and the like.

The term "alkenyl", alone or in combination, defines straight and branched chained hydrocarbon radicals containing from 2 to about 18 carbon atoms, preferably from 2 to 8 carbon atoms, more preferably 2-6 carbon atoms containing at least one double bond such as, for example, ethenyl, propenyl, butenyl, pentenyl, hexenyl and the like.

The term "alkynyl", alone or in combination, defines straight and branched chained hydrocarbon radicals having from 2 to 10 carbon atoms containing at least one triple bond, more preferably from 2 to about 6 carbon atoms. Examples of alkynyl radicals include ethynyl, propynyl, (propargyl), butynyl, pentynyl, hexynyl and the like.

The term "cycloalkyl" alone or in combination, means a saturated or partially saturated monocyclic, bicyclic or polycyclic alkyl radical wherein each cyclic moiety contains from about 3 to about 8 carbon atoms, more preferably from about 3 to about 7 carbon atoms. Examples of monocyclic cycloalkyl radicals include cyclopropyl, cyclobutyl, cyclopentyl, cyclohexyl, cycloheptyl, cyclooctyl and the like. Examples of polycyclic cycloalkyl radicals include decahydronaphthyl, bicyclo [5.4.0] undecyl, adamantyl, and the like.

The term "cycloalkylalkyl" means an alkyl radical as defined herein, in which at least one hydrogen atom on the alkyl radical is replaced by a cycloalkyl radical as defined herein. Examples of such cycloalkylalkyl radicals include cyclopropylmethyl, cyclobutylmethyl, cyclopentylmethyl, cyclohexylmethyl, 1-cyclopentylethyl, 1-cyclohexylethyl, 2-cyclopentylethyl, 2-cyclohexylethyl, cyclobutylpropyl, cyclopentylpropyl, 3-cyclopentylbutyl, cyclohexylbutyl and the like.

The term "aryl" alone or in combination, is meant to include phenyl and naphtyl which both may be optionally substituted with one or more substituents independently selected from alkyl, alkoxy, halogen, hydroxy, amino, nitro, cyano, haloalkyl, carboxy, alkoxycarbonyl, cycloalkyl, Het¹, amido, optionally mono- or disubstituted aminocarbonyl, methylthio, methylsulfonyl, and phenyl optionally substituted with one or more substituents selected from C₁₋₆alkyl, C₁₋₆alkyloxy, halogen, hydroxy, optionally mono- or disubstituted amino, nitro, cyano, haloC₁₋₆alkyl, carboxyl, C₁₋₆alkoxycarbonyl, C₃₋₇cycloalkyl, Het¹, optionally mono- or disubstituted aminocarbonyl, methylthio and methylsulfonyl; whereby the optional substituents on any amino function are independently selected from alkyl, alkyloxy, Het¹, Het¹alkyl, Het¹alkyl, Het¹oxy, Het¹oxyalkyl, phenyl, phenyloxy, phenyloxyalkyl, phenylalkyl, alkyloxycarbonylamino, amino, and aminoalkyl whereby each of the amino groups may optionally be mono- or where possible di-substituted with alkyl. Examples of aryl includes phenyl, p-tolyl, 4-methoxyphenyl, 4-(tert-butoxy)phenyl, 3-methyl-4-methoxyphenyl, 4-fluorophenyl, 4-chlorophenyl, 3-nitrophenyl, 3-aminophenyl, 3-acetamidophenyl, 4-acetamidophenyl, 2-methyl-3-acetamidophenyl, 2-methyl-3-aminophenyl, 3-methyl-4-aminophenyl, 2-amino-3-methylphenyl, 2,4-dimethyl-3-aminophenyl, 4-hydroxyphenyl, 3-methyl-4-hydroxyphenyl, 1-naphthyl, 2-naphthyl, 3-amino-1-naphthyl, 2-methyl-3-amino-1-naphthyl, 6-amino-2-naphthyl, 4,6-dimethoxy-2-naphthyl and the like.

The term "aralkyl" alone or in combination, means an alkyl as defined herein, wherein an alkyl hydrogen atom is replaced by an aryl as defined herein. Examples of aralkyl radicals include benzyl, phenethyl, dibenzylmethyl, methylphenylmethyl, 3- (2-naphthyl)-butyl, and the like.

As used herein, the term "oxo" or "=O" forms a carbonyl moiety with the carbon atom to which it is attached. As used herein, the term "carboxyl" or "-COOH" is an acid moiety whereby the carbon atom binds to the carbon atom to which it is attached.

The term "haloalkyl" alone or in combination, means an alkyl radical having the meaning as defined above wherein one or more hydrogens are replaced with a halogen, preferably, chloro or fluoro atoms, more preferably fluoro atoms. Examples of such haloalkyl radicals include chloromethyl, 1-bromoethyl, fluoromethyl, difluoromethyl, trifluoromethyl, 1,1,1-trifluoroethyl and the like.

The term "Het¹" alone or in combination, is defined as a saturated or partially unsaturated monocyclic, bicyclic or polycyclic heterocycle having preferably 3 to 12 ring members, more preferably 5 to 10 ring members and more preferably 5 to 6 ring members, which contains one or more heteroatom ring members selected from nitrogen, oxygen or sulfur and which is optionally substituted on one or more carbon atoms by alkyl, alkyloxy, halogen, hydroxy, oxo, optionally mono- or disubstituted amino, nitro, cyano, haloalkyl, carboxyl, alkoxycarbonyl, cycloalkyl, optionally mono- or disubstituted aminocarbonyl, methylthio, methylsulfonyl, aryl and a saturated or partially unsaturated monocyclic, bicyclic or tricyclic heterocycle having 3 to 12 ring members which contains one or more heteroatom ring members selected from nitrogen, oxygen or sulfur and whereby the optional substituents on any amino function are independently selected from alkyl, alkyloxy, Het², Het²alkyl, Het²oxy, Het²oxyalkyl, aryl, aryloxy, aryloxyalkyl, aralkyl, alkyloxycarbonylamino, amino, and aminoalkyl whereby each of the amino groups may optionally be mono- or where possible di-substituted with alkyl.

The term "Het²" as a group or part of a group is defined as an aromatic monocyclic, bicyclic or tricyclic heterocycle having preferably 3 to 12 ring members, more preferably 5 to 10 ring members and more preferably 5 to 6 ring members, which contains one or more heteroatom ring members selected from nitrogen, oxygen or sulfur and which is optionally substituted on one or more carbon atoms by alkyl, alkyloxy, halogen, hydroxy, optionally mono- or disubstituted amino, nitro, cyano, haloalkyl, carboxyl, alkoxycarbonyl, cycloalkyl, optionally mono- or disubstituted aminocarbonyl, methylthio, methylsulfonyl, aryl, Het¹ and an aromatic monocyclic, bicyclic or tricyclic heterocycle having 3 to 12 ring members; whereby the optional substituents on any amino function are independently selected from alkyl, alkyloxy, Het¹, Het¹alkyl, Het¹oxy, Het¹oxyalkyl, aryl, aryloxy, aryloxyalkyl, aralkyl, alkyloxycarbonylamino, amino, and aminoalkyl whereby each of the amino groups may optionally be mono- or where possible di-substituted with alkyl.

The term "alkoxy" or "alkyloxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert-butoxy, hexanoxy and the like.

The term "arylthioalkoxy" means alkoxy as defined herein, wherein an alkyl hydrogen atom is replaced by an arylthio as defined herein. Examples of (arylthio) alkoxy radicals include 2- (phenylthio)-ethoxy, and the like.

The term "alkanoyl" or "alkylcarbonyl", alone or in combination, means an acyl radical derived from an alkanecarboxylic acid, examples of which include acetyl, propionyl, butyryl, valeryl, 4-methylvaleryl, and the like.

The term "alkylamino" means an alkyl amine radical, wherein the term "alkyl" is defined as above. Examples of alkylamino radicals include methylamino (NHCH₃), ethylamino (NHCH₂CH₃), n-propylamino, isopropylamino, n-butylamino, isobutylamino, sec-butylamino, tert-butylamino, n-hexylamino, and the like.

The term "alkylthio" means an alkyl thioether radical, wherein the term "alkyl" is defined as above. Examples of alkylthio radicals include methylthio (SCH₃), ethylthio (SCH₂CH₃), n-propylthio, isopropylthio, n-butylthio, isobutylthio, sec-butylthio, tert-butylthio, n-hexylthio, and the like.

The term "aminoalkanoyl" means an acyl group derived from an amino-substituted alkylcarboxylic acid wherein the amino group can be a primary, secondary or tertiary amino group containing substituents selected from alkyl, aryl, aralkyl, cycloalkyl, cycloalkylalkyl radicals and the like.

The term "aminocarbonyl" alone or in combination, means an amino-substituted carbonyl (carbamoyl) group wherein the amino group can be a primary, secondary or tertiary amino group containing substituents selected from alkyl, aryl, aralkyl, cycloalkyl, cycloalkylalkyl radicals and the like.

The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydrocinnamoyl, 4-methoxyhydrocinnamoyl, and the like.

The term "aralkoxy" means alkoxy as defined herein, wherein an alkyl hydrogen atom is replaced by an aryl as defined herein. Examples of aralkoxy radicals include 2-phenylethoxy, 2-phenyl-1-propoxy, and the like.

The term "aralkoxycarbonyl", alone or in combination, means a radical of the formula aralkyl-O-C(O)- in which the term "aralkyl" has the significance given above. Examples of an aralkoxycarbonyl radical are benzyloxycarbonyl and 4-methoxyphenylmethoxycarbonyl.

The term "aralkylamino" means alkylamino as defined herein, wherein an alkyl hydrogen atom is replaced by an aryl as defined herein. Examples of aralkylamino radicals include 2-phenethylamino, 4-phenyl-n-butylamino, and the like.

The term "aralkylthio" means alkylthio as defined herein, wherein an alkyl hydrogen atom is replaced by an aryl as defined herein. Examples of aralkylthio radicals include 3-phenyl-2-propylthio, 2- (2-naphthyl)-ethylthio, and the like.

The term "aroyl" means an acyl radical derived from an arylcarboxylic acid, aryl having the meaning given above. Examples of such arylcarboxylic acid radicals include substituted and unsubstituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 6-carboxy-2 naphthoyl, 6-(benzyloxycarbonyl)-2-naphthoyl, 3-benzyloxy-2-naphthoyl, 3-hydroxy-2-naphthoyl, 3-(benzyloxyformamidol-2-naphthoyl, and the like.

The term "arylaminoalkoxy" means alkoxy as defined herein, wherein an alkyl hydrogen atom is replaced by an arylamino as defined herein. Examples of (arylamino) alkoxy radicals include 2- (phenylamino)-ethoxy, 2- (2- naphthylamino)-1-butoxy, and the like.

The term "arylaminoalkyl" means alkyl as defined herein, wherein an alkyl hydrogen atom is replaced by an arylamino as defined herein. Examples of arylaminoalkyl radicals include phenylaminoethyl, 4- (3-methoxyphenylamino)- 1-butyl, and the like.

The term "arylaminoalkylamino" means alkylamino as defined herein, wherein an alkyl hydrogen atom is replaced by an arylamino as defined herein. Examples of (arylamino) alkylamino radicals include 3- (naphthylamino)-propylamino, 4- (phenylamino)-1-butylamino, and the like.

The term "arylaminoalkylthio" means alkylthio as defined herein, wherein an alkyl hydrogen atom is replaced by an arylamino as defined herein. Examples of (arylamino) alkylthio radicals include 2- (phenylamino)- ethylthio, 3- (2-naphthylamino)-n-propylthio, and the like.

The term "aryloxy" means a radical of the formula aryl-O-in which the term aryl has the significance given above.

The term "aryloxyalkanoyl" means an acyl radical of the formula aryl-O-alkanoyl wherein aryl and alkanoyl have the meaning given above.

The term "aryloxyalkoxy" means alkoxy as defined herein, wherein an alkyl hydrogen atom is replaced by an aryloxy as defined herein. Examples of (aryloxy) alkoxy radicals include 2-phenoxyethoxy, 4- (3-aminophenoxy)-1- butoxy, and the like.

The term "aryloxyalkyl" means alkyl as defined herein, wherein an alkyl hydrogen atom is replaced by an aryloxy as defined herein. Examples of aryloxyalkyl radicals include phenoxyethyl, 4- (3-aminophenoxy)-I-butyl, and the like.

The term "aryloxyalkylamino" means alkylamino as defined herein, wherein an alkyl hydrogen atom is replaced by an aryloxy as defined herein. Examples of (aryloxy) alkylamino radicals include 3-phenoxy-npropylamino, 4-phenoxybutylamino, and the like.

The term "aryloxyalkylthio" means alkylthio as defined herein, wherein an alkyl hydrogen atom is replaced by an aryloxy as defined herein. Examples of (aryloxy) alkylthio radicals include 3-phenoxypropylthio, 4 (2-fluorophenoxy)-butylthio, and the like.

The term "arylthioalkylamino" means alkylamino as defined herein, wherein an alkyl hydrogen atom is replaced by an arylthio as defined herein. Examples of (arylthio) alkylamino radicals include 2- (phenylthio)- ethylamino, and the like.

The term "arylthioalkylthio" means alkylthio as defined herein, wherein an alkyl hydrogen atom is replaced by an arylthio as defined herein. Examples of (arylthio) alkylthio radicals include 2- (naphthylthio)- ethylthio, 3- (phenylthio)-propylthio, and the like.

The term "cycloalkylalkoxycarbonyl" means an acyl group derived from a cycloalkylalkoxycarboxylic acid of the formula cycloalkylalkyl-O-COOH wherein cycloalkylalkyl has the meaning given above.

The term "cycloalkylcarbonyl" means an acyl group derived from a monocyclic or bridged cycloalkanecarboxylic acid such as cyclopropylcarbonyl, cyclohexylcarbonyl, adamantylcarbonyl, and the like, or from a benz-fused monocyclic cycloalkanecarboxylic acid which is optionally substituted by one or more substituents selected from alkyl, alkoxy, halogen, hydroxy, amino, nitro, cyano, haloalkyl, carboxy, alkoxycarbonyl, cycloalkyl, heterocycloalkyl, alkanoylamino, amido, mono and dialkyl substituted amino, mono and dialkyl substituted amido and the like, such as 1,2,3,4-tetrahydro-2-naphthoyl, 2-acetamido-1,2,3,4-tetrahydro-2-naphthoyl.

The term "Het²alkoxy" means alkoxy as defined herein, wherein an alkyl hydrogen atom is replaced by a Het² as defined herein. Examples of Het²alkoxy radicals include 2-pyridylmethoxy, 4- (I-imidazolyl)-butoxy, and the like.

The term "Het²alkyl" means alkyl as defined herein, wherein an alkyl hydrogen atom is replaced by a Het² as defined herein. Examples of Het²alkyl radicals include 2-pyridylmethyl, 3- (4-thiazolyl)-propyl, and the like.

The term "Het²alkylamino" means alkylamino as defined herein, wherein an alkyl hydrogen atom is replaced by a Het² as defined herein. Examples of Het²alkylamino radicals include 4-pyridylmethylamino, 3 (2-furanyl)-propylamino, and the like.

The term "Het²alkylthio" means alkylthio as defined herein, wherein an alkyl hydrogen atom is replaced by a Het² as defined herein. Examples of Het²alkylthio radicals include 3-pyridylmethylthio, 3 (4-thiazolyl)-propylthio, and the like.

The term "Het²amino" means Het² as defined herein, wherein a hydrogen atom on the Het² ring is replaced by a nitrogen. Het²amino radicals include, for example, 4-thiazolylamino, 2-pyridylamino, and the like.

The term "Het²oxy" means Het² as defined herein, wherein a hydrogen atom on the Het² ring is replaced by an oxygen. Het²oxy radicals include, for example, 4-pyridyloxy, 5-quinolyloxy, and the like.

The term "Het²oxycarbonyl" means an acyl radical derived from a carbonic acid represented by Het²-O-COOH wherein Het² has the meaning given above.

The term "Het²thio" means Het² as defined herein, wherein a hydrogen atom on the Het² ring is replaced by a sulfur. Het²thio radicals include, for example, 3-pyridylthio, 3-quinolylthio, 4-imidazolylthio, and the like.

The term "Het¹alkanoyl" is an acyl radical derived from a Het¹-substituted alkylcarboxylic acid wherein Het¹ has the meaning given above.

The term "Het¹alkoxycarbonyl" means an acyl group derived from Het¹-O-COOH wherein Het¹ is as defined above.

As used herein before, the term "one or more" covers the possibility of all the available C-atoms, where appropriate, to be substituted, preferably, one, two or three. When any variable, e.g. halogen or alkyl, occurs more than one time in any constituent, each definition is independent.

Whenever used in the present invention the term "compounds of the invention" or "glycosylated steroid compounds" or a similar term is meant to include the compounds of general formula I and any subgroup thereof. This term also refers to the compounds as depicted in Table A and B and their derivatives, *N*-oxides, salts, solvates, hydrates, stereoisomeric forms, racemic mixtures, tautomeric forms, optical isomers, analogs, prodrugs, esters and metabolites, as well as their quaternized nitrogen analogues. The *N-*oxide forms of said compounds are meant to comprise compounds wherein one or several nitrogen atoms are oxidized to the so-called *N*-oxide.

The articles "a" and "an" are used herein to refer to one or to more than one, i.e. to at least one, the grammatical object of the article. By way of example, "a compound" means one compound or more than one compound.

The term "diseases associated with cell migration" as used herein refers to, but is not limited to, any type of cancer or condition involving cell migration, including for example chronic inflammation and restenosis in cardiovascular disease.

The term "pro-drug" as used herein means the pharmacologically acceptable derivatives such as esters, amides and phosphates, such that the resulting *in vivo* biotransformation product of the derivative is the active drug. The reference by Goodman and Gilman (The Pharmacological Basis of Therapeutics, 8th Ed, McGraw-Hill, Int. Ed. 1992, "Biotransformation of Drugs", p 13-15) describing pro-drugs generally is hereby incorporated. Pro-drugs of the compounds of the invention can be prepared by modifying functional groups present in said component in such a way that the modifications are cleaved, either in routine manipulation or *in vivo,* to the parent component. Typical examples of pro-drugs are described for instance in WO 99/33795, WO 99/33815, WO 99/33793 and WO 99/33792 all incorporated herein by reference. Pro-drugs are characterized by increased bio-availability and are readily metabolized into the active inhibitors *in vivo.*

The compounds according to the invention may also exist in their tautomeric forms. Such forms, although not explicitly indicated in the compounds described herein, are intended to be included within the scope of the present invention.

For therapeutic use, the salts of the compounds according to the invention are those wherein the counter-ion is pharmaceutically or physiologically acceptable.

As used herein and unless otherwise stated, the term " solvate " includes any combination which may be formed by a compound of this invention with a suitable inorganic solvent (e.g. hydrates) or organic solvent, such as but not limited to alcohols, ketones, esters and the like.

The pharmaceutically acceptable salts of the compounds according to the invention, i.e. in the form of water-, oil-soluble, or dispersible products, include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, dtrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such a sarginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

In a first embodiment the present invention relates to a glycosylated steroid compound of the formula I, stereoisomers, tautomers, racemics, prodrugs, metabolites thereof, or a pharmaceutically acceptable salt and/or solvate thereof, 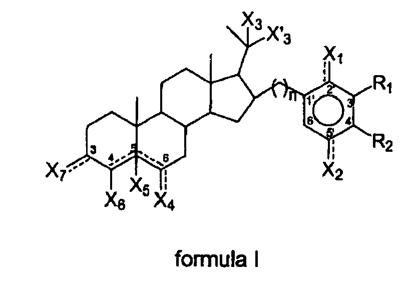 wherein each of X₁, X₂, R₁, R₂, X₃, X'₃, X₄, X₅, X₆ and X₇ are as defined in any of claims 1 to 11. In a more preferred embodiment, the present invention relates to a glycosylated steroid compound of the formula I as indicated above, stereoisomers, tautomers, racemics, prodrugs, metabolites thereof, or a pharmaceutically acceptable salt and/or solvate thereof,
wherein X₁, X₂ are independently-OMe, R₁ and R₂ are independently hydrogen,
wherein X₃ participates together with X'₃ to an oxo functional group, or wherein X₃ and X'₃ are independently selected from the group comprising hydrogen, hydroxyl, oxyalkyl, oxycarbonyl, glucosyl, fructosyl, galactosyl, mannosyl, ribosyl, ribulosyl, xylulosyl, erythrosyl, erythrulosyl, rhamnosyl, threosyl, sorbosyl, psicosyl, tagatosyl, fucosyl, arabinosyl, altrosyl, laminaribiosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-amino-2-deoxy-mannosyl, 2-acetamido-2-deoxy-mannosyl, 2-acetamido-2-deoxy-4-O-β-D-galactosyl-D-gfucasyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, combination thereof, deoxy derivatives thereof, hydroxyl-protected acetate derivatives thereof, amino derivatives thereof, amido derivatives thereof, thio derivatives thereof, disaccharide thereof, trisaccharide thereof, oligosaccharide and polysaccharide thereof;
wherein X₄ and X₇ are independently selected from the group comprising hydrogen, oxygen, oxo, hydroxyl, glucosyl, fructosyl, galactosyl, mannosyl, ribosyl, ribulosyl, xylulosyl, erythrosyl, erythrulosyl, rhamnosyl, threosyl, sorbosyl, psicosyl, tagatosyl, fucosyl, arabinosyl, altrosyl, laminaribiosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy galactosyl, 2-acetamido-2-deoxy-galactosyl, 2-amino-2-deoxy-mannosyl, 2-acetamido-2-deoxy-mannosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, combination thereof, deoxy derivatives thereof, hydroxyl-protected acetate or benzoyl derivatives thereof, amino derivatives thereof, amido derivatives thereof, thio derivatives thereof, disaccharide thereof, trisaccharide thereof, oligosaccharide and polysaccharide thereof;
wherein at least one of X₃ , X'₃, X₄ and X₇ is a glycosyl moiety selected from the group as indicated above;
wherein X₄ or X₆ are hydrogen and wherein X₅ participates to a double bond between the carbon atoms in position 4 and 5 or in position 5 and 6, and
wherein n is 0. In an embodiment, said compound is as defined in claim 2.

In a preferred embodiment the compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-oc-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X'₃ is selected from the group comprising hydrogen, alkyl or aralkyl, wherein X₄ is hydrogen, wherein X₅ participates to a double bond between the carbon atoms in position 5 and 6, wherein X₆ is -H, wherein X₇ is selected from the group comprising hydrogen, oxygen, hydroxyl or oxo, and wherein n is 0.

Another particularly preferred compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ is selected from the group comprising hydrogen, hydroxyl, oxyalkyl or oxycarbonyl, wherein X'₃ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-Amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₄ is hydrogen, wherein X₅ participates to a double bond between the carbon atoms in position 5 and 6, wherein X₆ is -H, wherein X₇ is selected from the group comprising hydrogen, oxygen, hydroxyl or oxo, and wherein n is 0.

Another particularly preferred compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ participates together with X'₃ to an oxo functional group, wherein X₄ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₅ participates to a double bond between the carbon atoms in position 4 and 5, wherein X₆ is -H, wherein X₇ is selected from the group comprising hydrogen, oxygen, hydroxyl , alkyloxy or oxo, and wherein n is 0.

Another particularly preferred compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ participates together with X'₃ to an oxo functional group, wherein X₄ is hydrogen, wherein X₅ participates to a double bond between the carbon atoms in position 5 and 6, wherein X₆ is -H, wherein X₇ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof; and wherein n is 0.

Another particularly preferred compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ or X'₃ are independently selected from the group comprising hydrogen or glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-Amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₄ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₅ and X₆ participates to a double bond between the carbon atoms in position 4 and 5, wherein X₆ is -H, wherein X₇ is selected from the group comprising hydrogen, oxygen, hydroxyl, alkyloxy or oxo, wherein at least one of X₃ and X'₃ is a glycosyl moiety selected from the group as indicated above and wherein n is 0.

Another particularly preferred compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ or X'₃ are independently selected from the group comprising hydrogen, glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₄ is hydrogen, wherein X₅ and X₆ participates to a double bond between the carbon atoms in position 5 and 6, wherein X₆ is -H, wherein X₇ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein at least one of X₃ and X'₃ is a glycosyl moiety selected from the group as indicated above and wherein n is 0.

Another particularly preferred compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ participates together with X'₃ to an oxo functional group or are independently selected from the group comprising hydrogen, hydroxyl, alkyloxy, wherein X₄ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₅ and X₆ participates to a double bond between the carbon atoms in position 4 and 5, wherein X₆ is -H, wherein X₇ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, and wherein n is 0.

Another particularly preferred compound according to the invention is a compound having the formula I as indicated above, stereoisomers, tautomers, racemics, prodrugs, metabolites thereof, or a pharmaceutically acceptable salt and/or solvate thereof, wherein X₁ and X₂ are -OMe, wherein R₁ and R₂ are -H, wherein X₃ or X'₃ are independently selected from the group comprising hydrogen, glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-Fucosyl-D-Lactosyl, 3-Fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₄ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-Fucosyl-D-Lactosyl, 3-Fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein X₅ and X₆ participates to a double bond between the carbon atoms in position 4 and 5, wherein X₆ is -H, wherein X₇ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-Fucosyl-D-Lactosyl, 3-Fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, a disaccharide or a trisaccharide thereof, wherein at least one of X₃ and X'₃ is a glycosyl moiety selected from the group as indicated above and wherein n is 0.

The invention encompasses compound as defined in claim 11.

The compounds according to the invention also show an anti-migratory effect. The compounds according to the invention have the ability to stop the migration of cells away from the neoplastic tumor tissue and thus enable to reduce the colonization of new tissues by these cells.

In addition the compounds according to the invention exhibit a low toxicity level. The terms "toxicity" or "toxic effects" as used herein refer to the detrimental effect(s) a compound may have on healthy cells, tissues or organs. The toxicity level of the compounds according to the invention is surprisingly low. The compounds according to the invention combine the essential features of a good anti-migratory activity and a low level of toxicity. Consequently the compounds according to the invention may be used in pharmaceutical compositions for the treatment of various diseases. In addition, because they have a low level of toxicity the compounds according to the invention may be used during longer periods of treatments.

### Method of preparation

In another embodiment, the present invention relates to methods for preparing the compounds according to the invention having the structural formula I 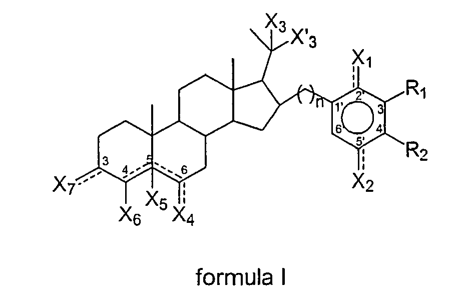 wherein X₁, X₂, X₃, X'₃, X₄, X₅, X₆, X₇, R₁, R₂ and n are independently selected from the group as indicated above, said method comprising the steps of
a) providing a starting material having the structural formula IV, 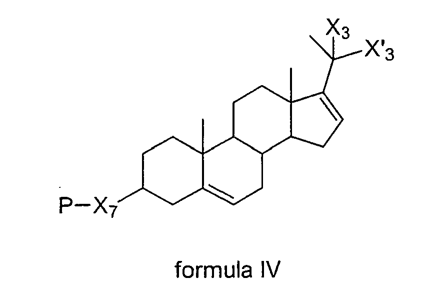 wherein X₃ participates together with X'₃ to an oxo functional group, or wherein X₃ and X'₃ are independently selected from the group comprising hydrogen, hydroxyl, sulfur, oxyalkyl, oxycarbonyl, alkyl, Het¹alkyl, alkyloxycarbonyl, alkenyl, alkynyl, aminoalkyl, aminoacyl, alkylcarbonylamino, alkylthiocarbonylamino, Het¹, optionally substituted by one or more substituents independently selected from the group comprising alkyl, aralkyl, aryl, Het¹, Het², cycloalkyl, alkyloxy, alkyloxycarbonyl, carboxyl, aminocarbonyl, mono- or di(alkyl)aminocarbonyl, aminosulfonyl, alkylS(=O)t, hydroxy, cyano, halogen or amino optionally mono- or disubstituted wherein the substituents are independently selected from the group comprising alkyl, aryl, aralkyl, aryloxy, arylamino, arylthio, aryloxyalkyl, arylaminoalkyl, aralkoxy, alkylthio, alkoxy, aryloxyalkoxy, aylaminoalkoxy, aralkylamino, aryloxyalkylamino, arylaminoalkylamino, arylthioalkoxy, arylthioalkylamino, aralkylthio, aryloxyalkylthio, arylaminoalkylthio, arylthioalkylthio, alkylamino, cycloalkyl and cycloalkylalkyl;
   wherein X₇ is selected from the group comprising hydrogen, oxygen, halogen, oxo, carbonyl, thiocarbonyl, hydroxyl, alkyl, aryl, Het¹, Het¹alkyl, Het¹aryl, alkenyl, alkynyl, hydroxyalkyl, hydroxycarbonyl, hydroxycarbonylalkyl, hydroxycarbonylaryl, hydroxycarbonyloxyalkyl optionally substituted by one or more substituents independently selected from the group comprising alkyl, aralkyl, aryl, Het¹, Het², cycloalkyl, alkyloxy, alkyloxycarbonyl, carboxyl, aminocarbonyl, mono- or di(alkyl)aminocarbonyl, aminosulfonyl, alkylS(=O)t, hydroxy, cyano, halogen or amino optionally mono- or disubstituted wherein the substituents are independently selected from the group comprising alkyl, aryl, aralkyl, aryloxy, arylamino, arylthio, aryloxyalkyl, arylaminoalkyl, aralkoxy, alkylthio, alkoxy, aryloxyalkoxy, aylaminoalkoxy, aralkylamino, aryloxyalkylamino, arylaminoalkylamino, arylthioalkoxy, arylthioalkylamino, aralkylthio, aryloxyalkylthio, arylaminoalkylthio, arylthioalkylthio, alkylamino, cycloalkyl and cycloalkylalkyl, and wherein X₃ and X'₃ preferably form oxo, and
   wherein P is a protecting group selected from the group comprising alkyl aryl silane, alkyl silane and carbonylalkylaryl, and wherein P preferably is t-butyl diphenyl silane,
b) effecting reaction between the compound of step a) with an organometallic compound having the structural formula V 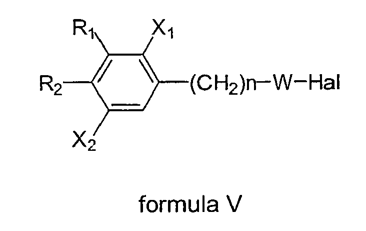
wherein X₁, X₂, R₁, R₂ and n are independently selected from the group as indicated above, wherein W is a metal or a combination of metals selected from the group comprising magnesium and preferably copper and wherein Hal is a halogen atom, and preferably selected from the group comprising bromine, chlorine and iodine, to result in an intermediate having the structural formula III' 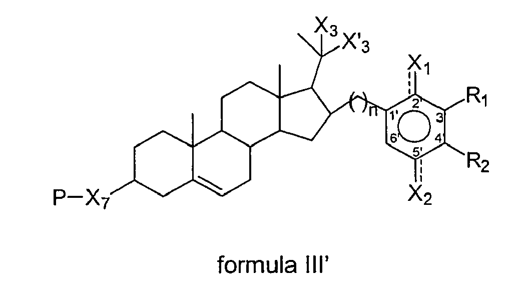 wherein X₁, X₇ R₁, R₂ and n are independently selected from the group as indicated above, wherein X₃, X'₃, X₇ are independently selected from the group as indicated in step
a) and preferably wherein X₃ participates together with X'₃ to an oxo functional group,
   wherein P is a protecting group as indicated above,
c) effecting reaction between the compound of step b) with an organometallic compound having the structural formula VI

   Hal-W-X'₃

   formula VI

   wherein X'₃ is selected from the group as indicated in step a), wherein W is a metal or a combination of metals selected from the group comprising magnesium and preferably copper, and wherein Hal is a halogen atom, preferably selected from the group comprising bromine, chlorine and iodine, to result in an intermediate having the structural formula III 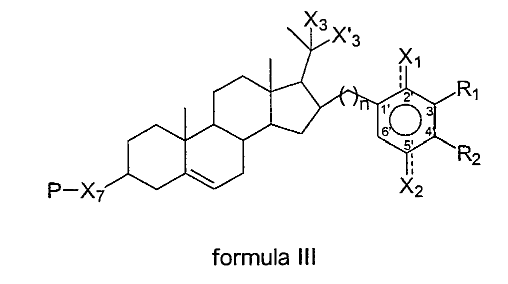 wherein X₁, X₂, R_{1'} R₂ and n are independently selected from the group as indicated above, wherein X₃, X'₃, X₇ are independently selected from the group as indicated in step a), wherein P is a protecting group,
d) deprotecting the X₇ group of the compound obtained in step c) to form a compound having the structural formula II 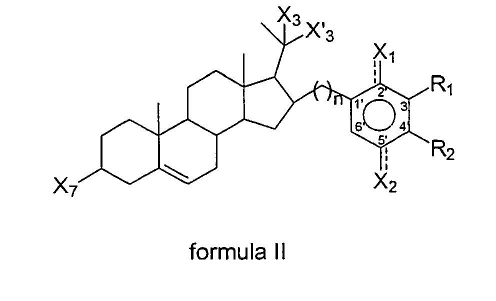 wherein X₁, X₂, R₁, R₂ and n are independently selected from the group as indicated above, wherein X₃, X'₃, X₇ are independently selected from the group as indicated in step a), and
e) coupling an O-protected glycosyl or non-protected glycosyl to form a compound of formula I wherein X₁, X₂, R₁, R₂ and n are independently selected from the group as indicated above, wherein X₃, X'₃ are independently selected from the group as indicated in step a), and X₇ is an O-protected glycosyl or a non-protected glycosyl, and
f) deprotecting the O-protected groups of glycosyl to form the compound having the formula I wherein X₁, X₂, X₄, X₅, X₆, R₁, R₂ and n are independently selected from the group as indicated above, wherein X₃, X'₃ are independently selected from the group as indicated in step a), and X₇ is selected from the group comprising a glycosyl, thio derivatives thereof, amido derivatives thereof, amino derivatives thereof, hydroxyl-protected derivatives thereof.

In another embodiment of the present invention, wherein step c) consists of reacting the compound of step b) with an O-protected glycosyl or non-protected glycosyl to result in an intermediate having the structural formula III wherein X₁, X₂, R₁, R₂ and n are independently selected from the group as indicated above, wherein X₃, X₇ are independently selected from the group as indicated in step a) of the present method, wherein P is a protecting group, and wherein X₃ or X'₃ is an O-protected glycosyl or a non protected glycosyl and continuing the reaction with steps d), e) and f) according to the present method to form a glycosylated steroid compound of structural formula I.

In yet another embodiment of the present invention, step e) consists of reacting the compound of step d) with an oxidizing reagent to form an intermediate and reducing said intermediate with a reducing reagent to result in another intermediate having the structural formula I wherein X₁, X₂, R₁, R₂ and n are independently selected from the group as indicated above, and X₃ or X'₃, and X₄ and X₇ are hydroxyl and continuing the reaction with steps e) and f) according to the present method to form a glycosylated steroid compound of structural formula I.

Protected forms of the inventive compounds are included within the scope of the present invention. A variety of protecting groups are disclosed, for example, in T. H. Greene and P. G. M. Wuts, Protective Groups in Organic Synthesis, Third Edition, John Wiley & Sons, New York (1999), which is incorporated herein by reference in its entirety. For example, hydroxy protected forms of the inventive compounds are those where at least a hydroxy protecting group protects one of the hydroxyl groups. Illustrative hydroxyl protecting groups include but not limited to tetrahydropyranyl; benzyl; methylthiomethyl; ethylthiomethyl; pivaloyl; phenylsulfonyl; triphenylmethyl; trisubstituted silyl such as trimethyl silyl, triethylsilyl, tributylsilyl, tri-isopropylsilyl, t-butyldimethylsilyl, tri-t-butylsilyl, methyldiphenylsilyl, ethyldiphenylsilyl, t-butyldiphenylsilyl and the like; acyl and aroyl such as acetyl, benzoyl, pivaloylbenzoyl, 4-methoxybenzoyl, 4-nitrobenzoyl and aliphatic acylaryl and the like. Keto groups in the inventive compounds may similarly be protected.

The glycosylated steroid compounds according to the present invention are prepared using an enone as the starting compound. These enones, having general formula IV, can be synthesized according to the procedure described in Tetrahedron, 1993, 49(23), 5079-5090 or starting from commercially available enones as 16-dehydropregnenolone acetate. For example this latter after deacetylation, 16-dehydropregnenolone was protected with protecting groups as defined above. The derivatives represented by formula V or formula VI are prepared either from corresponding commercially available halides or by known methods as described for instance in Tetrahedron, 1982, 3555-3561. Example 2 provided below illustrates the preparation of several different glycosylated steroid compounds according to the invention.

In another embodiment, the present invention also relates to a compound, which is obtained by any of the steps according to the above-described methods for synthesis of a compound of formula I. A number of these compounds identified herein as intermediates also find utility as pharmaceutical agents. Certain intermediate compounds obtained in any of the above-described steps of the synthesis methods may be useful in the treatment of disorders, in particular cancers.

### Uses of the compounds according to the invention

An important feature attributed to the compounds according to the invention is their broad application possibility. The compounds according to the invention exhibit anti-migratory activity on cancer cells, as illustrated in examples 3 and 4 provided below. There are therefore particularly suitable as anti-migratory agents.

When a malignant tumour has reached a certain size, tumour cells move away from the initial tumour site and start to migrate. The actin cytoskeleton, tubulin and adhesion molecules linking the constituents of extracellular matrix to intracellular actin cytoskeleton are central to locomotion. The extracellular matrix proteins such as fibronectin, laminin and collagen are recognized by endogenous lectins, which specifically bind to various sugar moieties (β-galactoside, fucose, manose, etc) present in said proteins. For example, the selectins and their ligands (fucose-related Lewis antigens) play critical roles in the invasion processes of various types of cancers (including those of the stomach, lung and melanomas) towards the liver. Various Lewis antigen types also exert significant roles in neoangiogenesis processes. This selectin/Lewis antigen system therefore represents new potential therapeutic targets in cancer field. For example, an increased expression of sialyl Lewis antigen correlates with poor survival in patients with colorectal carcinoma (Nakamori *et al,* 1993), an increased expression of Lewis^{x} antigen correlates with metastatic potential and poor prognostic in patients with gastric carcinoma (Mayer *et al,* 1996). Some of the compounds of the invention are believed to bind to the selectin of tumour cells thereby preventing said cells to migrate to site comprising the Lewis antigen. Other compounds of the invention are believed to bind to other lectins, including for example galectins or manose binding proteins.

Due to these interesting properties; in particular the anti-migratory activity and the low level of toxicity, the glycosylated steroid compounds according to the invention are particularly suitable for use as a medicament in the treatment of diseases associated with cell migration, and even in particular in the treatment of cancer. Therefore, in another embodiment, the invention relates to compounds according to the invention for use as a medicament. In yet another embodiment, the invention provides compounds for use in the preparation of a medicament for treating cancer.

The compounds of the invention may be especially used in the treatment of cancers such as but not limited to leukemia, non-small cell lung cancer, small cell lung cancer, CNS cancer, melanoma, ovarian cancer, kidney cancer, prostate cancer, breast cancer, glioma, colon cancer, bladder cancer, sarcoma, pancreatic cancer, colorectal cancer, head and neck cancer, liver cancer, bone cancer, bone marrow cancer, stomach cancer, duodenum cancer, oesophageal cancer, hematological cancer and lymphoma.

In addition, the compounds according to the invention may also be very suitable in the treatment of scar tissue and wounds. It is believed that most, if not all, of the compounds of the present invention can act as active ingredients in treating scar tissue and in promoting wound healing and tissue regeneration. In yet another embodiment, the invention provides compounds for use in the preparation of a medicament for treating scar tissue.

### Pharmaceutical compositions comprising the glycosylated steroid compounds

In another embodiment, the present invention relates to a pharmaceutical composition comprising a pharmaceutically acceptable excipient and a therapeutic amount of a compound according to the invention.

The term "therapeutically effective amount" as used herein means that amount of active compound or component or pharmaceutical agent that elicits the biological or medicinal response in a tissue, system, animal or human that is being sought by a researcher, veterinarian, medical doctor or other clinician, which includes alleviation of the symptoms of the disease being treated.

The pharmaceutical composition can be prepared in a manner known per se to one of skill in the art. For this purpose, at least one compound having formula I, one or more solid or liquid pharmaceutical excipients and, if desired, in combination with other pharmaceutical active compounds, are brought into a suitable administration form or dosage form which can then be used as a pharmaceutical in human medicine or veterinary medicine.

Particular forms of the pharmaceutical composition may be, for example, solutions, suspensions, emulsions, creams, tablets, capsules, nasal sprays, liposomes or micro-reservoirs, especially compositions in orally ingestible or sterile injectable form, for example, as sterile injectable aqueous or oleaginous suspensions or suppositories. The preferred form of composition contemplated is the dry solid form, which includes capsules, granules, tablets, pills, boluses and powders. The solid carrier may comprise one or more excipients, e.g. lactose, fillers, disintegrating agents, binders, e.g. cellulose, carboxymethylcellulose or starch or anti-stick agents, e.g. magnesium stearate, to prevent tablets from adhering to tabletting equipment. Tablets, pills and boluses may be formed so as to disintegrate rapidly or to provide slow release of the active ingredient.

In order to enhance the solubility and/or the stability of the compounds of a pharmaceutical composition according to the invention, it can be advantageous to employ α-, β- or γ-cyclodextrins or their derivatives. In addition, co-solvents such as alcohols may improve the solubility and/or the stability of the compounds. In the preparation of aqueous compositions, addition of salts of the compounds of the invention are obviously more suitable due to their increased water solubility.

Appropriate cyclodextrins are α-, β- or γ-cyclodextrins (CDs) or ethers and mixed ethers thereof wherein one or more of the hydroxy groups of the anhydroglucose units of the cyclodextrin are substituted with alkyl, particularly methyl, ethyl or isopropyl, e.g. randomly methylated β-CD; hydroxyalkyl, particularly hydroxyethyl, hydroxypropyl or hydroxybutyl; carboxyalkyl, particularly carboxymethyl or carboxyethyl; alkylcarbonyl, particularly acetyl; alkyloxycarbonylalkyl or carboxyalkyloxyalkyl, particularly carboxymethoxypropyl or carboxyethoxypropyl; alkylcarbonyloxyalkyl, particularly 2-acetyloxypropyl. Especially noteworthy as complexants and/or solubilizers are β-CD, randomly methylated β-CD, 2,6-dimethyl- β-CD, 2-hydroxyethyl-β-CD, 2-hydroxyethyl-γ-CD, 2-hydroxypropyl-γ-CD and (2-carboxymethoxy)propyl- β-CD, and in particular 2-hydroxypropyl- β-CD (2-HP- β-CD). The term mixed ether denotes cyclodextrin derivatives wherein at least two cyclodextrin hydroxy groups are etherified with different groups such as, for example, hydroxypropyl and hydroxyethyl. An interesting way of formulating the analogues in combination with a cyclodextrin or a derivative thereof has been described in EP-A-721,331. Although the formulations described therein are with antifungal active ingredients, they are equally interesting for formulating the analogues. Said formulations may also be rendered more palatable by adding pharmaceutically acceptable sweeteners and/or flavors.

More in particular, the compositions may be formulated in a pharmaceutical formulation comprising a therapeutically effective amount of particles consisting of a solid dispersion of the compounds of the invention and one or more pharmaceutically acceptable water-soluble polymers.

The term "a solid dispersion" defines a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase as defined in thermodynamics, such a solid dispersion is referred to as "a solid solution". Solid solutions are preferred physical systems because the components therein are usually readily bioavailable to the organisms to which they are administered. The term "a solid dispersion" also comprises dispersions that are less homogenous throughout than solid solutions. Such dispersions are not chemically and physically uniform throughout or comprise more than one phase.

The water-soluble polymer is conveniently a polymer that has an apparent viscosity of 1 to 100 mPa.s when dissolved in a 2 % aqueous solution at 20°C solution. Preferred water-soluble polymers are hydroxypropyl methylcelluloses or HPMC. HPMC having a methoxy degree of substitution from about 0.8 to about 2.5 and a hydroxypropyl molar substitution from about 0.05 to about 3.0 are generally water soluble. Methoxy degree of substitution refers to the average number of methyl ether groups present per anhydroglucose unit of the cellulose molecule. Hydroxy-propyl molar substitution refers to the average number of moles of propylene oxide which have reacted with each anhydroglucose unit of the cellulose molecule.

It may further be convenient to formulate the analogues in the form of nanoparticles which have a surface modifier adsorbed on the surface thereof in an amount sufficient to maintain an effective average particle size of less than 1000 nm. Suitable surface modifiers can preferably be selected from known organic and inorganic pharmaceutical excipients. Such excipients include various polymers, low molecular weight oligomers, natural products and surfactants. Preferred surface modifiers include nonionic and anionic surfactants.

Yet another interesting way of formulating the compounds according to the invention involves a pharmaceutical composition whereby the compounds are incorporated in hydrophilic polymers and applying this mixture as a coat film over many small beads, thus yielding a composition with good bio-availability which can conveniently be manufactured and which is suitable for preparing pharmaceutical dosage forms for oral administration. Said beads comprise (a) a central, rounded or spherical core, (b) a coating film of a hydrophilic polymer and an antiretroviral agent and (c) a seal-coating polymer layer. Materials suitable for use as cores in the beads are manifold, provided that said materials are pharmaceutically acceptable and have appropriate dimensions and firmness. Examples of such materials are polymers, inorganic substances, organic substances, and saccharides and derivatives thereof.

### Methods of treatment

The compounds according to the invention exhibit anti-migratory activity on cancer cells.

As indicated above, due to the favourable anti-migratory properties and the low level of toxicity, of the compounds according to the present invention are particularly useful in the treatment of diseases associated with cell migration, such as in the the treatment of individuals suffering from cancer. Therefore, in another embodiment, the present invention also relates to the use of the glycosylated steroid compounds according to the invention or to a pharmaceutical composition comprising said glycosylated steroid compounds in the treatment of cancer. A method of treating cancer comprises administering to an individual in need of such treatment a pharmaceutical composition comprising the glycosylated steroid compounds according to the invention.

The compounds of the invention may be especially used in the treatment of cancers such as but not limited to leukemia, non-small cell lung cancer, small cell lung cancer, CNS cancer, melanoma, ovarian cancer, kidney cancer, prostate cancer, breast cancer, glioma, colon cancer, bladder cancer, sarcoma, pancreatic cancer, colorectal cancer, head and neck cancer, liver cancer, bone cancer, bone marrow cancer, stomach cancer, duodenum cancer, oesophageal cancer, hematological cancer and lymphoma.

In addition, the compounds according to the invention may also be very useful in the treatment of scar tissue and wounds. It is believed that most, if not all, of the compounds of the present invention can act as active ingredients in treating scar tissue and in promoting wound healing and tissue regeneration. Therefore, in another embodiment, the present invention also relates to the use of the glycosylated steroid compounds according to the invention or to a pharmaceutical composition comprising said glycosylated steroid compounds in the treatment of scar tissue. A method of treating scar tissue comprises administering to an individual in need of such treatment a pharmaceutical composition comprising the glycosylated steroid compounds according to the invention.

In yet another embodiment, the present invention also relates to the use of the glycosylated steroid compounds according to the invention or to a pharmaceutical composition comprising said glycosylated steroid compounds for treating wounds and promoting wound healing and tissue regeneration. A method of treating wounds comprises administering to an individual in need of such treatment a pharmaceutical composition comprising the glycosylated steroid compounds according to the invention.

For these purposes, the pharmaceutical composition of the present invention may be administered orally, parenterally, i.e. including subcutaneous injections, intravenous, intramuscular, intrasternal injection or infusion techniques, by inhalation spray, or rectally, in dosage unit formulations containing conventional non-toxic pharmaceutically acceptable carriers, adjuvants and vehicles.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

Essentially, the primary modes of treatment of solid tumor cancers comprise surgery, radiation therapy and chemotherapy, separately and in combination. The compounds according to the invention are suitable for use in combination with these medicinal techniques. The compounds of the invention may be useful in increasing the sensitivity of tumor cells to radiation in radiotherapy and also in potentiating or enhancing damage to tumors by chemotherapeutic agents. The compounds and their pharmaceutically acceptable salts and/or solvates may also be useful for sensitising multidrug-resistant tumor cells. The compounds according to the invention are useful therapeutic compounds for administration in conjunction with other DNA-damaging cytotoxic drugs or radiation used in radiotherapy to potentiate their effect.

In another embodiment of the method of the invention, the administration may be performed with food, e.g., a high-fat meal. The term 'with food' means the consumption of a meal either during or no more than about one hour before or after administration of a pharmaceutical composition according to the invention.

For an oral administration form, the compositions of the present invention can be mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

The oral administration of a pharmaceutical composition comprising a glycosylated steroid compound according to the invention, or a pharmaceutical acceptable salt or ester and/or solvate thereof, is suitably accomplished by uniformly and intimately blending together a suitable amount of the glycosylated steroid compound in the form of a powder, optionally also including a finely divided solid carrier, and encapsulating the blend in, for example, a hard gelatin capsule. The solid carrier can include one or more substances, which act as binders, lubricants, disintegrating agents, coloring agents, and the like. Suitable solid carriers include, for example, calcium phosphate, magnesium stearate, talc, sugars, lactose, dextrin, starch, gelatin, cellulose, polyvinylpyrrolidine, low melting waxes and ion exchange resins.

Oral administration of a pharmaceutical composition comprising a glycosylated steroid compound according to the invention, or a pharmaceutically acceptable salt or ester and/or solvate thereof can also be accomplished by preparing capsules or tablets containing the desired amount of the glycosylated steroid compound, optionally blended with a solid carrier as described above. Compressed tablets containing the pharmaceutical composition of the invention can be prepared by uniformly and intimately mixing the active ingredient with a solid carrier such as described above to provide a mixture having the necessary compression properties, and then compacting the mixture in a suitable machine to the shape and size desired. Molded tablets maybe made by molding in a suitable machine, a mixture of powdered glycosylated steroid compound moistened with an inert liquid diluent.

When administered by nasal aerosol or inhalation, these compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the invention or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant.

For subcutaneous or intravenous administration, the active analogue, if desired with the substances customary therefor such as solubilizers, emulsifiers or further auxiliaries, are brought into solution, suspension, or emulsion. The compounds of the invention can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these formulations may be prepared by mixing the compounds according to the invention with a suitable non-irritating excipient, such as cocoa butter, synthetic glyceride esters or polyethylene glycols, which are solid at ordinary temperatures, but liquidify and/or dissolve in the rectal cavity to release the drug.

The pharmaceutical compositions of this invention can be administered to humans in dosage ranges specific for each analogue comprised in said compositions. The compounds comprised in said composition can be administered together or separately.

It will be understood, however, that specific dose level and frequency of dosage for any particular patient may be varied and will depend upon a variety of factors including the activity of the specific analogue employed, the metabolic stability and length of action of that compound, the age, body weight, general health, sex, diet, mode and time of administration, rate of excretion, drug combination, the severity of the particular condition, and the host undergoing therapy.

The following examples are meant to illustrate the present invention. These examples are presented to exemplify the invention and are not to be considered as limiting the scope of the invention. Example 1 provides a non-limiting list of examples of compounds according to the invention. Example 2 illustrates the preparation of different compounds according to the invention. Example 3 illustrates *in vitro* anti-tumor effects of several compounds according to the invention. Example 4 illustrates in vivo anti-tumor effects of two compounds according to the invention.

### Examples

The practice of the present invention will employ, unless otherwise indicated, conventional techniques of synthetic organic chemistry, biological testing, and the like, which are within the skill of the art. Such techniques are explained fully in the literature.

### Example 1 Non-limiting examples of compounds according to the invention having general formula I are listed hereunder in Table A

The present invention emcompasses stereoisomers, tautomers, racemics, prodrugs, metabolites thereof, or a pharmaceutically acceptable salt and/or solvate thereof of the compounds listed in Table A.

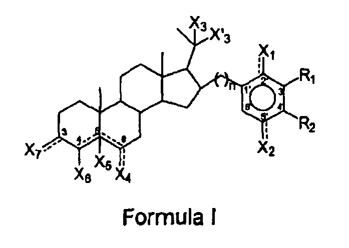

**TABLE A**

| **X₁** | **X₂** | **X₃** | **X'₃** | **X₄** | **X₅** | **X₆** | **X₇** | **R₁** | **R₂** | **n** |
|---|---|---|---|---|---|---|---|---|---|---|
| -O-CH₃ | -O-CH₃ | =O | | -H | -* | -H | palatinosyl | -H | -H | 0 |
| -OMe | -OMe | =O | | -H | -** | -H | maltosyl | H | H | 0 |
| -OMe | -OMe | =O | | -H | -** | -H | xylopyranosyl | H | H | 0 |
| -O-CH₃ | -O-CH₃ | H | β-D-mannosyl | =O | -* | -H | =O | -H | -H | 0 |
| -OMe | -OMe | H | β-D-Lactosyl | -H | -** | -H | -OH | -H | -H | 0 |
| -O-CH₃ | -O-CH₃ | kojibiosyl | -H | =O | -* | -H | -OH | -H | -H | 0 |
| -O-CH₃ | -O-CH₃ | -OH | -(CH₂)-CH-(CH₃)₂ | β-D-Meliblosyl | -* | -H | =O | -H | -H | 0 |
| -OMe | -OMe | =O | | galactopyranosyl | -* | -H | =O | H | H | 0 |
| -OMe | -OMe | =O | | mannopyranosyl | -* | -H | =O | H | H | 0 |
| -OMe | -OMe | =O | | xylopyranosyl | -* | -H | =O | H | H | 0 |
| -OMe | -OMe | =O | | cellobiosyl | -* | -H | =O | H | H | 0 |
| -OMe | -OMe | =O | | lactosyl | -* | -H | =O | H | H | 0 |
| -OMe | -OMe | =O | | glucofuranosyl | -* | -H | =O | H | H | 0 |
| -OMe | -OMe | =O | | maltosyl | -* | -H | =O | H | H | 0 |
| -OMe | -OMe | =O | | gentiobiosyl | -* | -H | =O | H | H | 0 |
| -O-CH₃ | -O-CH₃ | =O | | -H | -** | -H | D-Glucosyl | -H | -H | 0 |
| -O-CH₃ | -O-CH₃ | =O | | -H | -** | -H | D-Mannosyl | -H | -H | 0 |
| -O-CH₃ | -O-CH₃ | =O | | -H | -** | -H | D-Galactosyl | -H | -H | 0 |
| -O-CH₃ | -O-CH₃ | =O | | -H | -** | -H | D-Cellobiosyl | -H | -H | 0 |
| -O-CH₃ | -O-CH₃ | =O | | -H | -** | -H | D-isomaltosyl | -H | -H | 0 |
| -O-CH₃ | -O-CH₃ | =O | | -H | -** | -H | L-Fucosyl | -H | -H | 0 |
| -O-CH₃ | -O-CH₃ | =O | | -H | -** | -H | D-Lactosyl | -H | -H | 0 |
| -O-CH₃ | -O-CH₃ | =O | | -H | -** | -H | D-Gentiobiosyl | -H | -H | 0 |
| -O-CH₃ | -O-CH₃ | =O | | -H | -** | -H | D-Maltosyl | -H | -H | 0 |
| -O-CH₃ | -O-CH₃ | =O | | -H | -** | -H | 2-acetamido-2-deoxy-D-Glucosyl | -H | -H | 0 |
| -O-CH₃ | -O-CH₃ | =O | | -H | -** | -H | 2-acetamido-2deoxy-D-Galactosyl | -H | -H | 0 |
| -O-CH₃ | -O-CH₃ | L- Fucosyl | H | -H | -** | -H | L-Fucosyl | -H | -H | 0 |
| -O-CH₃ | -O-CH₃ | L-Fucosyl | -H | -H | -** | -H | L-Fucosyl | -H | -H | 0 |
| -O-CH₃ | -O-CH₃ | D-Isomaltosyl | -H | -H | -** | -H | D-Isomaltosyl | -H | -H | 0 |
| -O-CH₃ | -O-CH₃ | D-Gentiobiosyl | -H | -H | -** | -H | D-Gentiobiosyl | -H | -H | 0 |
| -O-CH₃ | -O-CH₃ | D-maltosyl | -H | -H | -** | -H | D-maltosyl | -H | -H | 0 |
| -O-CH₃ | -O-CH₃ | D-Lactosyl | -H | -H | -** | -H | D-Lactosyl | -H | -H | 0 |
| -O-CH₃ | -O-CH₃ | N-acetyllactosaminyl | -H | -H | -** | -H | N-acetyllactosaminyl | -H | -H | 0 |
| -O-CH₃ | -O-CH₃ | 2-acetamido-2-deoxy-4-O-β-D-Galactosyl-D-Glucosyl | -H | -H | -** | -H | 2-acetamido-2-deoxy-4-O-2-deoxy-4-O-β-D-Galactosyl-D-Glucosyl | -H | -H | 0 |
| -O-CH₃ | -O-CH₃ | 3-fucosyl-2-acetamido-2-deoxy-4-O-β-D-galactosyl-D-glucosyl | -H | -H | -** | -H | 3-fucosyl-2-acetamido-2-deoxy-4-O-β-D-galactosyl-D-glucosyl | -H | -H | 0 |
| -O-CH₃ | -O-CH₃ | 3-fucosyl-D-lactosyl | -H | -H | -** | -H | 3-fucosyl-D-lactosyl | -H | -H | 0 |
| -O-CH₃ | -O-CH₃ | D-Melibiosyl | -H | -H | -** | -H | D-Melibiosyl | -H | -H | 0 |
| -O-CH₃ | -O-CH₃ | D-Maltotriosyl | -H | -H | -** | -H | D-Maltotriosyl | -H | -H | 0 |
| -O-CH₃ | -O-CH₃ | D-Lactulosyl | -H | -H | -** | -H | D-Lactulosyl | -H | -H | 0 |
| -O-CH₃ | -O-CH₃ | D-Palatinosyl | -H | -H | -** | -H | D-Palatinosyl | -H | -H | 0 |
| -O-CH₃ | -O-CH₃ | D-Lactosyl | -H | -H | -** | -H | D-Cellobiosyl | -H | -H | 0 |
| -O-CH₃ | -O-CH₃ | D-Lactosyl | -H | -H | -** | -H | D-Isomaltosyl | -H | -H | 0 |
| -O-CH₃ | -O-CH₃ | D-Lactosyl | -H | -H | -** | -H | L-Fucosyl | -H | -H | 0 |
| -O-CH₃ | -O-CH₃ | D-Lactosyl | -H | -H | -** | -H | D-Gentiobiosyl | -H | -H | 0 |
| -O-CH₃ | -O-CH₃ | D-Lactosyl | -H | -H | -** | -H | D-maltosyl | -H | -H | 0 |
| -O-CH₃ | -O-CH₃ | D-Lactosyl | -H | -H | -** | -H | 2-acetamido-2-deoxy-4-O-β-D- Galactosyl-D-Glucosyl | -H | -H | 0 |
| -O-CH₃ | -O-CH₃ | D-Cellobiosyl | -H | -H | -** | -H | D-Lactosyl | -H | -H | 0 |
| -O-CH₃ | -O-CH₃ | D-Cellobiosyl | -H | -H | -** | -H | D-Isomaltosyl | -H | -H | 0 |
| -O-CH₃ | -O-CH₃ | D-Cellobiosyl | -H | -H | -** | -H | L-Fucosyl | -H | -H | 0 |
| -O-CH₃ | -O-CH₃ | D-Cellobiosyl | -H | -H | -** | -H | D- Gentiobiosyl | -H | -H | 0 |
| -O-CH₃ | -O-CH₃ | D-Cellobiosyl | -H | -H | -** | -H | D-maltosyl | -H | -H | 0 |
| -O-CH₃ | -O-CH₃ | D-Cellobiosyl | -H | -H | -** | -H | 2-acetamido-2-deoxy-4-O-β-D-Galactosyl-D-Glucosyl | -H | -H | 0 |
| -O-CH₃ | -O-CH₃ | D-Isomaltosyl | -H | -H | -** | -H | D-Lactosyl | -H | -H | 0 |
| -O-CH₃ | -O-CH₃ | D-Isomaltosyl | -H | -H | -** | -H | D-Cellobiosyl | -H | -H | 0 |
| -O-CH₃ | -O-CH₃ | D-isomaltosyl | -H | -H | -** | -H | L-Fucosyl | -H | -H | 0 |
| -O-CH₃ | -O-CH₃ | D-Isomaltosyl | -H | -H | -** | -H | D-Gentiobiosyl | -H | -H | 0 |
| -O-CH₃ | -O-CH₃ | D-Isomaltosyl | -H | -H | -** | -H | D-maltosyl | -H | -H | 0 |
| -O-CH₃ | -O-CH₃ | D-Isomaltosyl | -H | -H | -** | -H | 2-acetamido-2-deoxy-4-O-β-D- Gal actosyl-D-Glucosyl | -H | -H | 0 |
| -O-CH₃ | -O-CH₃ | L-Fucosyl | -H | -H | -** | -H | D-Lactosyl | -H | -H | 0 |
| -O-CH₃ | -O-CH₃ | L-Fucosyl | -H | -H | -** | -H | D-Cellobiosyl | -H | -H | 0 |
| -O-CH₃ | -O-CH₃ | L-Fucosyl | -H | -H | -** | -H | D-Isomaltosyl | -H | -H | 0 |
| -O-CH₃ | -O-CH₃ | L-Fucosyl | -H | -H | -** | -H | D-Gentiobiosyl | -H | -H | 0 |
| -O-CH₃ | -O-CH₃ | L-Fucosyl | -H | -H | -** | -H | D-maltosyl | -H | -H | 0 |
| -O-CH₃ | -O-CH₃ | L-Fucosyl | -H | -H | -** | -H | 2-acetamido-2-deoxy-4-O-β-D-Galactosyl-D-Glucosyl | -H | -H | 0 |
| -O-CH₃ | -O-CH₃ | D-Gentiobiosyl | -H | -H | -** | -H | D-Lactosyl | -H | -H | 0 |
| -O-CH₃ | -O-CH₃ | D-Gentiobiosyl | -H | -H | -** | -H | D-Cellobiosyl | -H | -H | 0 |
| -O-CH₃ | -O-CH₃ | D-Gentiobiosyl | -H | -H | -** | -H | D-Isomaltosyl | -H | -H | 0 |
| -O-CH₃ | -O-CH₃ | D-Gentiobiosyl | -H | -H | -** | -H | L-Fucosyl | -H | -H | 0 |
| -O-CH₃ | -O-CH₃ | D-Gentiobiosyl | -H | -H | -** | -H | D-maltosyl | -H | -H | 0 |
| -O-CH₃ | -O-CH₃ | D-Gentiobiosyl | -H | -H | -** | -H | 2-acetamido-2-deoxy-4-O-β-D-Galactosyl-D-Glucosyl | -H | -H | 0 |
| -O-CH₃ | -O-CH₃ | D-maltosyl | -H | -H | -** | -H | D-Lactosyl | -H | -H | 0 |
| -O-CH₃ | -O-CH₃ | D-maltosyl | -H | -H | -** | -H | D-Cellobiosyl | -H | -H | 0 |
| -O-CH₃ | -O-CH₃ | D-maltosyl | -H | -H | -** | -H | D-Isomaltosyl | -H | -H | 0 |
| -O-CH₃ | -O-CH₃ | D-maltosyl | -H | -H | -** | -H | L-Fucosyl | -H | -H | 0 |
| -O-CH₃ | -O-CH₃ | D-maltosyl | -H | -H | -** | -H | D-Gentiobiosyl | -H | -H | 0 |
| -O-CH₃ | -O-CH₃ | D-maltosyl | -H | -H | -** | -H | 2-acetamido-2-deoxy-4-O-β-D-Gal actosyl-D-Glucosyl | -H | -H | 0 |
| -O-CH₃ | -O-CH₃ | 2-acetamido-2-deoxy-4-O-β-D-Galactosyl-D-Glucosyl | -H | -H | -** | -H | D-Lactosyl | -H | -H | 0 |
| -O-CH₃ | -O-CH₃ | 2-acetamido-2-deoxy-4-O-β-D-Galactosyl-D-Glucosyl | -H | -H | -** | -H | D-Cellobiosyl | -H | -H | 0 |
| -O-CH₃ | -O-CH₃ | 2-acetamido-2-deoxy-4-O-β-D-Galactosyl-D-Glucosyl | -H | -H | -** | -H | D-Isomaltosyl | -H | -H | 0 |
| -O-CH₃ | -O-CH₃ | 2-acetamido-2-deoxy-4-O-β-D-Galactosyl-D-Glucosyl | -H | -H | -** | -H | L-Fucosyl | -H | -H | 0 |
| -O-CH₃ | -O-CH₃ | 2-acetamido-2-deoxy-4-O-β-D-Galactosyl-D-Glucosyl | -H | -H | -** | -H | D-Gentiobiosyl | -H | -H | 0 |
| -O-CH₃ | -O-CH₃ | 2-acetamido-2-deoxy-4-O-β-D- Galactosyl-D-Glucosyl | -H | -H | -** | -H | D-maltosyl | -H | -H | 0 |
| -O-CH₃ | -O-CH₃ | L-Fucosyl | -H | L-Fucosyl | -* | -H | -OH | -H | -H | 0 |
| -O-CH₃ | -O-CH₃ | D-Cellobiosyl | -H | D-Cellobiosyl | -* | -H | -OH | -H | -H | 0 |
| -O-CH₃ | -O-CH₃ | D-isomaltosyl | -H | D-Isomaltosyl | -* | -H | -OH | -H | -H | 0 |
| -O-CH₃ | -O-CH₃ | D-Gentiobiosyl | -H | D-Gentiobiosyl | -* | -H | -OH | -H | -H | 0 |
| -O-CH₃ | -O-CH₃ | D-maltosyl | -H | D-maltosyl | -* | -H | -OH | -H | -H | 0 |
| -O-CH₃ | -O-CH₃ | D-Lactosyl | -H | D-Lactosyl | -* | -H | -OH | -H | -H | 0 |
| -O-CH₃ | -O-CH₃ | N-acetyl- lactosaminyl | -H | N-acetyl- lactosaminyl | -* | -H | -OH | -H | -H | 0 |
| -O-CH₃ | -O-CH₃ | 2-acetamido-2-deoxy-4-O-β-D-Galactosyl-D-Glucosyl | -H | 2-acetamido-2-deoxy-4-O-β-D-Galactosyl-D-Glucosyl | -* | -H | -OH | -H | -H | 0 |
| -O-CH₃ | -O-CH₃ | 3-fucosyl-2-acetamido-2-deoxy-4-O-β-D-galactosyl- D-glucosyl | -H | 3-fucosyl-2-acetamido-2-deoxy-4-O-β-D- galactosyl-D-glucosyl | -* | -H | -OH | -H | -H | 0 |
| -O-CH₃ | -O-CH₃ | 3-fucosyl-D-lactosyl | -H | 3-fucosyl-D-lactosyl | -* | -H | -OH | -H | -H | 0 |
| -O-CH₃ | -O-CH₃ | D-Melibiosyl | -H | D-Melibiosyl | -* | -H | -OH | -H | -H | 0 |
| -O-CH₃ | -O-CH₃ | D-Maltotriosyl | -H | D-Maltotriosyl | -* | -H | -OH | -H | -H | 0 |
| -O-CH₃ | -O-CH₃ | D-Lactulosyl | -H | D-Lactulosyl | -* | -H | -OH | -H | -H | 0 |
| -O-CH₃ | -O-CH₃ | D-Palatinosyl | -H | D-Palatinosyl | -* | -H | -OH | -H | -H | 0 |
| -O-CH₃ | -O-CH₃ | L-Fucosyl | -H | L-Fucosyl, | -* | -H | L-Fucosyl | -H | -H | 0 |
| -O-CH₃ | -O-CH₃ | D-Cellobiosyl | -H | D-Cellobiosyl | -* | -H | D-Cellobiosyl | -H | -H | 0 |
| -O-CH₃ | -O-CH₃ | D-isomaltosyl | -H | D-Isomaltosyl | -* | -H | D-isomaltosyl | -H | -H | 0 |
| -O-CH₃ | -O-CH₃ | D-Gentiobiosyl | -H | D-Gentiobiosyl | -* | -H | Gentiobiosyl | -H | -H | 0 |
| -O-CH₃ | -O-CH₃ | D-maltosyl | -H | D-maltosyl | -* | -H | D-maltosyl | -H | -H | 0 |
| -O-CH₃ | -O-CH₃ | D-Lactosyl | -H | D-Lactosyl | -* | -H | D-Lactosyl | -H | -H | 0 |
| -O-CH₃ | -O-CH₃ | N-acetyl- lactosaminyl | -H | N-acetyl-lactosaminyl | * | -H | N-acetyl-lactosaminyl | -H | -H | 0 |
| -O-CH₃ | -O-CH₃ | 2-acetamido-2-deoxy-4-O-β-D-Galactosyl-D-Glucosyl | -H | 2-acetamido-2-deoxy-4-O-β-D-Galactosyl-D-Glucosyl | -* | -H | 2-acetamido-2-deoxy-4-O-β-D-Glucosyl | -H | -H | 0 |
| -O-CH₃ | -O-CH₃ | 3-fucosyl-2-acetamido-2-deoxy-4-O-β-D-galactosyl-D-glucosyl | -H | 3-fucosyl-2-acetamido-2-deoxy-4-O-β-D-galactosyl-D-glucosyl | -* | -H | 3-fucosyl-2-acetamido-2-deoxy-4-O-β-D-galactosyl-D-glucosyl | -H | -H | 0 |
| -O-CH₃ | -O-CH₃ | 3-fucosyl-D-lactosyl | -H | 3-fucosyl-D-lactosyl | -* | -H | 3-fucosyl-D-lactosyl | -H | -H | 0 |
| -O-CH₃ | -O-CH₃ | D-Melibiosyl | -H | D-Melibiosyl | -* | -H | D-Melibiosyl | -H | -H | 0 |
| -O-CH₃ | -O-CH₃ | D-Maltotriosyl | -H | D-Maltotriosyl | -* | -H | D-Maltotriosy | -H | -H | 0 |
| -O-CH₃ | -O-CH₃ | D-Lactulosyl | -H | D-Lactulosyl | -* | -H | D-Lactulosyl | -H | -H | 0 |
| -O-CH₃ | -O-CH₃ | D-Palatinosyl | -H | D-Palatinosyl | -* | -H | D-Palatinosyl | -H | -H | 0 |
| -O-CH₃ | -O-CH₃ | =O | | L-Fucosyl | -* | -H | L-Fucosyl | -H | -H | 0 |
| -O-CH₃ | -O-CH₃ | =O | | D-Cellobiosyl | -* | -H | D-Cellobiosyl | -H | -H | 0 |
| -O-CH₃ | -O-CH₃ | =O | | D-Isomaltosyl | -* | -H | D-Isomaltosyl | -H | -H | 0 |
| -O-CH₃ | -O-CH₃ | =O | | D-Gentiobiosyl | -* | -H | Gentiobiosyl | -H | -H | 0 |
| -O-CH₃ | -O-CH₃ | =O | | D-maltosyl | -* | -H | D-maltosyl | -H | -H | 0 |
| -O-CH₃ | -O-CH₃ | =O | | D-Lactosyl | -* | -H | D-Lactosyl | -H | -H | 0 |
| -O-CH₃ | -O-CH₃ | =O | | N-acetyl-lactosaminyl | * | -H | N-acetyl-lactosaminyl | -H | -H | 0 |
| -O-CH₃ | -O-CH₃ | =O | | 2-acetamido-2-deoxy-4-O-β-D-Galactosyl-D-Glucosyl | -* | -H | 2-acetamido-2-deoxy-4-O-β-D-Galactosyl-D-Glucosyl | -H | -H | 0 |
| -O-CH₃ | -O-CH₃ | =O | | 3-fucosyl-2-acetamido-2-deoxy-4-O-β-D-galactosyl-D-glucosyl | -* | -H | 3-fucosyl-2-acetamido-2-deoxy-4-O-β-D-galactosyl-D-glucosyl | -H | -H | 0 |
| -O-CH₃ | -O-CH₃ | =O | | 3-fucosyl-D-lactosyl | -* | -H | 3-fucosyl-D-lactosyl | -H | -H | 0 |
| -O-CH₃ | -O-CH₃ | =O | | D-Melibiosyl | -* | -H | D-Melibiosyl | -H | -H | 0 |
| -O-CH₃ | -O-CH₃ | =O | | D-Maltotriosyl | -* | -H | D-Maltotriosyl | -H | -H | 0 |
| -O-CH₃ | -O-CH₃ | =O | | D-Lactulosyl | -* | -H | D-Lactulosyl | -H | -H | 0 |
| -O-CH₃ | -O-CH₃ | =O | | D-Palatinosyl | -* | -H | D-Palatinosyl | -H | -H | 0 |
| -O-CH₃ | -O-CH₃ | =O | | -H | -** | -H | N-acetyl-lactosaminyl | -H | -H | 0 |
| -O-CH₃ | -O-CH₃ | =O | | -H | -** | -H | 2-acetamido-2-deoxy-4-O-β-D-Galactosyl-D-Glucosyl | -H | -H | 0 |
| -O-CH₃ | -O-CH₃ | =O | | -H | -** | -H | 3-fucosyl-2-acetamido-2-deoxy-4-O-β-D-galactosyl-D-glucosyl | -H | -H | 0 |
| -O-CH₃ | -O-CH₃ | =O | | -H | ** | -H | 3-fucosyl-D-lactosyl | -H | -H | 0 |
| -O-CH₃ | -O-CH₃ | =O | | -H | -** | -H | D-Melibiosyl | -H | -H | 0 |
| -O-CH₃ | -O-CH₃ | =O | | -H | -** | -H | D-Maltotrisosyl | -H | -H | 0 |
| -O-CH₃ | -O-CH₃ | =O | | -H | -** | -H | D-Lactulosyl | -H | -H | 0 |
| -O-CH₃ | -O-CH₃ | =O | | -H | -** | -H | D-palatinosyl | -H | -H | 0 |

| | | | | | | | | | | |
|---|---|---|---|---|---|---|---|---|---|---|
| * refers to fact that X₅ participates to a double bond between the carbon atoms in position 4 and 5 ** refers to fact that X₅ participates to a double bond between the carbon atoms in position 5 and 6 | | | | | | | | | | |

### Example 2 Preparation of glycosylated steroid derivatives according to the invention

The present example provides evidence for the preparation of thirteen different compounds according to the invention, UBS3268, UBS3270, UBS3285, UBS3327, UBS3328, UBS3501, UBS3585, UBS3597, UBS3976, UBS4066, UBS4067, UBS4095, UBS4104, UBS4109, UBS4209 and UBS4373. The prepared compounds and their intermediates are represented in Table B. The present invention emcompasses stereoisomers, tautomers, racemics, prodrugs, metabolites thereof, or a pharmaceutically acceptable salt and/or solvate thereof of the compounds listed in Table B.

First compounds of formula IV can be prepared for example with this method: To a solution of 16-dehydropregnenolone acetate (100 mg; 0.28mmol) in methanol (8 ml) was added a solution of K₂CO₃ (640 mg; 4.6 mmol) in distilled water (10 ml). After stirring for 2h at room temperature, the solvent was evaporated and the residue was extracted with CH₂Cl₂ (3x50 ml) and water (50 ml). The combined extracts were dried with Na₂SO₄ and concentrated to dryness. The crude product was then dissolved in DMF (2 ml) then imidazole (95 mg; 1.4 mmol) and tert-butyldiphenylsilyl chloride (154 mg; 0.56 mmol) were added. The solution was stirred for 17h at room temperature. After extraction of the product with hexane (3x50ml) and concentration in vacuo, silica gel flash chromatography (cyc/ohexane/acetone 99:1) led to a white product having formula IV (143 mg, 0.26 mmol, 90%).

### 1. Preparation of compound UBS3268

A solution of 1-bromo-2,5-dimethoxybenzene (3.3 g, 15.3 10⁻³ mol) and 1,2-dibromoethane (2.9 g, 15.3 10⁻³ mol) in dry Et₂O (4 ml) was added dropwise to Mg turnings (1.1 g, 45.6 10⁻³ mol) in dry Et₂O (5 ml) with two crystals of I₂ under N₂ at 0°C. After 30min, copper (I) iodide (0.36 g, 2 10⁻³ mol) was added. After 15min, a solution of compound of formula IV (2.12 g, 3.8 10⁻³ mol) in dry Et₂O was added. After 30 min, the mixture was treated with aqueous NH₄Cl and extracted with Et₂O (3x50 ml). Purification of the crude mixture by silica gel flash chromatography (*cyclo*hexane/acetone 98/2) afforded compound UBS1513 (1.57 g, 2.3 10⁻³ mol). The yield of the preparation was 59%.

To a solution of UBS1513 (150 mg, 0.22 mmol) in THF was subsequently added a 1M solution of *n*-Bu₄NF (650 µl, 0.65 mmol) in THF and the mixture was stirred for 2 days at room temperature. The solvent was evaporated. Purification of the crude mixture by silica gel flash chromatography (*cyclo*hexane/ ethyl acetate 2:1) afforded compound UBS1634 (86 mg, 0.2 mmol). The yield of the preparation was 88%.

UBS3267 was prepared by coupling at -20°C the compound UBS1634 (50 mg, 0.11 10⁻³ mol) in 8 ml of dichloromethane, 2ml of toluene and tetrabenzoylglucoside bromide (131 mg, 0.20 10⁻³ mol) in presence of silver trifluoromethane sulfonate (52 mg, 0.20 10⁻³ mol) and allyltrimethylsilane (72 mg, 0.62 10⁻³ mol). Tetrabenzoylglucoside bromide and others carbohydrate derivatives were prepared according to the procedure described in Steroids 63:44-49, 1998. The mixture was stirred overnight at room temperature. Purification of the crude mixture by silica gel chromatography (cyclohexane/AcOEt 8/2) provided 14 mg of the compound UBS3267. The yield of this preparation process was 91%.

Subsequently, a solution of sodium methanolate 33% wt in methanol (0.084 ml, 0.46 10⁻³ mol) was added at room temperature to a stirred solution of UBS3267 (80 mg, 7.76 10⁻⁵ mol) in methanol/dichloromethane (4/2 v/v). The reaction mixture was stirred for 30 min at room temperature. After neutralization and evaporation, the residue was purified by column chromatography on silica gel (CH₂Cl₂/MeOH 95/5) in order to provide 43 mg of the compound **UBS3268**. The yield of this preparation process was 90%.

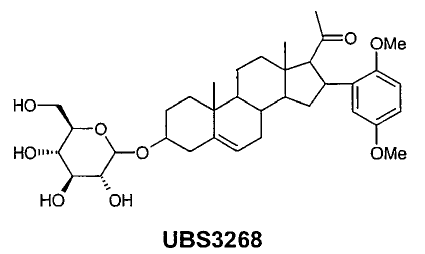

### 2. Preparation of compound UBS3270

In a similar manner as described for the preparation of UBS3267, the compound UBS1634 (60 mg, 0.13 10⁻³ mol) was treated with tetrabenzoylmannoside bromide (158 mg, 0.24 10⁻³ mol) in presence of silver trifluoromethane sulfonate (62 mg, 0.24 10⁻³ mol) and allyltrimethylsilane (120 µl, 86 mg, 0.74 10⁻³ mol) to obtain 112mg of the compound UBS3269. The yield of this preparation process was 82%.

In a similar manner as described for the preparation of UBS3268, the compound UBS3269 (80 mg, 7.76 10⁻⁵ mol) was treated with sodium methanolate 33% in methanol (0.084 ml, 0.46 10⁻³ mol) at room temperature for 30 min to give 28 mg of compound UBS3270. The yield of this preparation process was 58%.

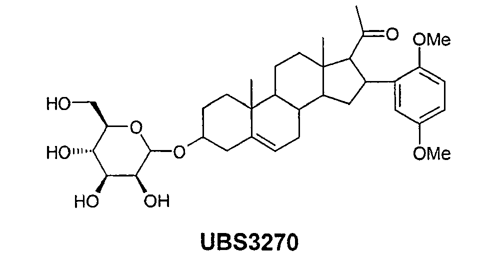

### 3. Preparation of compound UBS3285

A solution of tetrabenzylgalactopyranose (50 mg, 9.2 10⁻⁵ mol), p-toluenesulfonyl chloride (20 mg, 1 10⁻⁴ mol), tetrabutylammonium iodide (20 mg, 5 10⁻⁵ mol) and the compound UBS1634 (150 mg, 3 10⁻⁴ mol) in 10 ml of dichloromethane was stirred with 40% aqueous NaOH (5 ml) at room temperature. After 48h the organic layer was separated, washed with H₂O and dried (MgSO₄). The solvent was evaporated and the crude product was chromatographed on silica gel using (cyclohexane/AcOEt 9/1) in order to provide 25 mg of compound. The yield of this preparation process was 56%.

Subsequently, the later compound (20 mg, 2 10⁻⁵ mol) in 5 ml of ethanol and 5 ml of AcOEt. Pd/C (20 mg) and cyclohexene (1 ml) were added and the mixture was heated under reflux for 2h. The palladium was filtered and the solvent was evaporated under reduced pressure to give 12 mg of compound UBS3285. The yield of this preparation process was 99%.

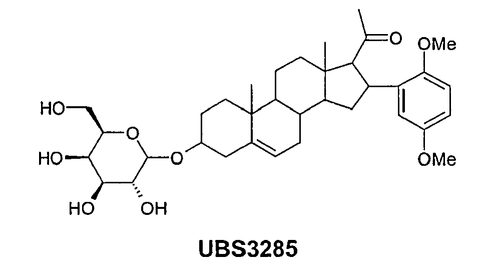

### 4. Preparation of compound UBS3327

In a similar manner as described for the preparation of UBS3267, the compound UBS1634 (50 mg, 0.11 10⁻³ mol) was treated with heptabenzoylcellobioside bromide (188 mg, 0.16 10⁻³ mol) in presence of silver trifluoromethane sulfonate (44 mg, 0.15 10⁻³ mol) and allyltrimethylsilane (100 µl, 72 mg, 0.62 10⁻³ mol) to obtain 126 mg of compound. The yield of this preparation process was 75%.

In a similar manner as described for the preparation of UBS3268, the later compound (120 mg, 7.9 10⁻⁵ mol) was subsequently treated with sodium methanolate 33% wt in methanol (0.143 ml, 7.9 10⁻⁴ mol) at room temperature for 30 min to give 73 mg of compound UBS3327. The yield of this preparation process was 69%.

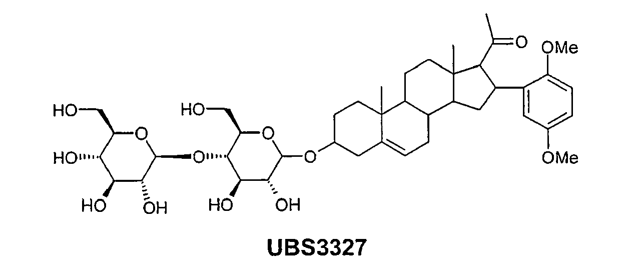

### 5. Preparation of compound UBS3328

In a similar manner as described for the preparation of UBS3267, the compound UBS1634 (50 mg, 0.11 10⁻³ mol) was treated with heptabenzoylisomaltoside bromide (188mg, 0.16 10⁻³ mol) in presence of silver trifluoromethane sulfonate (44 mg, 0.15 10⁻³ mol) and allyltrimethylsilane (100 µl, 72 mg, 0.62 10⁻³ mol) to obtain 57 mg of compound. The yield of this preparation process was 34%.

In a similar manner as described for the preparation of UBS3268, the later compound (45 mg, 3.0 10⁻⁵ mol) was subsequently treated with sodium methanolate 33%wt in methanol (54 µl, 3 10⁻⁴ mol) at room temperature for 30 min to give 20 mg of compound UBS3328. The yield of this preparation process was 86%.

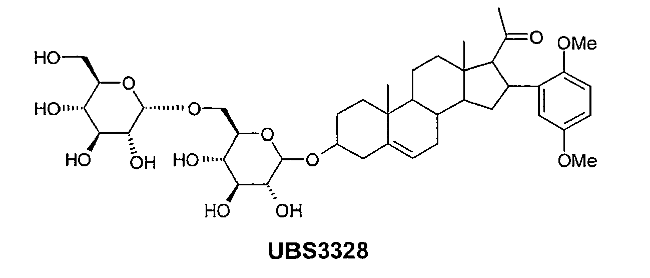

### 6. Preparation of compound UBS3501

In a similar manner as described for the preparation of UBS3267, the compound UBS1634 (50 mg, 0.11 mmol) was treated with tribenzoylfucoside bromide (119 mg, 0.22 mmol) in presence of silver trifluoromethane sulfonate (57 mg, 0.22 mmol) and allyltrimethylsilane (100 µl, 72 mg, 0.624 mmol) to obtain 82mg of compound. The yield of this preparation process was 81%.

In a similar manner as described for the preparation of UBS3268, the later compound (70 mg, 0.0768 mmol) was subsequently treated with sodium methanolate 33% wt in methanol (62 µl, 0.346 mmol) at room temperature for 30 min to give 42 mg of compound UBS3501. The yield of this preparation process was 92%.

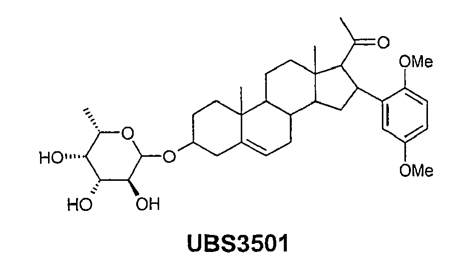

### 7. Preparation of compound UBS3585

To a suspension of 2-acetamido-2-deoxy-D-Glucose (0.196 g, 0.886 mmol) and UBS1634 (0.98 g, 2.17 mmol) in dry acetonitrile (30 ml) was added under Ar boron trifluoride diethyl etherate (22.5 µl, 0.177 mmol) and the reaction was stirred under reflux for 18h. After cooling, the solvent was evaporated under reduced pressure. The residue was purified by column chromatography (CH₂Cl₂/MeOH 9/1) to give 142 mg of UBS3585 (mixture of α and β forms) as a white solid. The yield of the preparation was 24%.

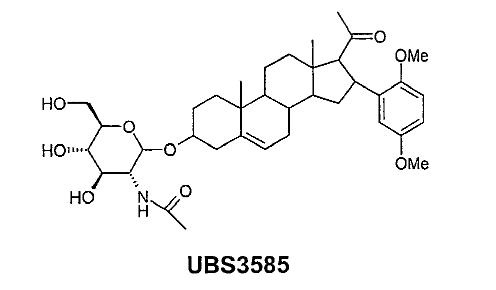

### 8. Preparation of compound UBS3597

In a similar manner as described for the preparation of UBS3585, the compound UBS1634 (0.99 g, 2.19 mmol) was treated with 2-acetamido-2-deoxy-D-Galactose (0.196 g, 0.886 mmol) and boron trifluoride (22 µl, 0.177 mmol) to obtain 50 mg of the compound UBS3597 (mixture of α and β forms) after chromatography on silica gel (CH₂Cl₂/MeOH 9/1). The yield of this preparation process was 9%.

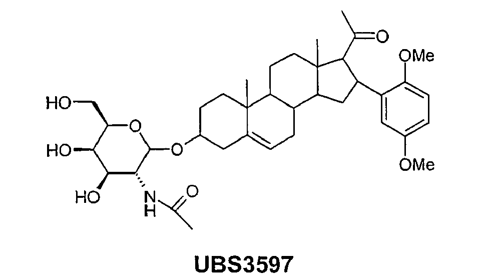

### 9. Preparation of compound UBS3976

In a similar manner as described for the preparation of UBS3267, the compound UBS1634 (100 mg, 0.22 mmol) was treated with heptabenzoyllactoside bromide (502 mg, 0.44 mmol) in presence of silver trifluoromethane sulfonate (115 mg, 0.44 mmol) and allyltrimethylsilane (200 µl, 1.25 mmol) to obtain 330 mg of compound. The yield of this preparation process was 99%.

In a similar manner as described for the preparation of UBS3268, the later compound (230 mg, 0.1528 mmol) was subsequently treated with sodium methanolate 33% wt in methanol (250 µl, 1.528 mmol) at room temperature for 30 min to give 72 mg of compound UBS3976 after chromatography on silica gel (CH₂Cl₂/MeOH 85/15). The yield of this preparation process was 61 %.

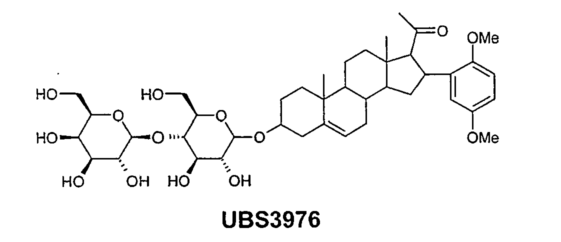

### 10. Preparation of compound UBS4066

A solution of UBS1634 (200 mg, 0.442 mmol) and sodium borohydride (102 mg, 1.654 mmol) in 5 ml of methanol was stirred at room temperature. After 24h the solvent was evaporated and the crude product was chromatographed on silica gel using (cyclohexane/AcOEt 7/3) in order to provide 195 mg of compound. The yield of this preparation process was 95%.

In a similar manner as described for the preparation of UBS3267, the obtained compound above (50 mg, 0.11 mmol) was treated with tribenzoylfucoside bromide (238 mg, 0.44 mmol) in presence of silver trifluoromethane sulfonate (115 mg, 0.44 mmol) and allyltrimethylsilane (200 µl, 1.25 mmol) to obtain 82 mg of compound. The yield of this preparation process was 54%.

In a similar manner as described for the preparation of UBS3268, the later compound (65 mg, 0.047 mmol) was subsequently treated with sodium methanolate 33% wt in methanol (70 µl, 0.426 mmol) at room temperature for 30 min to give 20 mg of compound UBS4066 after flash chromatography (CH₂Cl₂/MeOH 9/1). The yield of this preparation process was 56%.

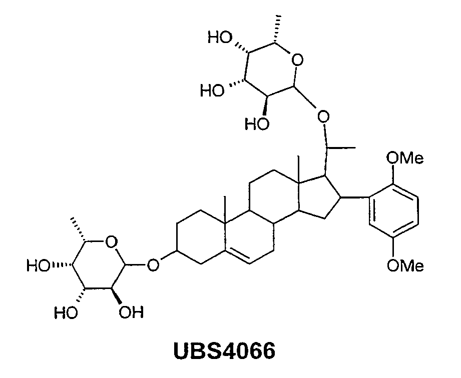

### 11. Preparation of compound UBS4067

In a similar manner as described for the preparation of UBS3267, the compound UBS1634 (150 mg, 0.33 mmol) was treated with heptabenzoylgentiobioside bromide (752 mg, 0.66 mmol) in presence of silver trifluoromethane sulfonate (172 mg, 0.66 mmol) and allyltrimethylsilane (300 µl, 1.89 mmol) to obtain 366 mg of compound. The yield of this preparation process was 73%.

In a similar manner as described for the preparation of UBS3268, the later compound (340 mg, 0.226 mmol) was subsequently treated with sodium methanolate 33% wt in methanol (370 µl, 2.26 mmol) at room temperature for 30 min to give 126 mg of compound UBS4067 after flash chromatography (CH₂Cl₂/MeOH 8/2). The yield of this preparation process was 72%.

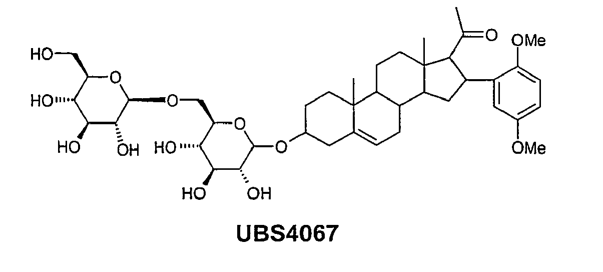

### 12. Preparation of compound UBS4095

In a similar manner as described for the preparation of UBS3267, the compound UBS1634 (150 mg, 0.33 mmol) was treated with heptabenzoylmaltoside bromide (752 mg, 0.66 mmol) in presence of silver trifluoromethane sulfonate (172 mg, 0.66 mmol) and allyltrimethylsilane (300 µl, 1.89 mmol) to obtain 400 mg of compound. The yield of this preparation process was 80%.

In a similar manner as described for the preparation of UBS3268, the later compound (350 mg, 0.232 mmol) was subsequently treated with sodium methanolate 33% wt in methanol (780 µl, 4.75 mmol) at room temperature for 30 min to give 150 mg of compound UBS4095 after flash chromatography (CH₂Cl₂/MeOH 9/1). The yield of this preparation process was 83%.

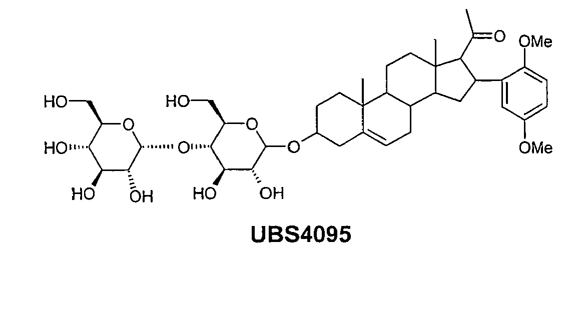

### 13. Preparation of compound UBS4104

A solution of 2-acetamido-3,4,6-tri-O-acetyl-2-deoxy-α-D-Glucopyranosyl chloride (1.62 g, 4.42 10⁻³ mol), calcium sulfate (0.904 g, 6.64 10⁻³ mol), compound UBS1634 (1 g, 2.21 10⁻³ mol) in 15 ml of dichloromethane was stirred at room temperature. After 15 min mercuric(II) cyanide (1.70 g, 6.64 10⁻³ mol) was added and the mixture is left stirring during 24h at the room temperature then diluted with dichloromethane, and washed with sodium bicarbonate, 10% potassium iodide and water. The organic phase was dried with sodium sulfate, filtrated and concentrated. The crude product was chromatographed on silica gel using (cyclohexane/AcOEt 3/7) providing 1.38 g of compound. The yield of this preparation process was 80%.

In a similar manner as described for the preparation of UBS3268, the later compound (1.3 g, 1.66 mmol) was subsequently treated with sodium methanolate 33% wt in methanol (1.23 ml, 7.49 mmol) at room temperature for 30 min to give 926 mg of compound UBS4104 (β form) after flash chromatography (CH₂Cl₂/MeOH 85/15). The yield of this preparation process was 85%.

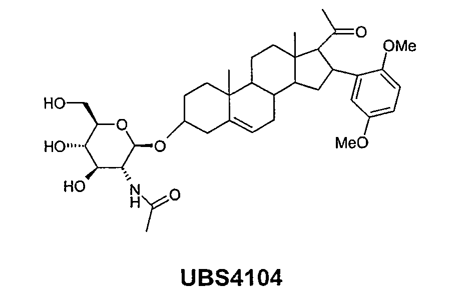

### 14. Preparation of compound UBS4109

A solution of UBS1634 (200 mg, 0.442 mmol) and sodium borohydride (102 mg, 1.654 mmol) in 5 ml of methanol was stirred at room temperature. After 24h the solvent was evaporated and the crude product was chromatographed on silica gel using (cyclohexane/AcOEt 7/3) in order to provide 195 mg of compound. The yield of this preparation process was 95%.

In a similar manner as described for the preparation of UBS3267, the compound obtained above (160 mg, 0.352 mmol) was treated with heptabenzoylcellobioside bromide (996 mg, 0.878 mmol) in presence of silver trifluoromethane sulfonate (228 mg, 0.878 mmol) and allyltrimethylsilane (200 µl, 1.25 mmol) to obtain 456 mg of compound. The yield of this preparation process was 51 %.

In a similar manner as described for the preparation of UBS3268, the later compound (420 mg, 0.164 mmol) was subsequently treated with sodium methanolate 33% wt in methanol (563 µl, 3.44 mmol) at room temperature for 30 min to give 80 mg of compound UBS4109 after flash chromatography (CH₂Cl₂/MeOH 7/3). The yield of this preparation process was 44%.

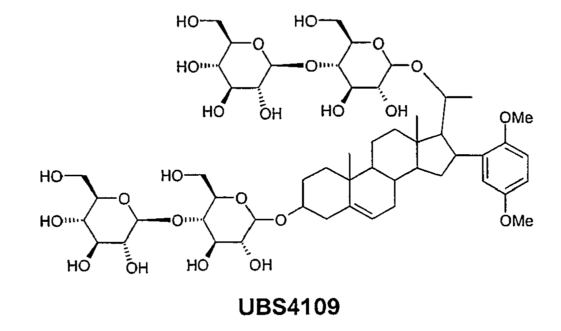

### 15. Preparation of compound UBS4209

A solution of UBS1634 (200 mg, 0.442 mmol) and sodium borohydride (102 mg, 1.654 mmol) in 5 ml of methanol was stirred at room temperature. After 24h the solvent was evaporated and the crude product was chromatographed on silica gel using (cyclohexane/AcOEt 7/3) in order to provide 195 mg of compound. The yield of this preparation process was 95%.

In a similar manner as described for the preparation of UBS3267, the compound obtained above (160 mg, 0.352 mmol) was treated with heptabenzoyllactoside bromide (996 mg, 0.878 mmol) in presence of silver trifluoromethane sulfonate (228 mg, 0.878 mmol) and allyltrimethylsilane (200 µl, 1.25 mmol) to obtain 550 mg of compound. The yield of this preparation process was 61 %.

In a similar manner as described for the preparation of UBS3268, the later compound (550 mg, 0.215 mmol) was subsequently treated with sodium methanolate 33% wt in methanol (1126 µl, 6.88 mmol) at room temperature for 30 min to give 150 mg of compound UBS4209 after flash chromatography (CH₂Cl₂/MeOH 7/3). The yield of this preparation process was 64%.

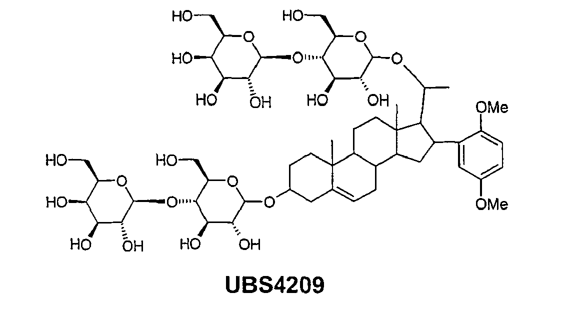

### 16. Preparation of compound UBS4373

A solution of UBS1634 (200 mg, 0.442 mmol) and sodium borohydride (102 mg, 1.654 mmol) in 5 ml of methanol was stirred at room temperature. After 24h the solvent was evaporated and the crude product was chromatographed on silica gel using (cyclohexane/AcOEt 7/3) in order to provide 195 mg of compound. The yield of this preparation process was 95%.

In a similar manner as described for the preparation of UBS3267, the obtained compound above (160 mg, 0.352 mmol) was treated with heptabenzoylisomaltoside bromide (996 mg, 0.878 mmol) in presence of silver trifluoromethane sulfonate (228 mg, 0.878 mmol) and allyltrimethylsilane (200 µl, 1.25 mmol) to obtain 280 mg of the compound. The yield of this preparation process was 31 %.

In a similar manner as described for the preparation of UBS3268, the later compound (280 mg, 0.110 mmol) was subsequently treated with sodium methanolate 33% wt in methanol (1126 µl, 6.88 mmol) at room temperature for 30 min to give 93 mg of compound UBS4373 after flash chromatography (CH₂Cl₂/MeOH 6/4). The yield of this preparation process was 78%.

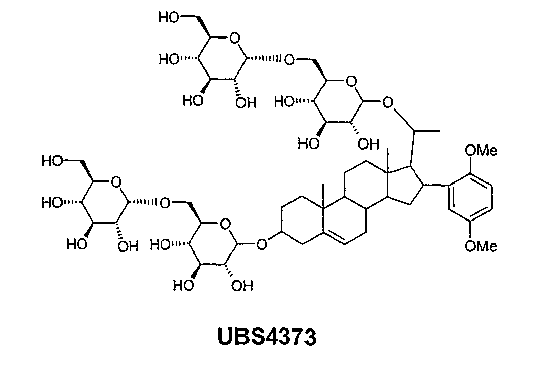

**TABLE B Compounds and their intermediates according to the invention**

| | **P** | **X₁** | **X₂** | **X₃** | **X_{3'}** | **X₄** | **X₅** | **X₆** | **X₇** | **R₁** | **R₂** | **n** |
|---|---|---|---|---|---|---|---|---|---|---|---|---|
| UBS1513 | tBuPh₂Si | -OMe | -OMe | =O | | -H | -** | -H | -O- | H | H | 0 |
| UBS1634 | - | -OMe | -OMe | =O | | -H | -** | -H | -OH | H | H | 0 |
| UBS3267 | - | -OMe | -OMe | =O | | -H | -** | -H | Tetrabenzoyl D-Glucosyl | H | H | 0 |
| UBS3268 | - | -OMe | -OMe | =O | | -H | -** | -H | D-Glucosyl | H | H | 0 |
| UBS3269 | - | -OMe | -OMe | =O | | -H | -** | -H | Tetrabenzoyl D-Mannosyl | H | H | 0 |
| UBS3270 | - | -OMe | -OMe | =O | | -H | -** | -H | D-Mannosyl | H | H | 0 |
| UBS3285 | - | -OMe | -OMe | =O | | -H | -** | -H | D-Galactosyl | H | H | 0 |
| UBS3327 | - | -OMe | -OMe | =O | | -H | -** | -H | D-Cellobiosyl | H | H | 0 |
| UBS3328 | - | -OMe | -OMe | =O | | -H | -** | -H | D-Isomaltosyl | H | H | 0 |
| UBS3501 | - | -OMe | -OMe | =O | | -H | -** | -H | L-Fucosyl | H | H | 0 |
| UBS3585 | - | -OMe | -OMe | =O | | -H | -** | -H | 2-acetamido-2-deoxy-D- Glucosyl | H | H | 0 |
| UBS3597 | - | -OMe | -OMe | =O | | -H | -** | -H | 2-acetamido-2-deoxy-D- Galactosyl | H | H | 0 |
| UBS3976 | - | -OMe | -OMe | =O | | -H | -** | -H | D-Lactosyl | H | H | 0 |
| UBS4066 | - | -OMe | -OMe | L-Fucosyl | -H | -H | -** | -H | L-Fucosyl | H | H | 0 |
| UBS4067 | - | -OMe | -OMe | =O | | -H | -** | -H | D-Gentiobiosyl | H | H | 0 |
| UBS4095 | - | -OMe | -OMe | =O | | -H | -** | -H | D-Maltosyl | H | H | 0 |
| UBS4104 | - | -OMe | -OMe | =O | | -H | -** | -H | 2-acetamido-2-deoxy-β-D- Glucosyl | H | H | 0 |
| UBS4109 | - | -OMe | -OMe | D-Cellobiosyl | -H | -H | -** | -H | D-Cellobiosyl | H | H | 0 |
| UBS4209 | - | -OMe | -OMe | D-Lactosyl | -H | -H | -** | -H | D-Lactosyl | H | H | 0 |
| UBS4373 | - | -OMe | -OMe | D-Isomaltosyl | -H | -H | -** | -H | D-Isomaltosyl | H | H | 0 |

| | | | | | | | | | | | | |
|---|---|---|---|---|---|---|---|---|---|---|---|---|
| * refers to fact that X₅ participates to a double bond between the carbon atoms in position 4 and 5 ** refers to fact that X₅ participates to a double bond between the carbon atoms in position 5 and 6 | | | | | | | | | | | | |

### Example 3 Effect of compounds according to the invention on cell migration

The present example illustrates the effect of the compounds UBS3270, UBS3285, UBS3327, UBS3328, UBS3501, UBS3585, UBS3597, UBS3976, UBS4066, UBS4095, UBS4104, UBS4109, UBS4209 and UBS4373 according to the invention on the migration of cancer cells.

Cells of different types of cancer, i.e. U-373 MG (Glioma), Hs578T (breast cancer), PC-3 (prostate cancer) and A549 (lung cancer) were seeded on culture flask 48 hours before the migration experiment. On the test day, cells were treated with or without compounds UBS3270, UBS3285, UBS3327, UBS3328, UBS3501, UBS3585, UBS3597, UBS3976, UBS4066, UBS4095, UBS4104, UBS4109, UBS4209 and UBS4373 in closed Falcon dishes containing a buffered medium at a controlled temperature (37.0 ± 0.1°C) for 12 or 24 hours. The compounds were administered at 4 concentrations (10⁻⁷ M to 10⁻¹⁰ M). Migration of the cells was observed by means of a CCD-camera mounted on a phase-contrast microscope. A statistical analyse of the migration, with the non-parametric Mann-Whitney test, was established for 25% - 50% of the most motile cells and for the entire cell population for UBS3270, UBS3285, UBS3327, UBS3328, UBS3501, UBS3585, UBS3597, UBS3976, UBS4066, UBS4095, UBS4104, UBS4109, UBS4209 and UBS4373 compounds. The table C below illustrates the anti-migratory effect of the compound according to the invention.

**TABLE C Anti-migratory effect of the compounds UBS3270, UBS3285, UBS3327, UBS3328, UBS3501, UBS3585, UBS3597, UBS3976 and UBS4066 on human cancer cell lines**

| Compounds | Cell lines | Max. effects | Conditions |
|---|---|---|---|
| UBS3270 | Hs578T | -27% / p < 0.001 | For 24 hours on the 50% of most motile cells, at 10⁻⁷M |
| UBS3285 | U-373 MG | - 22% / p < 0.001 | For 24 hours on the 50% of most motile cells, at 10⁻⁸ M |
| UBS3327 | A549 | - 34% / p < 0.001 | For 24 hours on the 25% of most motile cells, at 10⁻⁸ M |
| | Hs578T | - 21 % / p < 0.001 | For 24 hours on the 25% of most motile cells, at 10⁻⁷ M |
| UBS3328 | A549 | - 40% / p< 0.001 | For 24 hours on the 25% of most motile cells, at 10⁻⁷ M |
| | U-373 MG | - 27% / p< 0.001 | For 12 hours on the entire cell population, at 10⁻¹⁰ M |
| UBS3501 | U-373 MG | -38% / p = 0.01 | For 24 hours on the 25% of most motile cells, at 10⁻⁸ M |
| | PC-3 | -23% / p < 0.001 | For 12 hours on the 25% of most motile cells, at 10⁻⁷ M |
| UBS3585 | Hs578T | -27% / p < 0.001 | For 24 hours on the entire cell population, at 10⁻⁷ M |
| UBS3597 | PC-3 | -31 % / p < 0.001 | For 24 hours on the 25% of most motile cells, at 10⁻⁹ M |
| UBS3976 | PC-3 | -51 % / p < 0.001 | For 12 hours on the 25% of most motile cells, at 10⁻⁸ M |
| | U-373 MG | -46% / p < 0.001 | For 24 hours on the 25% of most motile cells, at 10⁻¹⁰ M |
| UBS4066 | Hs578T | -29% / P < 0.001 | For 12 hours on the 25% of most motile cells, at 10⁻⁹ M |
| | PC-3 | -45% / p < 0.001 | For 24 hours on the 25% of most motile cells, at 10⁻⁹ M |
| UBS4095 | U-373 MG | -27% / p < 0.001 | For 24 hours on the entire cell population, at 10⁻⁹ M |
| UBS4104 | A549 | -46 % / p < 0.001 | For 24 hours on the 25% of most motile cells, at 10⁻¹⁰ M |
| | U-373 MG | -23 % / p < 0.001 | For 12 hours on the 50% of most motile cells, at 10⁻¹⁰ M |
| UBS4109 | PC-3 | -34 % / p < 0.001 | For 12 hours on the entire cell population, at 10⁻⁹ M |
| UBS4209 | A549 | -26 % / p < 0.001 | For 12 hours on the 25% of most motile cells, at 10⁻⁸ M |
| | Hs578T | -26 % / p < 0.001 | For 24 hours on the 25% of most motile cells, at 10⁻⁷ M |
| UBS4373 | U-373 MG | -24 % / p < 0.001 | For 12 hours on the entire cell population, at 10⁻⁹ M |

In conclusion, the compounds UBS3270, UBS3285, UBS3327, UBS3328, UBS3501, UBS3585, UBS3597, UBS3976, UBS4066, UBS4095, UBS4104, UBS4109, UBS4209 and UBS4373 induced a decrease in the migration level of U-373 MG, Hs578T, PC-3 and/or A549 cancer cells at the studied concentrations, populations and times.

### Example 4 Effect of compounds according to the invention on survival of P388 bearing mouse

The compounds UBS3328 and UBS3501 were evaluated on the aggressive P388 lymphoma cancer model. When leukemic P388 cells of lymphoblastic origin (Pihl A. UICC Study Group on chemosensitivity testing of human tumors. Problems--applications--future prospects. Int J Cancer. 1986 Jan 15;37(1):1-5) are grafted subcutaneously, they develop as very biologically aggressive anaplastic lymphomas markedly metastasizing first into the liver, then in the lungs and occasionally in kidneys. The mice suffering from P388 lymphomas usually die about 2 weeks after the cell injection. The P388 lymphoma was thus used as a model representative of a clinically terminal state of cancer.

The model was performed with 5 mice per group and the compounds UBS3328 and UBS3501 were injected subcutaneously at 40 mg/kg for 4 days, two times per day, for the two first weeks post graft followed by one administration everyday, one times per day for the third week post graft.

The effect of the compounds UBS3328 and UBS3501 on the survival of the P388 cancer bearing mice is evaluated following the analysis of the T/C index. T/C index is calculated by dividing the median day of death in a treated group T by the median day of death in the control group C. T/C values of 130 % or more (i.e. a prolongation of mice survival of 30% or more) indicate a significant prolongation of survival.

The T/C index for the compounds UBS3328 and UBS3501 is respectively 135 and 141%. As a conclusion, the tested compounds show a significant effect on prolongation of survival of the P388 cancer bearing mice.

## Claims

1. A compound having the structural formula I, stereoisomers, tautomers, racemics thereof, or a pharmaceutically acceptable salt and/or solvate thereof, 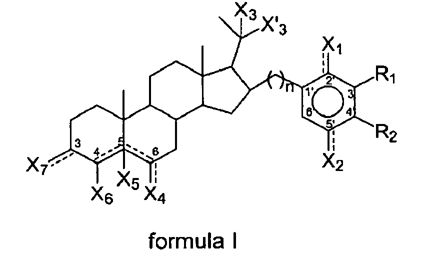 wherein X₁, X₂ are independently -OMe, R₁ and R₂ are independently hydrogen,
wherein X₃ participates together with X'₃ to an oxo functional group, or wherein X₃ and X'₃ are independently selected from the group comprising hydrogen, hydroxyl, oxyalkyl, oxycarbonyl, glucosyl, fructosyl, galactosyl, mannosyl, ribosyl, ribulosyl, xylulosyl, erythrosyl, erythrulosyl, rhamnosyl, threosyl, sorbosyl, psicosyl, tagatosyl, fucosyl, arabinosyl, altrosyl, laminaribiosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-amino-2-deoxy-mannosyl, 2-acetamido-2-deoxy-mannosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl. 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, combination thereof, deoxy derivatives thereof, hydroxyl-protected acetate derivatives thereof, amino derivatives thereof, amido derivatives thereof, thio derivatives thereof, disaccharide thereof, trisaccharide thereof, oligosaccharide and polysaccharide thereof;
wherein X₄ and X₇ are independently selected from the group comprising hydrogen, oxygen, oxo, hydroxyl, glucosyl, fructosyl, galactosyl, mannosyl, ribosyl, ribulosyl, xylulosyl, erythrosyl, erythrulosyl, rhamnosyl, threosyl, sorbosyl, psicosyl, tagatosyl, fucosyl, arabinosyl, altrosyl, laminaribiosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy galactosyl, 2-acetamido-2-deoxy-galactosyl, 2-amino-2-deoxy-mannosyl, 2-acetamido-2-deoxy-mannosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form threreof, pyranose or furanose form thereof, combination thereof, deoxy derivatives thereof, hydroxyl-protected acetate or benzoyl derivatives thereof, amino derivatives thereof, amido derivatives thereof, thio derivatives thereof, disaccharide thereof, trisaccharide thereof, oligosaccharide and polysaccharide thereof;
wherein at least one of X₃ , X'₃, X₄ and X₇ is a glycosyl moiety selected from the group as indicated above;
wherein X₄ or X₆ are hydrogen and wherein X₅ participates to a double bond between the carbon atoms in position 4 and 5 or in position 5 and 6, and
wherein n is 0.

2. A compound according to claim 1, wherein X₁ and X₂ are -OMe, wherein R₁ and R₂ are -H, wherein n is 0, wherein X₆ is -H,
and
wherein X₃ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X'₃ is selected from the group comprising hydrogen, alkyl or aralkyl, wherein X₄ is hydrogen, wherein X₅ participates to a double bond between the carbon atoms in position 5 and 6, and wherein X₇ is selected from the group comprising hydrogen, oxygen, hydroxyl or oxo; or
wherein X₃ is selected from the group comprising hydrogen, hydroxyl, oxyalkyl or oxycarbonyl, wherein X'₃ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-Amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is hydrogen, wherein X₅ participates to a double bond between the carbon atoms in position 5 and 6, and wherein X₇ is selected from the group comprising hydrogen, oxygen, hydroxyl or oxo; or
wherein X₃ participates together with X'₃ to an oxo functional group, wherein X₄ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ participates to a double bond between the carbon atoms in position 4 and 5, wherein X₆ is -H, and wherein X₇ is selected from the group comprising hydrogen, oxygen, hydroxyl , alkyloxy or oxo; or
wherein X₃ participates together with X'₃ to an oxo functional group, wherein X₄ is hydrogen, wherein X₅ participates to a double bond between the carbon atoms in position 5 and 6, and wherein X₇ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-(3-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof; or
wherein X₃ or X'₃ are independently selected from the group comprising hydrogen or glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-Amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3'-Fucosyl-D-Lactosyl, 3'-Fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ and X₆ participates to a double bond between the carbon atoms in position 4 and 5, wherein X₇ is selected from the group comprising hydrogen, oxygen, hydroxyl, alkyloxy or oxo, and wherein at least one of X₃ and X'₃ is a glycosyl moiety selected from the group as indicated above; or
wherein X₃ or X'₃ are independently selected from the group comprising hydrogen, glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deaxy-4-O-β-D-galactosyl-D-glucosyl, 6'-N-acetylglucosaminyllactasyl, 2-acetamido-2-deaxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is hydrogen, wherein X₅ and X₆ participates to a double bond between the carbon atoms in position 5 and 6, wherein X₇ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, and wherein at least one of X₃ and X'₃ is a glycosyl moiety selected from the group as indicated above; or
wherein X₃ participates together with X'₃ to an oxo functional group or are independently selected from the group comprising hydrogen, hydroxyl, alkyloxy, wherein X₄ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ and X₆ participates to a double bond between the carbon atoms in position 4 and 5, and wherein X₇ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof; or
wherein X₃ or X'₃ are independently selected from the group comprising hydrogen, glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-b-D-galactosyl-D-glucosyl, 2-amino-2-deoxy-4-O-b-D-galactosyl-D-glucosyl, 6'-N-acetylglucosaminyllactosyl, 2-acetamido-2-deoxy-3-O-a-L-fucosyl-D-glucosyl, 6-O(2-acetamido-2-deoxy-b-D-glucosyl)-D-galactosyl, 2-acetamido-2-deoxy-3-O-b-D-galactosyl-D-glucosyl, 2'-acetamido-2'-deoxy-3-O-b-D-glucosyl-D-galactosyl, 3-fucosyl-D-lactosyl, 3-fucosyl-2-acetamido-2-deoxy-4-O-b-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-b-D-galactosyl-D-glucosyl, 2-amino-2-deoxy-4-O-b-D-galactosyl-D-glucosyl, 6'-N-acetylglucosaminyllactosyl, 2-acetamido-2-deoxy-3-O-a-L-fucosyl-D-glucosyl, 6-O(2-acetamido-2-deoxy-b-D-glucosyl)-D-galactosyl, 2-acetamido-2-deoxy-3-O-b-D-galactosyl-D-glucosyl, 2'-acetamido-2'-deoxy-3-O-b-D-glucosyl-D-galactosyl, 3-fucosyl-D-lactosyl, 3-fucosyl-2-acetamido-2-deoxy-4-O-b-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ and X₆ participates to a double bond between the carbon atoms in position 4 and 5, wherein X₇ is selected from the group comprising glucosyl, fructosyl, galactosyl, mannosyl, fucosyl, isomaltosyl, maltosyl, lactosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-b-D-galactosyl-D-glucosyl, 2-amino-2-deoxy-4-O-b-D-galactosyl-D-glucosyl, 6'-N-acetylglucosaminyllactosyl, 2-acetamido-2-deoxy-3-O-a-L-fucosyl-D-glucosyl, 6-O(2-acetamido-2-deoxy-b-D-glucosyl)-D-galactosyl, 2-acetamido-2-deoxy-3-O-b-D-galactosyl-D-glucosyl, 2'-acetamido-2'-deoxy-3-O-b-D-glucosyl-D-galactosyl, 3-fucosyl-D-lactosyl, 3-fucosyl-2-acetamido-2-deoxy-4-O-b-D-galactosyl-D-glucosyl, L or D isomers thereof, α or β form thereof, pyranuronic or furanuronic form thereof, pyranose or furanose form thereof, a disaccharide or a trisaccharide thereof, wherein at least one of X₃ and X'₃ is a glycosyl moiety selected from the group as indicated above.

3. A compound according to claim 1 or 2 , stereoisomers, tautomers, racemics, thereof, or a pharmaceutically acceptable salt and/or solvate thereof, wherein X₁ and X₂ are - OMe, wherein R₁ and R₂ are -H, wherein X₃ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X'₃ is selected from the group comprising hydrogen, alkyl or aralkyl, wherein X₄ is hydrogen, wherein X₅ participates to a double bond between the carbon atoms in position 5 and 6, wherein X₆ is -H, wherein X₇ is selected from the group comprising hydrogen, oxygen, hydroxyl or oxo, and wherein n is 0.

4. A compound according to claim 1 or 2, stereoisomers, tautomers, racemics, thereof, or a pharmaceutically acceptable salt and/or solvate thereof, wherein X₁ and X₂ are - OMe, wherein R₁ and R₂ are -H, wherein X₃ is selected from the group comprising hydrogen, hydroxyl, oxyalkyl or oxycarbonyl, wherein X'₃ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-Amino-2-deoxy-4-O-β-D-galactosyl-D-glucosyl, 6'-N-acetylglucosaminyllactosyl, 2-acetamido-2-deoxy-3-O-α-L-fucosyl-D-gfucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is hydrogen, wherein X₅ participates to a double bond between the carbon atoms in position 5 and 6, wherein X₆ is -H, wherein X₇ is selected from the group comprising hydrogen, oxygen, hydroxyl or oxo, and wherein n is 0.

5. A compound according to claim 1 or 2, stereoisomers, tautomers, racemics, thereof, or a pharmaceutically acceptable salt and/or solvate thereof, wherein X₁ and X₂ are - OMe, wherein R₁ and R₂ are -H, wherein X₃ participates together with X'₃ to an oxo functional group, wherein X₄ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ participates to a double bond between the carbon atoms in position 4 and 5, wherein X₆ is -H, wherein X₇ is selected from the group comprising hydrogen, oxygen, hydroxyl , alkyloxy or oxo, and wherein n is 0.

6. A compound according to claim 1 or 2, stereoisomers, tautomers, racemics, thereof, or a pharmaceutically acceptable salt and/or solvate thereof, wherein X₁ and X₂ are-OMe, wherein R₁ and R₂ are -H, wherein X₃ participates together with X'₃ to an oxo functional group, wherein X₄ is hydrogen, wherein X₅ participates to a double bond between the carbon atoms in position 5 and 6, wherein X₆ is -H, wherein X₇ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof; and wherein n is 0.

7. A compound according to any of claim 1 or 2, stereoisomers, tautomers, racemics, thereof, or a pharmaceutically acceptable salt and/or solvate thereof, wherein X₁ and X₂ are -OMe, wherein R₁ and R₂ are -H, wherein X₃ or X'₃ are independently selected from the group comprising hydrogen or glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-Amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3'-Fucosyl-D-Lactosyl, 3'-Fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is selected from the group comprising glucosyl, fructosyl, galactosyl, mannosyl, fucosyl, isomaltosyl, isomaltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-0(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ and X₆ participates to a double bond between the carbon atoms in position 4 and 5, wherein X₆ is -H, wherein X₇ is selected from the group comprising hydrogen, oxygen, hydroxyl, alkyloxy or oxo, wherein at least one of X₃ and X'₃ is a glycosyl moiety selected from the group as indicated above and wherein n is 0.

8. A compound according to claim 1 or 2, stereoisomers, tautomers, racemics, thereof, or a pharmaceutically acceptable salt and/or solvate thereof, wherein X₁ and X₂ are - OMe, wherein R₁ and R₂ are -H, wherein X₃ or X'₃ are independently selected from the group comprising hydrogen, glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is hydrogen, wherein X₅ and X₆ participates to a double bond between the carbon atoms in position 5 and 6, wherein X₆ is -H, wherein X₇ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein at least one of X₃ and X'₃ is a glycosyl moiety selected from the group as indicated above and wherein n is 0.

9. A compound according to claim 1 or 2, stereoisomers, tautomers, racemics, thereof, or a pharmaceutically acceptable salt and/or solvate thereof, wherein X₁ and X₂ are - OMe, wherein R₁ and R₂ are -H, wherein X₃ participates together with X'₃ to an oxo functional group or are independently selected from the group comprising hydrogen, hydroxyl, alkyloxy, wherein X₄ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyl-D-glucosyl, 2-amino-2-deoxy-4-O-β-D-galactosyl-D-glucosyl, 6'-N-acetylglucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ and X₆ participates to a double bond between the carbon atoms in position 4 and 5, wherein X₆ is -H, wherein X₇ is selected from the group comprising glucosyl, fructosyl, galactosyl, mannosyl, fucosyl, isomattosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-β-D-galactosyt-D-glucosyl, 2-amino-2-deoxy-4-O-β-D-galactosyl-D-glucosyl, 6'-N-acetylgfucosaminyllactosyl, 2-acetamido-2-deoxy-3-O-α-L-fucosyl-D-glucosyl, 6-O(2-acetamido-2-deoxy-β-D-glucosyl)-D-galactosyl, 2-acetamido-2-deoxy-3-O-β-D-galactosyl-D-glucosyl, 2'-acetamido-2'-deoxy-3-O-β-D-glucosyl-D-galactosyl, 3-fucosyl-D-lactosyl, 3-fucosyl-2-acetamido-2-deoxy-4-O-β-D-galactosyl-D-glucosyl, L or D isomers thereof, α or β form thereof, pyranuronic or furanuronic form thereof, pyranose or furanose form thereof, a disaccharide or a trisaccharide thereof, and wherein n is 0.

10. A compound according to claim 1 or 2, stereoisomers, tautomers, racemics, thereof, or a pharmaceutically acceptable salt and/or solvate thereof, wherein X₁ and X₂ are - OMe, wherein R₁ and R₂ are -H, wherein X₃ or X'₃ are independently selected from the group comprising hydrogen, glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-b-D-galactosyl-D-glucosyl, 2-amino-2-deoxy-4-O-b-D-galactosyl-D-glucosyl, 6'-N-acetylglucosaminyllactosyl, 2-acetamido-2-deoxy-3-O-a-L-fucosyl-D-glucosyl, 6-O(2-acetamido-2-deoxy-b-D-glucosyl)-D-galactosyl, 2-acetamido-2-deoxy-3-O-b-D-galactosyl-D-glucosyl, 2'-acetamido-2'-deoxy-3-O-b-D-glucosyl-D-galactosyl, 3-fucosyl-D-lactosyl, 3-fucosyl-2-acetamido-2-deoxy-4-O-b-D-galactosyl-D-glucosyl, L or D isomers thereof, α or β form thereof, pyranuronic or furanuronic form thereof, pyranose or furanose form thereof, a disaccharide or a trisaccharide thereof, wherein X₄ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy-glucosyl, 2-acetamido-2-deoxy-glucosyl, 2-amino-2-deoxy-galactosyl, 2-acetamido-2-deoxy-galactosyl, 2-acetamido-2-deoxy-4-O-b-D-galactosyl-D-glucosyl, 2-amino-2-deoxy-4-O-b-D-galactosyl-D-glucosyl, 6'-N-acetylglucosaminyllactosyl, 2-acetamido-2-deoxy-3-O-a-L-fucosyl-D-glucosyl, 6-O(2-acetamido-2-deoxy-b-D-glucosyl)-D-galactosyl, 2-acetamido-2-deoxy-3-O-b-D-galactosyl-D-glucosyl, 2'-acetamido-2'-deoxy-3-O-b-D-glucosyl-D-gaiactosyl, 3-fucosyl-D-lactosyl, 3-fucosyl-2-acetamido-2-deoxy-4-O-b-D-galactosyl-D-glucosyl, L or D isomers thereof, α or β form thereof, pyranuronic or furanuronic form thereof, pyranose or furanose form thereof, a disaccharide or a trisaccharide thereof, wherein X₅ and X₆ participates to a double bond between the carbon atoms in position 4 and 5,
wherein X₆ is -H, wherein X₇ is selected from the group comprising glucosyl, fructosyl, galactosyl, mannosyl, fucosyl, isomaltosyl, maltosyl, cellobiosyl, gentiobiosyl, melibiosyl, palatinosyl, lactulosyl, 3-mannobiosyl, 6-mannobiosyl, 3-galactobiosyl, 4-galactobiosyl, maltotriosyl, maltotetraosyl, 2-amino-2-deoxy glucosyl, 2-acetamido-2-deoxy-glucosyl, 2-amino-2-deoxy-galactosyl, 2-acetamido-2-deoxy-galactosyl, 2-acetamido-2-deoxy-4-O-b-D-galactosyl-D-glucosyl, 2-amino-2-deoxy-4-O-b-D-galactosyl-D-glucosyl, 6'-N-acetylglucosaminyllactosyl, 2-acetamido-2-deoxy-3-O-a-L-fucosyl-D-glucosyl, 6-O(2-acetamido-2-deoxy-b-D-glucosyl)-D-galactosyl, 2-acetamido-2-deoxy-3-O-b-D-galactosyl-D-glucosyl, 2'-acetamido-2'-deoxy-3-O-b-D-glucosyl-D-galactosyl, 3-fucosyl-D-lactosyl, 3-fucosyl-2-acetamido-2-deoxy-4-O-b-D-galactosyl-D-glucosyl, L or D isomers thereof, α or β form thereof, pyranuronic or furanuronic form thereof, pyranose or furanose form thereof, a disaccharide or a trisaccharide thereof, wherein at least one of X₃ and X'₃ is a glycosyl moiety selected from the group as indicated above and wherein n is 0.

11. Compound of formula I, stereoisomers, tautomers, racemics thereof, or a pharmaceutically acceptable salt and/or solvate thereof, wherein X₁ and X₂ are -OMe, R₁ and R₂ are -H, X₃ participates together with X'₃ to an oxo functional group, X₄ and X₆ are -H, and n is 0, X₅ participates to a double bond between the carbon atoms in position 5 and 6, and X₇ is selected from D-Glucosyl, D-Mannosyl, D-Galactosyl, D-Cellobiosyl, D-isomaltosyl, L-Fucosyl, D-Lactosyl, D-Gentiobiosyl, D-Maltosyl, 2-acetamido-2-deoxy-D-Glucosyl, 2-acetamido-2-deoxy-D-Galactosyl, xylopyranosyl, N-acetyl-lactosaminyl, 2-acetamido-2-deoxy-4-O-β-D-Galactosyl-D-Glucosyl, 3-fucosyl-2-acetamido-2-deoxy-4-O-β-D-galactosyl-D-glucosyl, 3-fucosyl-D-lactosyl, D-Melibiosyl, D-Maltotrisosyl, D-Lactulosyl, or D-palatinosy ; or
wherein X₁ and X₂ are -OMe, R₁ and R₂ are -H, X₃ participates together with X'₃ to an oxo functional group, X₆ are -H, n is 0, X₅ participates to a double bond between the carbon atoms in position 4 and 5, and X₄ and X₇ are the same and are selected from L-Fucosyl, D-Cellobiosyl, D-Isomaltosyl, D-Gentiobiosyl, D-Maltosyl, D-Lactosyl, N-acetyl-lactosaminyl, 2-acetamido-2-deoxy-4-O-β-D-Galactosyl-D-Glucosyl, 3-fucosyl-2-acetamido-2-deoxy-4-O-β-D-galactosyl-D-glucosyl, 3-fucosyl-D-lactosyl, D-Melibiosyl, D-Maltotrisosyl, D-Lactulosyl, or D-palatinosy ; or
wherein X₁ and X₂ are -OMe, R₁ and R₂ are -H, X'₃ is H, and X₆ is -H, and n is 0, X₅ participates to a double bond between the carbon atoms in position 4 and 5, X₃, X₄ and X₇ are the same and are selected from L-Fucosyl, D-Cellobiosyl, D-Isomaltosyl, D-Gentiobiosyl, D-Maltosyl, D-Lactosyl, N-acetyl-lactosaminyl, 2-acetamido-2-deoxy-4-O-β-D-Galactosyl-D-Glucosyl, 3-fucosyl-2-acetamido-2-deoxy-4-O-β-D-galactosyl-D-glucosyl, 3-fucosyl-D-lactosyl, D-Melibiosyl, D-Maltotrisosyl, D-Lactulosyl, or D-palatinosy ; or
wherein X₁ and X₂ are -OMe, R₁ and R₂ are -H, X'₃ is H, and X₆ is -H, and n is 0, X₅ participates to a double bond between the carbon atoms in position 4 and 5, X₇ is hydroxyl, X₃ and X₄ are the same and are selected from L-Fucosyl, D-Cellobiosyl, D-lsomaltosyl, D-Gentiobiosyl, D-Maltosyl, D-Lactosyl, N-acetyl-lactosaminyl, 2-acetamido-2-deoxy-4-o-β-D-Galactosyl-D-Glucosyl, 3-fucosyl-2-acetamido-2-deoxy-4-O-β-D-galactosyl-D-glucosyl, 3-fucosyl-D-lactosyl, D-Melibiosyl, D-Maltotrisosyl, D-Lactulosyl, or D-palatinosy ; or
wherein X₁ and X₂ are -OMe, R₁ and R₂ are -H, X'₃ is H, and X₆ is -H, and n is 0, X₅ participates to a double bond between the carbon atoms in position 5 and 6, X₄ is -H, X₃ and X₇ are the same and are selected from L-Fucosyl, D-Cellobiosyl, D-Isomaltosyl, D-Gentiobiosyl, D-Maltosyl, D-Lactosyl, N-acetyl-lactosaminyl, 2-acetamido-2-deoxy-4-O-β-D-Galactosyl-D-Glucosyl, 3-fucosyl-2-acetamido-2-deoxy-4-O-β-D-galactosyl-D-glucosyl, 3-fucosyl-D-lactosyl, D-Melibiosyl, D-Maltotrisosyl, D-Lactulosyl, or D-palatinosy ; or
wherein X₁ and X₂ are -OMe, R₁ and R₂ are -H, X₃ participates together with X'₃ to an oxo functional group, X₇ is an oxo group and X₆ is -H, and n is 0, X₅ participates to a double bond between the carbon atoms in position 4 and 5, and X₄ is selected from β-D-glucopyranosyl, galactopyranosyl, mannopyranosyl, xylopyranosyl, cellobiosyl, lactosyl, glucofuranosyl, maltosyl, or gentiobiosyl; or
wherein X₁ and X₂ are -OMe, R₁ and R₂ are -H, X₃ is L-fucosyl, X'₃ is H, and X₆ is -H, and n is 0, X₅ participates to a double bond between the carbon atoms in position 5 and 6, X₄ is H, and X₇ is selected from L-fucosyl, D-cellobiosyl, D-isomaltosyl, D-gentiobiosyl, D-maltosyl, or 2-acetamido-2-deoxy-4-O-β-D-glactosyl-D-glucosyl; or
wherein X₁ and X₂ are -OMe, R₁ and R₂ are -H, X₃ is D-cellobiosyl, X'₃ is H, and X₆ is -H, and n is 0, X₅ participates to a double bond between the carbon atoms in position 5 and 6, X₄ is H, and X₇ is selected from D-cellobiosyl, D-lactosyl, D-isomaltosyl, L-fucosyl, D-gentiobiosyl, D-maltosyl, or 2-acetamido-2-deoxy-4-O-β-D-glactosyl-D-glucosyl; or
wherein X₁ and X₂ are -OMe, R₁ and R₂ are -H, X₃ is D-gentiobiosyl, X'₃ is H, and X₆ is -H, and n is 0, X₅ participates to a double bond between the carbon atoms in position 5 and 6, X₄ is H, and X₇ is selected from D-gentiobiosyl, D-lactosyl, D-cellobiosyl, D-isomaltosyl, L-fucosyl, D-maltosyl, or 2-acetamido-2-deoxy-4-O-β-D-glactosyl-D-glucosyl; or
wherein X₁ and X₂ are -OMe, R₁ and R₂ are -H, X₃ is D-lactosyl, X'₃ is H, and X₆ is -H, and n is 0, X₅ participates to a double bond between the carbon atoms in position 5 and 6, X₄ is H, and X₇ is selected from hydroxyl, D-lactosyl, D-cellobiosyl, D-isomaltosyl, L-fucosyl, D-gentiobiosyl, D-maltosyl, or 2-acetamido-2-deoxy-4-O-β-D-glactosyl-D-glucosyl; or
wherein X₁ and X₂ are -OMe, R₁ and R₂ are -H, X₃ is D-isomaltosyl, X'₃ is H, and X₆ is -H, and n is 0, X₅ participates to a double bond between the carbon atoms in position 5 and 6, X₄ is H, and X₇ is selected from D-isomaltosyl, D-lactosyl, D-cellobiosyl, L-fucosyl, D-gentiobiosyl, D-maltosyl, or 2-acetamido-2-deoxy-4-O-β-D-glactosyl-D-glucosyl; or
wherein X₁ and X₂ are -OMe, R₁ and R₂ are -H, X₃ is D-maltosyl, X'₃ is H, and X₆ is - H, and n is 0, X₅ participates to a double bond between the carbon atoms in position 5 and 6, X₄ is H, and X₇ is selected from D-maltosyl, D-lactosyl, D-cellobiosyl, D-isomaltosyl, L-fucosyl, D-gentiobiosyl, or 2-acetamido-2-deoxy-4-O-β-D-glactosyl-D-glucosyl; or
wherein X₁ and X₂ are -OMe, R₁ and R₂ are -H, X₃ is D-2-acetamido-2-deoxy-4-O-b-D-glactosyl-D-glucosyl, X'₃ is H, and X₆ is -H, and n is 0, X₅ participates to a double bond between the carbon atoms in position 5 and 6, X₄ is H, and X₇ is selected from 2-acetamido-2-deoxy-4-O-β-D-glactosyl-D-glucosyl, D-maltosyl, D-lactosyl, D-cellobiosyl, D-isomaltosyl, L-fucosyl, or D-gentiobiosyl.

12. Method for synthesizing a compound having the structural formula I 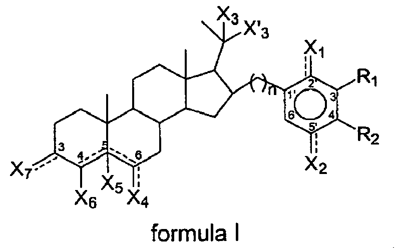 wherein X₁, X₂, X₃, X₄, X₅, X₆, X₇, R₁, R₂ and n are independently selected from the group as indicated in any of claims 1 to 10, said method comprising the steps of
a) providing a starting material having the structural formula IV, 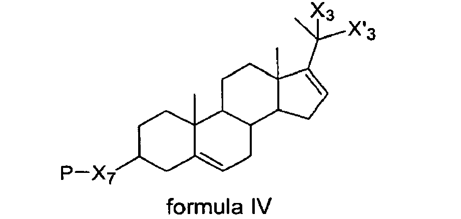 wherein X₃ participates together with X'₃ to an oxo functional group, or wherein X₃ and X'₃ are independently selected from the group comprising hydrogen, hydroxyl, sulfur, oxyalkyl, oxycarbonyl, alkyl, Het¹alkyl, alkyloxycarbonyl, alkenyl, alkynyl, aminoalkyl, aminoacyl, alkylcarbonylamino, alkylthiocarbonylamino, Het¹, optionally substituted by one or more substituents independently selected from the group comprising alkyl, aralkyl, aryl, Het¹, Het², cycloalkyl, alkyloxy, alkyloxycarbonyl, carboxyl, aminocarbonyl, mono- or di(alkyl)aminocarbonyl, aminosulfonyl, alkylS(=O)t, hydroxy, cyano, halogen or amino optionally mono- or disubstituted wherein the substituents are independently selected from the group comprising alkyl, aryl, aralkyl, aryloxy, arylamino, arylthio, aryloxyalkyl, arylaminoalkyl, aralkoxy, alkylthio, alkoxy, aryloxyalkoxy, aylaminoalkoxy, aralkylamino, aryloxyalkylamino, arylaminoalkylamino, arylthioalkoxy, arylthioalkylamino, aralkylthio, aryloxyalkylthio, arylaminoalkylthio, arylthioalkylthio, alkylamino, cycloalkyl and cycloalkylalkyl;
wherein X₇ is selected from the group comprising hydrogen, oxygen, halogen, oxo, carbonyl, thiocarbonyl, hydroxyl, alkyl, aryl, Het¹, Het¹ alkyl, Het¹ aryl, alkenyl, alkynyl, hydroxyalkyl, hydroxycarbonyl, hydroxycarbonylalkyl, hydroxycarbonylaryl, hydroxycarbonyloxyalkyl optionally substituted by one or more substituents independently selected from the group comprising alkyl, aralkyl, aryl, Het¹, Het², cycloalkyl, alkyloxy, alkyloxycarbonyl, carboxyl, aminocarbonyl, mono- or di(alkyl)aminocarbonyl, aminosulfonyl, alkylS(=O)t, hydroxy, cyano, halogen or amino optionally mono- or disubstituted wherein the substituents are independently selected from the group comprising alkyl, aryl, aralkyl, aryloxy, arylamino, arylthio, aryloxyalkyl, arylaminoalkyl, aralkoxy, alkylthio, alkoxy, aryloxyalkoxy, aylaminoalkoxy, aralkylamino, aryloxyalkylamino, arylaminoalkylamino, arylthioalkoxy, arylthioalkylamino, aralkylthio, aryloxyalkylthio, arylaminoalkylthio, arylthioalkylthio, alkylamino, cycloalkyl and cycloalkylalkyl; and wherein P is a protecting group,
b) effecting reaction between the compound of step a) with an organometallic compound having the structural formula V 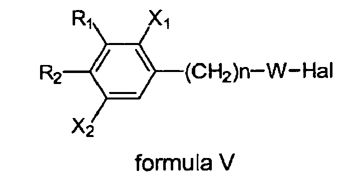 wherein X₁, X₂, R₁, R₂ and n are independently selected from the group as indicated in any of claims 1 to 10, wherein W is a metal or a combination of metals and wherein Hal is a halogen atom,
to result in an intermediate having the structural formula III' 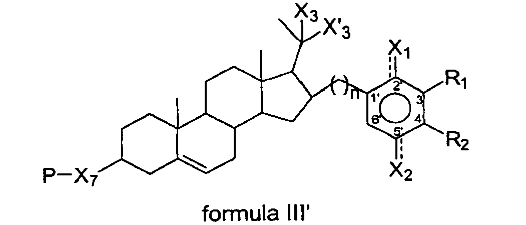 wherein X₁, X₂, R₁, R₂ and n are independently selected from the group as indicated in any of claims 1 to 10, wherein X₃, X'₃, X₇ are independently selected from the group as indicated in step a) and wherein P is a protecting group,
c) effecting reaction between the compound of step b) with an organometallic compound having the structural formula VI
Hal-W-X'₃ formula VI
wherein X'₃ is selected from the group as indicated in step a), wherein W is a metal or a combination of metals, and wherein Hal is a halogen atom,
to result in an intermediate having the structural formula III 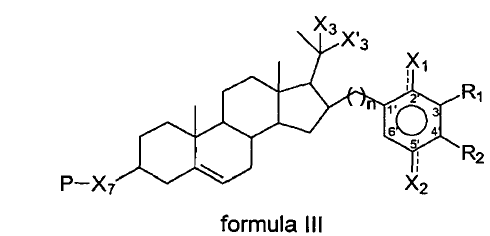 wherein X₁, X₂, R₁, R₂ and n are independently selected from the group as indicated in any of claims 1 to 10, wherein X₃, X'₃, X₇ are independently selected from the group as indicated in step a), wherein P is a protecting group,
d) deprotecting the X₇ group of the compound obtained in step c) to form an compound having the structural formula li 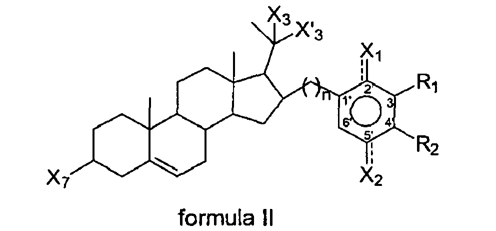 wherein X₁, X₂, R₁, R₂ and n are independently selected from the group as indicated in any of claims 1 to 10, wherein X₃, X'₃, X₇ are independently selected from the group as indicated in step a), and
e) coupling an O-protected glycosyl or non-protected glycosyl to form a compound of formula IIB wherein X₁, X₂, R₁, R₂ and n are independently selected from the group as indicated in any of claims 1 to 10, wherein X₃ and X'₃ are independently selected from the group as indicated in step a), and X₇ is an O-protected glycosyl or a non-protected glycosyl, and
f) deprotecting the O-protected groups of glycosyl to form a compound of formula IB
wherein X₁, X₂, X₄, X₅, X₆, R₁, R₂ and n are independently selected from the group as indicated in any of claims 1 to 10, wherein X₃, X'₃ are independently selected from the group as indicated in step a), and X₇ is selected from the group comprising glycosyl, thio derivatives thereof, amino derivatives thereof, amido derivatives thereof, hydroxyl-protected derivatives thereof.

13. Method according to claim 11, wherein step e) consists of reacting the compound of step d) with an oxidizing reagent to form an intermediate and reducing said intermediate with a reducing reagent to result in another intermediate having the structural formula I wherein X₁, X₂, R₁, R₂ and n are independently selected from the group as indicated in any of claims 1 to 10, and X₃ or X'₃, and X₄ and X₇ are hydroxyl and continuing the reaction with steps e) and f) according to claim 11 to form a glycosylated steroid compound of structural formula I.

14. Method according to claim 11, wherein step c) consists of reacting the compound of step b) with an O-protected glycosyl or non-protected glycosyl to result in an intermediate having the structural formula III wherein X₁, X₂, R₁, R₂ and n are independently selected from the group as indicated in any of claims 1 to 10, wherein X₃, X₇ are independently selected from the group as indicated in step a) of claim 11, wherein P is a protecting group, and wherein X₃ or X'₃ is an O-protected glycosyl or a non protected glycosyl and continuing the reaction with steps d), e) and f) according to claim 11 to form a glycosylated steroid compound of structural formula I.

15. A compound of structural formula:

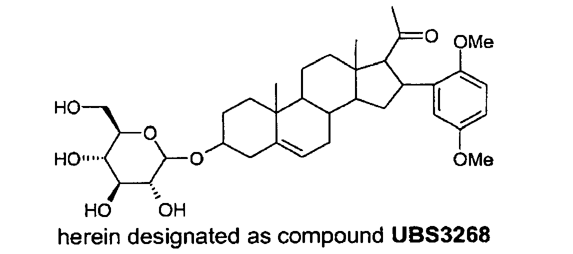

16. A compound of structural formula:

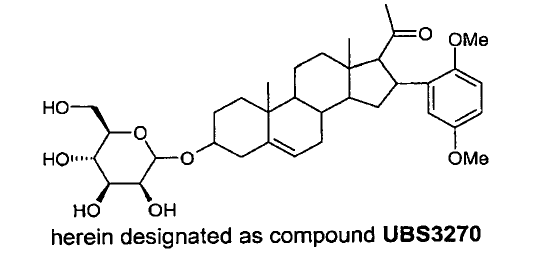

17. A compound of structural formula:

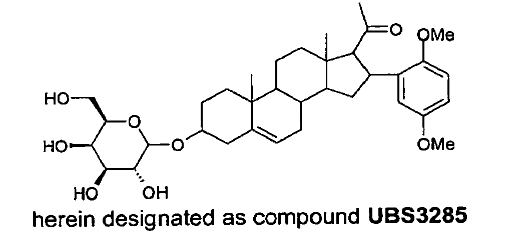

18. A compound of structural formula:

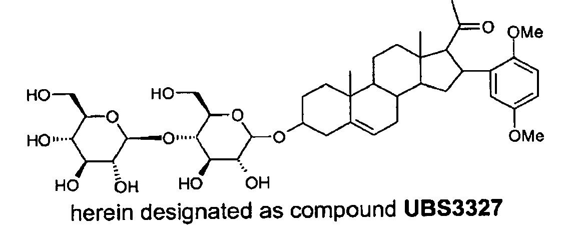

19. A compound of structural formula:

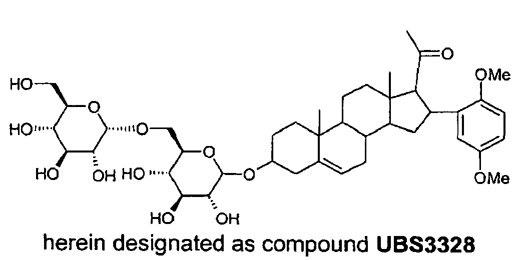

20. A compound of structural formula:

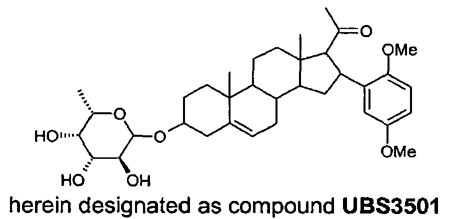

21. A compound of structural formula:

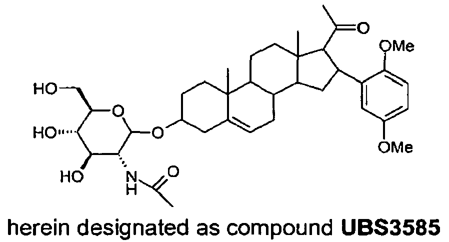

22. A compound of structural formula:

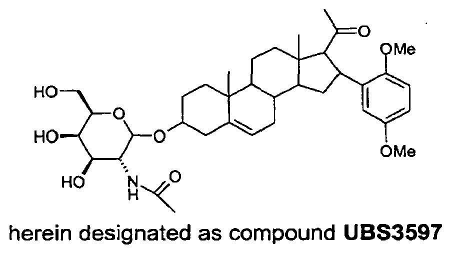

23. A compound of structural formula:

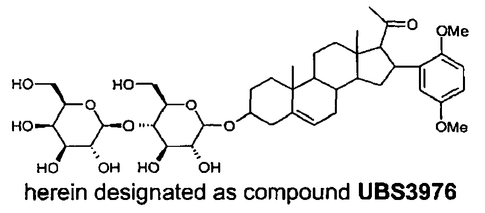

24. A compound of structural formula:

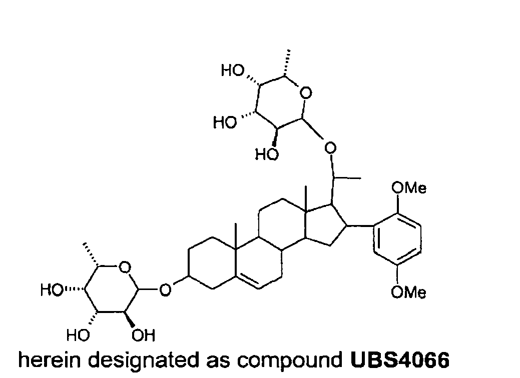

25. A compound of structural formula:

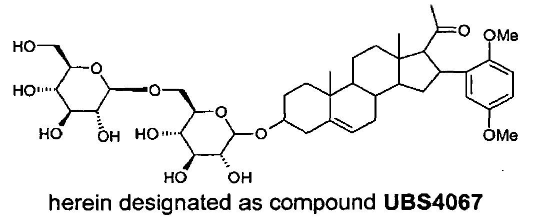

26. A compound of structural formula:

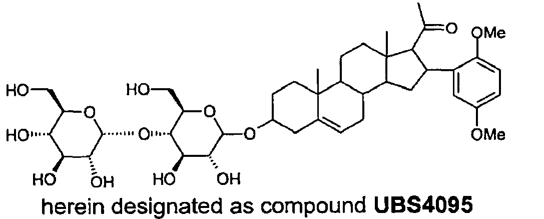

27. A compound of structural formula:

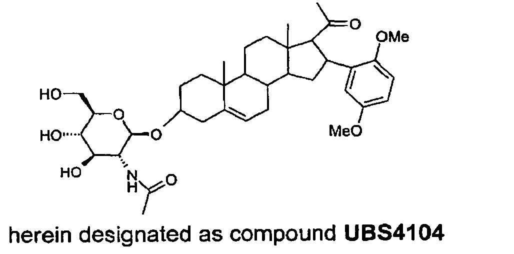

28. A compound of structural formula:

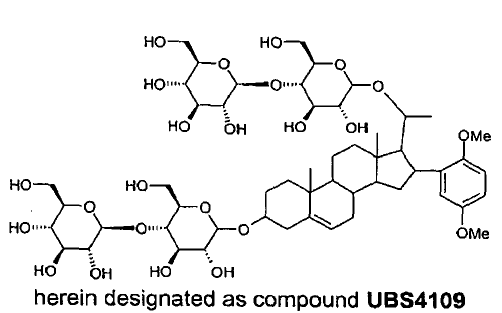

29. A compound of structural formula:

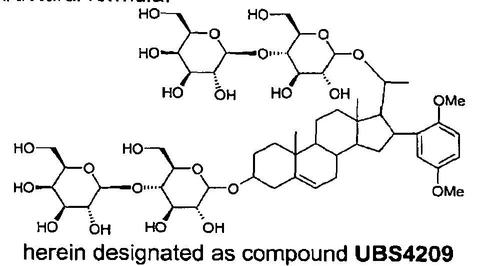

30. A compound of structural formula:

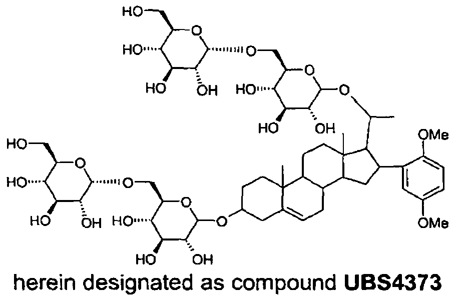

31. A compound according to any of claims 1 to 10 and 14 to 29 for use as a medicament.

32. A compound according to any of claims 1 to 10 and 14 to 29 for use as an anti-migratory agent.

33. Use of a compound according to any of claims 1 to 10 and 14 to 29 for the preparation of a medicament for treating cancer.

34. A pharmaceutical composition comprising a pharmaceutically acceptable excipient and a therapeutically effective amount of a compound according to any of claims 1 to 10 and 14 to 29.

35. Use of a pharmaceutical composition according to claim 33 for the preparation of a medicament for the treatment of cancer.

## Patentansprüche

1. Verbindung nach der Strukturformel I, deren Stereoisomere, Tautomere, Racemate, oder ein pharmazeutisch unbedenkliches Salz und/oder Solvat derselben, 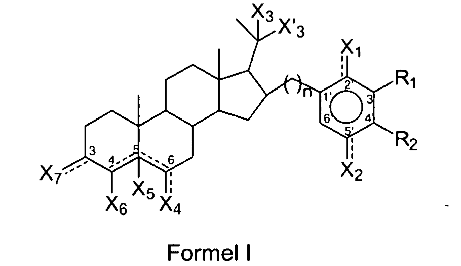 wobei X₁, X₂ unabhängig voneinander für -OMe stehen, sowie R₁ und R₂ unabhängig voneinander für Wasserstoff stehen,
wobei X₃ zusammen mit X'₃ an einer funktionellen Oxogruppe beteiligt ist, oder
wobei X₃ und X'₃ unabhängig voneinander aus der Gruppe gewählt sind, die Wasserstoff, Hydroxyl, Oxyalkyl, Oxycarbonyl, Glucosyl, Fructosyl, Galactosyl, Mannosyl, Ribosyl, Ribulosyl, Xylulosyl, Erythrosyl, Erythrulosyl, Rhamnosyl, Threosyl, Sorbosyl, Psicosyl, Tagatosyl, Fucosyl, Arabinosyl, Altrosyl, Laminaribiosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Amino-2-desoxy-mannosyl, 2-Acetamido-2-desoxy-mannosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-a-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, eine Kombination derselben, Desoxyderivate derselben, hydroxylgeschützte Acetatderivate derselben, Aminoderivate derselben, Amidoderivate derselben, Thioderivate derselben, Disaccharide derselben, Trisaccharide derselben, Oligosaccharide und Polysaccharide derselben umfasst;
wobei X₄ und X₇ unabhängig voneinander aus der Gruppe gewählt sind, die Wasserstoff, Sauerstoff, Oxo, Hydroxyl, Glucosyl, Fructosyl, Galactosyl, Mannosyl, Ribosyl, Ribulosyl, Xylulosyl, Erythrosyl, Erythrulosyl, Rhamnosyl, Threosyl, Sorbosyl, Psicosyl, Tagatosyl, Fucosyl, Arabinosyl, Altrosyl, Laminaribiosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy galactosyl, 2-Acetamido-2-desoxy-galactosyl, 2-Amino-2-desoxy-mannosyl, 2-Acetamido-2-desoxy-mannosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, eine Kombination derselben, Desoxyderivate derselben, hydroxylgeschützte Acetat- oder Benzoylderivate derselben, Aminoderivate derselben, Amidoderivate derselben, Thioderivate derselben, Disaccharide derselben, Trisaccharide derselben, Oligosaccharide und Polysaccharide derselben umfasst;
wobei es sich bei mindestens einer der Gruppen X₃, X'₃, X₄ und X₇ um eine Glycosyleinheit, die aus der Gruppe gemäß den obigen Angaben gewählt ist, handelt;
wobei X₄ oder X₆ für Wasserstoff stehen und wobei X₅ an einer Doppelbindung zwischen den Kohlenstoffatomen in Position 4 und 5 oder in Position 5 und 6 beteiligt ist, und
wobei den Wert 0 annimmt.

2. Verbindung gemäß dem Anspruch 1, wobei X₁ und X₂ für -OMe stehen, wobei R₁ und R₂ für -H stehen, wobei n den Wert 0 annimmt und wobei X₆ für -H steht,
und
wobei X₃ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy glucosyl, 2-Acetamido-2-desoxy-glucosyl, 2-Amino-2-desoxy galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X'₃ aus der Gruppe gewählt ist, die Wasserstoff, Alkyl oder Aralkyl umfasst, wobei X₄ für Wasserstoff steht, wobei X₅ an einer Doppelbindung zwischen den Kohlenstoffatomen in Position 5 und 6 beteiligt ist, und wobei X₇ aus der Gruppe gewählt ist, die Wasserstoff, Sauerstoff, Hydroxyl oder Oxo umfasst; oder
wobei X₃ aus der Gruppe gewählt ist, die Wasserstoff, Hydroxyl, Oxyalkyl oder Oxycarbonyl umfasst, wobei X'₃ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ für Wasserstoff steht, wobei X₅ an einer Doppelbindung zwischen den Kohlenstoffatomen in Position 5 und 6 beteiligt ist, und wobei X₇ aus der Gruppe gewählt ist, die Wasserstoff, Sauerstoff, Hydroxyl oder Oxo umfasst; oder
wobei X₃ zusammen mit X'₃ an einer funktionellen Oxogruppe beteiligt ist, wobei X₄ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ an einer Doppelbindung zwischen den Kohlenstoffatomen in Position 4 und 5 beteiligt ist, wobei X₆ für -H steht und wobei X₇ aus der Gruppe gewählt ist, die Wasserstoff, Sauerstoff, Hydroxyl Alkyloxy oder Oxo umfasst; oder
wobei X₃ zusammen mit X'₃ an einer funktionellen Oxo-Gruppe beteiligt ist, wobei X₄ für Wasserstoff steht, wobei X₅ an einer Doppelbindung zwischen den Kohlenstoffatomen in Position 5 und 6 beteiligt ist, wobei X₇ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst; oder
wobei X₃ oder X'₃ unabhängig voneinander aus der Gruppe gewählt sind, die Wasserstoff oder Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3'-Fucosyl-D-Lactosyl, 3'-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ und X₆ an einer Doppelbindung zwischen den Kohlenstoffatomen in Position 4 und 5 beteiligt ist, wobei X₇ aus der Gruppe gewählt ist, die Wasserstoff, Sauerstoff, Hydroxyl, Alkyloxy oder Oxy umfasst, und wobei es sich bei mindestens einer der Gruppen X₃ und X'₃ um eine Glycosyleinheit handelt, die aus der Gruppe gemäß den obigen Angaben gewählt ist; oder
wobei X₃ oder X'₃ unabhängig voneinander aus der Gruppe gewählt sind, die Wasserstoff, Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ für Wasserstoff steht, wobei X₅ und X₆ an einer Doppelbindung zwischen den Kohlenstoffatomen in Position 5 und 6 beteiligt sind, wobei X₇ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, und wobei es sich bei mindestens einer der Gruppen X₃ und X'₃ um eine Glycosyleinheit handelt, die aus der Gruppe gemäß den obigen Angaben gewählt ist, oder
wobei X₃ zusammen mit X'₃ an einer funktionellen Oxogruppe beteiligt ist oder diese unabhängig voneinander aus der Gruppe gewählt sind, die Wasserstoff, Hydroxyl, Alkyloxy umfasst, wobei X₄ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ und X₆ an einer Doppelbindung zwischen den Kohlenstoffatomen in Position 4 und 5 beteiligt sind, und wobei X₇ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst; oder
wobei X₃ oder X'₃ unabhängig voneinander aus der Gruppe gewählt sind, die Wasserstoff, Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-b-D-galactosyl-D-glucosyl, 2-Amino-2-desoxy-4-O-b-D-galactosyl-D-glucosyl, 6'-N-Acetylglucosaminyllactosyl, 2-Acetamido-2-desoxy-3-O-a-L-fucosyl-D-glucosyl, 6-O(2-Acetamido-2-desoxy-b-D-glucosyl)-D-galactosyl, 2-Acetamido-2-desoxy-3-O-b-D-galactosyl-D-glucosyl, 2'-Acetamido-2'-desoxy-3-O-b-D-glucosyl-D-galactosyl, 3-Fucosyl-D-lactosyl, 3-Fucosyl-2-acetamido-2-desoxy-4-0-b-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxyglucosyl, 2-Amino-2-desoxy-galactosyl, 2-Acetamido-2-desoxy-galactosyl, 2-Acetamido-2-desoxy-4-O-b-D-galactosyl-D-glucosyl, 2-Amino-2-desoxy-4-O-b-D-galactosyl-D-glucosyl, 6'-N-Acetylglucosaminyllactosyl, 2-Acetamido-2-desoxy-3-O-a-L-fucosyl-D-glucosyl, 6-O(2-Acetamido-2-desoxy-b-D-glucosyl)-D-galactosyl, 2-Acetamido-2-desoxy-3-O-b-D-galactosyl-D-glucosyl, 2'-Acetamido-2'-desoxy-3-O-b-D-glucosyl-D-galactosyl, 3-Fucosyl-D-lactosyl, 3-Fucosyl-2-acetamido-2-desoxy-4-O-b-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ und X₆ an einer Doppelbindung zwischen den Kohlenstoffatomen in Position 4 und 5 beteiligt sind, wobei X₇ aus der Gruppe gewählt ist, die Glucosyl, Fructosyl, Galactosyl, Mannosyl, Fucosyl, Isomaltosyl, Maltosyl, Lactosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-b-D-galactosyl-D-glucosyl, 2-Amino-2-desoxy-4-O-b-D-galactosyl-D-glucosyl, 6'-N-Acetylglucosaminyllactosyl, 2-Acetamido-2-desoxy-3-O-a-L-fucosyl-D-glucosyl, 6-O(2-Acetamido-2-desoxy-b-D-glucosyl)-D-galactosyl, 2-Acetamido-2-desoxy-3-O-b-D-galactosyl-D-glucosyl, 2'-Acetamido-2'-desoxy-3-O-b-D-glucosyl-D-galactosyl, 3-Fucosyl-D-lactosyl, 3-Fucosyl-2-acetamido-2-desoxy-4-O-b-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei es sich bei mindestens einer de Gruppen X₃ und X'₃ um eine Glycosyleinheit handelt, die aus der Gruppe gemäß den obigen Angaben gewählt ist.

3. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X'₃ aus der Gruppe gewählt ist, die Wasserstoff, Alkyl oder Aralkyl umfasst, wobei X₄ für Wasserstoff steht, wobei X₅ an einer Doppelbindung zwischen den Kohlenstoffatomen in Position 5 und 6 beteiligt ist, wobei X₆ für -H steht, wobei X₇ aus der Gruppe gewählt ist, die Wasserstoff, Sauerstoff, Hydroxyl oder Oxo umfasst, und wobei n den Wert 0 annimmt.

4. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ aus der Gruppe gewählt ist, die Wasserstoff, Hydroxyl, Oxyalkyl, Oxycarbonyl umfasst, wobei X'₃ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ für Wasserstoff steht, wobei X₅ an einer Doppelbindung zwischen den Kohlenstoffatomen in Position 5 und 6 beteiligt ist, wobei X₆ für -H steht, wobei X₇ aus der Gruppe gewählt ist, die Wasserstoff, Sauerstoff, Hydroxyl oder Oxo umfasst, und wobei n den Wert 0 annimmt.

5. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ zusammen mit X'₃ an einer funktionellen Oxogruppe beteiligt ist, wobei X₄ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxyglucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ an einer Doppelbindung zwischen den Kohlenstoffatomen in Position 4 und 5 beteiligt ist, wobei X₆ für -H steht, wobei X₇ aus der Gruppe gewählt ist, die Wasserstoff, Sauerstoff, Hydroxyl, Alkyloxy oder Oxo umfasst, und wobei n den Wert 0 annimmt.

6. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ zusammen mit X'₃ an einer funktionellen Oxogruppe beteiligt ist, wobei X₄ für Wasserstoff steht, wobei X₅ an einer Doppelbindung zwischen den Kohlenstoffatomen in Position 5 und 6 beteiligt ist, wobei X₆ für -H steht, wobei X₇ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst; und wobei n den Wert 0 annimmt.

7. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ oder X'₃ unabhängig voneinander aus der Gruppe gewählt sind, die Wasserstoff oder Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ und X₆ an einer Doppelbindung zwischen den Kohlenstoffatomen 4 und 5 beteiligt sind, wobei X₆ für -H steht, wobei X₇ aus der Gruppe gewählt ist, die Wasserstoff, Sauerstoff, Hydroxyl, Alkyloxy oder Oxo umfasst, wobei es sich bei mindestens einer der Gruppen X₃ und X'₃ um eine Glycosyleinheit handelt, die aus der Gruppe gemäß den obigen Angaben gewählt ist, und wobei n den Wert 0 annimmt.

8. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ oder X'₃ unabhängig voneinander aus der Gruppe gewählt sind, die Wasserstoff, Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ für Wasserstoff steht, wobei X₅ und X₆ an einer Doppelbindung zwischen den Kohlenstoffatomen in Position 5 und 6 beteiligt sind, wobei X₆ für -H steht, wobei X₇ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei es sich bei mindestens einer der Gruppen X₃ und X'₃ um eine Glycosyleinheit handelt, die aus der Gruppe gemäß den obigen Angaben gewählt ist, und wobei n den Wert 0 annimmt.

9. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ zusammen mit X'₃ an einer funktionellen Oxogruppe beteiligt ist oder diese unabhängig voneinander aus der Gruppe gewählt sind, die Wasserstoff, Hydroxyl, Alkyloxy umfasst, wobei X₄ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ und X₆ an einer Doppelbindung zwischen den Kohlenstoffatomen in Position 4 und 5 beteiligt sind, wobei X₆ für -H steht, wobei X₇ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, und wobei n den Wert 0 annimmt.

10. Verbindung gemäß den Ansprüchen 1 oder 2, deren Stereoisomere, Tautomere, Racemate, oder ein pharmazeutisch unbedenkliches Salz und/oder Solvat derselben, wobei X₁ und X₂ für -OMe stehen, wobei R₁ und R₂ für -H stehen, wobei X₃ oder X'₃ unabhängig voneinander aus der Gruppe gewählt sind, die Wasserstoff, Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₄ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei X₅ und X₆ an einer Doppelbindung zwischen den Kohlenstoffatomen in Position 4 und 5 beteiligt sind, wobei X₆ für -H steht, wobei X₇ aus der Gruppe gewählt ist, die Glucosyl, Fructosyl, Galactosyl, Mannosyl, Fucosyl, Isomaltosyl, Maltosyl, Cellobiosyl, Gentiobiosyl, Melibiosyl, Palatinosyl, Lactulosyl, 3-Mannobiosyl, 6-Mannobiosyl, 3-Galactobiosyl, 4-Galactobiosyl, Maltotriosyl, Maltotetraosyl, 2-Amino-2-desoxy-glucosyl, 2-Acetamido-2-desoxy-glucosyl, 2-Amino-2-desoxy-galactosyl, 2-Acetamido-2-desoxy-galactosyl, 2-Acetamido-2-desoxy-4-O-β-D-galactosyl-D-glucosyl, 2-Amino-2-desoxy-4-O-β-D-galactosyl-D-glucosyl, 6'-N-Acetylglucosaminyllactosyl, 2-Acetamido-2-desoxy-3-O-α-L-fucosyl-D-glucosyl, 6-O(2-Acetamido-2-desoxy-β-D-glucosyl)-D-galactosyl, 2-Acetamido-2-desoxy-3-O-β-D-galactosyl-D-glucosyl, 2'-Acetamido-2'-desoxy-3-O-β-D-glucosyl-D-galactosyl, 3-Fucosyl-D-lactosyl, 3-Fucosyl-2-acetamido-2-desoxy-4-O-β-D-galactosyl-D-glucosyl, L- oder D-Isomere derselben, α- oder β-Formen derselben, Pyranuron- oder Furanuronformen derselben, Pyranose- und Furanoseformen derselben, ein Disaccharid oder ein Trisaccharid derselben umfasst, wobei es sich bei mindestens einer der Gruppen X₃ und X'₃ um eine Glycosyleinheit handelt, die aus der Gruppe gemäß den obigen Angaben gewählt ist, und wobei n den Wert 0 annimmt.

11. Verbindung nach Formel I, deren Stereoisomere, Tautomere, Racemate, oder ein pharmazeutisch unbedenkliches Salz und/oder Solvat derselben, wobei X₁ und X₂ für - OMe stehen, R₁ und R₂ für -H stehen, X₃ zusammen mit X'₃ an einer funktionellen Oxo-Gruppe beteiligt ist, X₄ und X₆ für -H stehen und n den Wert 0 annimmt, X₅ an einer Doppelbindung zwischen den Kohlenstoffatomen in Position 5 und 6 beteiligt ist und X₇ aus D-Mannosyl, D-Galactosyl, D-Cellobiosyl, D-Isomaltosyl, L-Fucosyl, D-Lactosyl, D-Gentiobiosyl, D-Maltosyl, 2-Acetamido-2-desoxy-D-glucosyl, 2-Acetamido-2-desoxy-D-galactosyl, Xylopyranosyl, N-Acetyl-lactosaminyl, 2-Acetamido-2-desoxy-4-O-β-D-galactosyl-D-glucosyl, 3-Fucosyl-2-acetamido-2-desoxy-4-O-β-D-galactosyl-D-glucosyl, 3-Fucosyl-D-lactosyl, D-Melibiosyl, D-Maltotrisosyl, D-Lactulosyl oder D-Palatinosyl gewählt ist; oder
wobei X₁ und X₂ für -OMe stehen, R₁ und R₂ für -H stehen, X₃ zusammen mit X'₃ an einer funktionellen Oxo-Gruppe beteiligt ist, X₆ für -H steht, n den Wert 0 annimmt, X₅ an einer Doppelbindung zwischen den Kohlenstoffatomen in Position 4 und 5 beteiligt ist und X₄ und X₇ gleichartig sind und aus L-Fucosyl, D-Cellobiosyl, D-Isomaltosyl, D-Gentiobiosyl, D-Maltosyl, D-Lactosyl, N-Acetyl-lactosaminyl, 2-Acetamido-2-desoxy-4-O-β-D-galactosyl-D-glucosyl, 3-Fucosyl-2-acetamido-2-desoxy-4-O-β-D-galactosyl-D-glucosyl, 3-Fucosyl-D-lactosyl, D-Melibiosyl, D-Maltotrisosyl, D-Lactulosyl oder D-Palatinosyl gewählt sind; oder
wobei X₁ und X₂ für -OMe stehen, R₁ und R₂ für -H stehen, X'₃ für H steht und X₆ für -H steht und n den Wert 0 annimmt, X₅ an einer Doppelbindung zwischen den Kohlenstoffatomen in Position 4 und 5 beteiligt ist, X₃, X₄ und X₇ gleichartig sind und aus L-Fucosyl, D-Cellobiosyl, D-Isomaltosyl, D-Gentiobiosyl, D-Maltosyl, D-Lactosyl, N-Acetyl-lactosaminyl, 2-Acetamido-2-desoxy-4-O-β-D-galactosyl-D-glucosyl, 3-Fucosyl-2-acetamido-2-desoxy-4-O-β-D-galactosyl-D-glucosyl, 3-Fucosyl-D-lactosyl, D-Melibiosyl, D-Maltotrisosyl, D-Lactulosyl oder D-Palatinosyl gewählt sind; oder
wobei X₁ und X₂ für -OMe stehen, R₁ und R₂ für -H stehen, X'₃ für H steht und X₆ für -H steht und n den Wert 0 annimmt, X₅ an einer Doppelbindung zwischen den Kohlenstoffatomen in Position 4 und 5 beteiligt ist, X₇ für Hydroxyl steht, X₃ und X₄ gleichartig sind und aus L-Fucosyl, D-Cellobiosyl, D-Isomaltosyl, D-Gentiobiosyl, D-Maltosyl, D-Lactosyl, N-Acetyl-lactosaminyl, 2-Acetamido-2-desoxy-4-O-β-D-galactosyl-D-glucosyl, 3-Fucosyl-2-acetamido-2-desoxy-4-O-β-D-galactosyl-D-glucosyl, 3-Fucosyl-D-lactosyl, D-Melibiosyl, D-Maltotrisosyl, D-Lactulosyl oder D-Palatinosyl gewählt sind; oder
wobei X₁ und X₂ für -OMe stehen, R₁ und R₂ für -H stehen, X'₃ für H steht und X₆ für -H steht und n den Wert 0 annimmt, X₅ an einer Doppelbindung zwischen den Kohlenstoffatomen in Position 5 und 6 beteiligt ist, X₄ für -H steht, X₃ und X₇ gleichartig sind und aus L-Fucosyl, D-Cellobiosyl, D-Isomaltosyl, D-Gentiobiosyl, D-Maltosyl, D-Lactosyl, N-Acetyl-lactosaminyl, 2-Acetamido-2-desoxy-4-O-β-D-galactosyl-D-glucosyl, 3-Fucosyl-2-acetamido-2-desoxy-4-O-β-D-galactosyl-D-glucosyl, 3-Fucosyl-D-lactosyl, D-Melibiosyl, D-Maltotrisosyl, D-Lactulosyl oder D-Palatinosyl gewählt sind; oder
wobei X₁ und X₂ für -OMe stehen, R₁ und R₂ für -H stehen, X₃ zusammen mit X'₃ an einer funktionellen Oxogruppe beteiligt ist, X₇ für eine Oxogruppe steht und X₆ für -H steht und n den Wert 0 annimmt, X₅ an einer Doppelbindung zwischen den Kohlenstoffatomen in Position 4 und 5 beteiligt ist und X₄ aus β-D-Glucopyranosyl, Galactopyranosyl, Mannopyranosyl, Xylopyranosyl, Cellobiosyl, Lactosyl, Glucofuranosyl, Maltosyl oder Gentiobiosyl gewählt ist; oder
wobei X₁ und X₂ für -OMe stehen, R₁ und R₂ für -H stehen, X₃ für L-Fucosyl steht, X'₃ für H steht und X₆ und für -H steht und n den Wert 0 annimmt, X₅ an einer Doppelbindung zwischen den Kohlenstoffatomen in Position 5 und 6 beteiligt ist, X₄ für H steht und X₇ aus L-Fucosyl, D-Cellobiosyl, D-Isomaltosyl, D-Gentiobiosyl, D-Maltosyl oder 2-Acetamido-2-desoxy-4-O-β-D-galactosyl-D-glucosyl gewählt ist; oder
wobei X₁ und X₂ für -OMe stehen, R₁ und R₂ für -H stehen, X₃ für D-Cellobiosyl steht, X'₃ für H steht und X₆ für -H steht und n den Wert 0 annimmt, X₅ an einer Doppelbindung zwischen den Kohlenstoffatomen in Position 5 und 6 beteiligt ist, X₄ für H steht und X₇ aus D-Cellobiosyl, D-Lactosyl, D-Isomaltosyl, L-Fucosyl, D-Gentiobiosyl, D-Maltosyl oder 2-Acetamido-2-desoxy-4-O-β-D-galactosyl-D-glicosyl gewählt ist; oder
wobei X₁ und X₂ für -OMe stehen, R₁ und R₂ für -H stehen, X₃ für D-Gentiobiosyl steht, X'₃ für H steht und X₆ für -H steht und n den Wert 0 annimmt, X₅ an einer Doppelbindung zwischen den Kohlenstoffatomen in Position 5 und 6 beteiligt ist, X₄ für H steht und X₇ aus D-Gentiobiosyl, D-Lactosyl, D-Cellobiosyl, D-Isomaltosyl, L-Fucosyl, D-Maltosyl oder 2-Acetamido-2-desoxy-4-O-β-D-galactosyl-D-glucosyl gewählt ist; oder
wobei X₁ und X₂ für -OMe stehen, R₁ und R₂ für -H stehen, X₃ für D-Lactosyl steht, X'₃ für H steht und X₆ für -H steht und n den Wert 0 annimmt, X₅ an einer Doppelbindung zwischen den Kohlenstoffatomen in Position 5 und 6 beteiligt ist, X₄ für H steht und X₇ aus Hydroxyl, D-Lactosyl, D-Cellobiosyl, D-Isomaltosyl, L-Fucosyl, D-Gentiobiosyl, D-Maltosyl oder 2-Acetamido-2-desoxy-4-O-fl-D-galactosyl-D-glucosyl gewählt ist; oder
wobei X₁ und X₂ für -OMe stehen, R₁ und R₂ für -H stehen, X₃ für D-Isomaltosyl steht, X'₃ für H steht und X₆ für -H steht und n den Wert 0 annimmt, X₅ an einer Doppelbindung zwischen den Kohlenstoffatomen in Position 5 und 6 beteiligt ist, X₄ für H steht und X₇ aus D-Isomaltosyl, D-Lactosyl, D-Cellobiosyl, L-Fucosyl, D-Gentiobiosyl, D-Maltosyl oder 2-Acetamido-2-desoxy-4-O-β-D-galactosyl-D-glucosyl gewählt ist; oder
wobei X₁ und X₂ für -OMe stehen, R₁ und R₂ für -H stehen, X₃ für D-Maltosyl steht, X'₃ für H steht und X₆ für -H steht und n den Wert 0 annimmt, X₅ an einer Doppelbindung zwischen den Kohlenstoffatomen in Position 5 und 6 beteiligt ist, X₄ für H steht und X₇ aus D-Maltosyl, D-Lactosyl, D-Cellobiosyl, D-Isomaltosyl, L-Fucosyl, D-Gentiobiosyl oder 2-Acetamido-2-desoxy-4-O-β-D-galactosyl-D-glucosyl gewählt ist; oder
wobei X₁ und X₂ für -OMe stehen, R₁ und R₂ für -H stehen, X₃ für 2-Acetamido-2-desoxy-4-O-β-D-galactosyl-D-glucosyl steht, X'₃ für H steht und X₆ für -H steht und n den Wert 0 annimmt, X₅ an einer Doppelbindung zwischen den Kohlenstoffatomen in Position 5 und 6 beteiligt ist, X₄ für H steht und X₇ aus 2-Acetamido-2-desoxy-4-O-β-D-galactosyl-D-glucosyl, D-Maltosyl, D-Lactosyl, D-Cellobiosyl, D-Isomaltosyl, L-Fucosyl oder D-Gentiobiosyl gewählt ist.

12. Verfahren zur Herstellung einer Verbindung nach der Strukturformel I 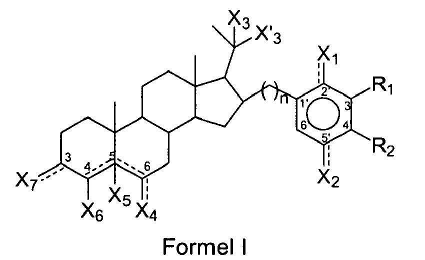 wobei X₁, X₂, X₃, X₄, X₅, X₆, X₇, R₁, R₂ und n unabhängig voneinander aus der Gruppe gemäß den Angaben in einem beliebigen der Ansprüche 1 bis 10 gewählt sind, wobei das Verfahren die folgende Schritte umfasst
a) Bereitstellen eines Ausgangsstoffes nach der Strukturformel IV, 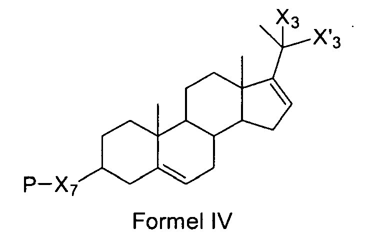 wobei X₃ zusammen mit X'₃ an einer funktionellen Oxogruppe beteiligt ist oder wobei X₃ und X'₃ unabhängig voneinander aus der Gruppe gewählt sind, die Wasserstoff, Hydroxyl, Schwefel, Oxyalkyl, Oxycarbonyl, Alkyl, Het¹-alkyl, Alkyloxycarbonyl, Alkenyl, Alkinyl, Aminoalkyl, Aminoacyl, Alkylcarbonylamino, Alkylthiocarbonylamino, Het¹ umfasst, wobei diese möglicherweise einen oder mehrere Substituenten aufweisen, die unabhängig voneinander aus der Gruppe gewählt sind, die Alkyl, Aralkyl, Aryl, Het¹, Het², Cycloalkyl, Alkyloxy, Alkyloxycarbonyl, Carboxyl, Aminocarbonyl, Mono- oder Di(alkyl)aminocarbonyl, Aminosulfonyl, AlkylS(=O)t, Hydroxy, Cyano, Halogen oder Amino umfasst, wobei diese wiederum möglicherweise einen oder zwei Substituenten aufweisen, die unabhängig voneinander aus der Gruppe gewählt sind, die Alkyl, Aryl, Aralkyl, Aryloxy, Arylamino, Arylthio, Aryloxyalkyl, Arylaminoalkyl, Aralkoxy, Alkylthio, Alkoxy, Aryloxyalkoxy, Arylaminoalkoxy, Aralkylamino, Aryloxyalkylamino, Arylaminoalkylamino, Arylthioalkoxy, Arylthioalkylamino, Aralkylthio, Aryloxyalkylthio, Arylaminoalkylthio, Arylthioalkylthio, Alkylamino, Cycloalkyl und Cycloalkylalkyl umfasst;
wobei X₇ aus der Gruppe gewählt ist, die Wasserstoff, Sauerstoff, Halogen, Oxo, Carbonyl, Thiocarbonyl, Hydroxyl, Alkyl, Aryl, Het¹, Het¹-alkyl, Het¹-aryl, Alkenyl, Alkinyl, Hydroxyalkyl, Hydroxycarbonyl, Hydroxycarbonylalkyl, Hydroxycarbonylaryl, Hydroxycarbonyloxyalkyl, wobei diese möglicherweise einen oder mehrere Substituenten aufweisen, die unabhängig voneinander aus der Gruppe gewählt sind, die Alkyl, Aralkyl, Aryl, Het¹, Het², Cycloalkyl, Alkyloxy, Alkyloxycarbonyl, Carboxyl, Aminocarbonyl, Mono- oder Di(alkyl)aminocarbonyl, Aminosulfonyl, AlkylS(=O)t, Hydroxy, Cyano, Halogen oder Amino umfasst, wobei diese wiederum möglicherweise einen oder zwei Substituenten aufweisen, die unabhängig voneinander aus der Gruppe gewählt sind, die Alkyl, Aryl, Aralkyl, Aryloxy, Arylamino, Arylthio, Aryloxyalkyl, Arylaminoalkyl, Aralkoxy, Alkylthio, Alkoxy, Aryloxyalkoxy, Arylaminoalkoxy, Aralkylamino, Aryloxyalkylamino, Arylaminoalkylamino, Arylthioalkoxy, Arylthioalkylamino, Aralkylthio, Aryloxyalkylthio, Arylaminoalkylthio, Arylthioalkylthio, Alkylamino, Cycloalkyl und Cycloalkylalkyl umfasst; und wobei P für eine Schutzgruppe steht,
b) Umsetzen der Verbindung des Schritt a) mit einer organometallischen Verbindung nach der Strukturformel V 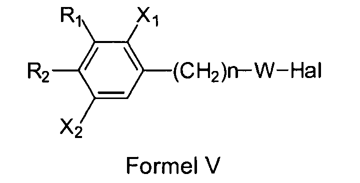 wobei X₁, X₂, R₁, R₂ und n unabhängig voneinander aus der Gruppe gemäß den Angaben in einem beliebigen der Ansprüche 1 bis 10 gewählt sind, wobei W für ein Metall oder eine Kombination von Metallen steht und wobei Hal für ein Halogenatom steht,
um ein Zwischenprodukt nach der Strukturformel III' zu erhalten 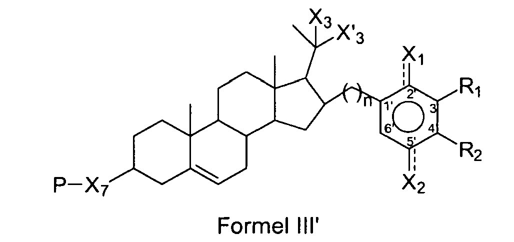 wobei X₁, X₂, R₁, R₂ und n unabhängig voneinander aus der Gruppe gemäß den Angaben in einem beliebigen der Ansprüche 1 bis 10 gewählt sind, wobei X₃, X'₃, X₇ unabhängig voneinander aus der Gruppe gemäß den Angaben in Schritt a) gewählt sind und wobei P für eine Schutzgruppe steht,
c) Umsetzen der Verbindung des Schrittes b) mit einer organometallischen Verbindung nach der Strukturformel VI
Hal-W-X'₃ Formel VI
wobei X'₃ aus der Gruppe gemäß den Angaben in Schritt a) gewählt ist, wobei W für ein Metall oder eine Kombination von Metallen steht und wobei Hal für ein Halogenatom steht,
um ein Zwischenprodukt nach der Strukturformel III zu erhalten 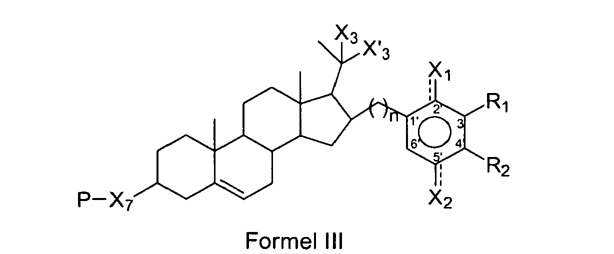 wobei X₁, X₂, R_{1'} R₂ und n unabhängig voneinander aus der Gruppe gemäß den Angaben in einem beliebigen der Ansprüche 1 bis 10 gewählt sind, wobei X₃, X'₃, X₇ unabhängig voneinander aus der Gruppe gemäß den Angaben in Schritt a) gewählt sind, wobei P für eine Schutzgruppe steht,
d) Entfernen der Schutzgruppe von der Gruppe X₇ der Verbindung, die in Schritt c) erhalten wurde, um eine Verbindung nach der Strukturformel II zu bilden 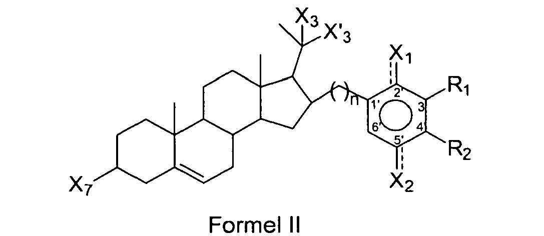 wobei X₁, X₂, R₁, R₂ und n unabhängig voneinander aus der Gruppe gemäß den Angaben in einem beliebigen der Ansprüche 1 bis 10 gewählt sind, wobei X₃, X'₃, X₇ unabhängig voneinander aus der Gruppe gemäß den Angaben in Schritt a) gewählt sind, und
e) Einfügen eines O-geschützten Glycosyls oder eines ungeschützten Glycosyls mittels einer Kupplungsreaktion, wobei X₁, X₂, R₁, R₂ und n unabhängig voneinander aus der Gruppe gemäß den Angaben in einem beliebigen der Ansprüche 1 bis 10 gewählt sind, wobei X₃ und X'₃ unabhängig voneinander aus der Gruppe gemäß den Angaben in Schritt a) gewählt sind und X₇ für ein O-geschütztes Glycosyl oder ein ungeschütztes Glycosyl steht, und
f) Entfernen der Schutzgruppen von den O-geschützten Glycosylgruppen, um eine Verbindung nach der Formel IB zu bilden, wobei X₁, X₂, X₄, X₅, X₆, R₁, R₂ und n unabhängig voneinander aus der Gruppe gemäß den Angaben in einem beliebigen der Ansprüche 1 bis 10 gewählt sind, wobei X₃, X'₃ unabhängig voneinander aus der Gruppe gemäß den Angaben in Schritt a) gewählt sind und X₇ aus einer Gruppe gewählt ist, die Glycosyl, Thioderivate desselben, Aminoderivate desselben, Amidoderivate desselben, hydroxyl-geschützte Derivate desselben umfasst.

13. Verfahren gemäß dem Anspruch 11, wobei der Schritt e) darin besteht, die Verbindung des Schrittes d) mit einem Oxidationsreagenz umzusetzen, um ein Zwischenprodukt zu erhalten und dieses Zwischenprodukt mit einem Reduktionsreagenz umzusetzen, um ein weiteres Zwischenprodukt nach der Strukturformel I zu erhalten, wobei X₁, X₂, R₁, R₂ und n unabhängig voneinander aus der Gruppe gemäß den Angaben in einem beliebigen der Ansprüche 1 bis 10 gewählt sind und X₃ oder X'₃ und X₄ und X₇ für Hydroxyl stehen und die Reaktion mit den Schritten e) und f) gemäß dem Anspruch 11 fortgeführt wird, um eine glycosylierte Steroidverbindung nach der Strukturformel I zu erhalten.

14. Verfahren gemäß dem Anspruch 11, wobei der Schritt c) darin besteht, die Verbindung des Schrittes b) mit einem O-geschützten Glycosyl oder einem ungeschützten Glycosyl umzusetzen, um ein Zwischenprodukt nach der Strukturformel III zu erhalten, wobei X₁, X₂, R₁, R₂ und n unabhängig voneinander aus der Gruppe gemäß den Angaben in einem beliebigen der Ansprüche 1 bis 10 gewählt sind, wobei X₃, X₇ unabhängig voneinander aus der Gruppe gemäß den Angaben in Schritt a) des Anspruchs 11 gewählt sind, wobei P für eine Schutzgruppe steht und wobei X₃ oder X'₃ für ein O-geschütztes Glycosyl oder ein ungeschütztes Glycosyl stehen und die Reaktion mit den Schritten d), e) und f) gemäß dem Anspruch 11 fortgeführt wird, um eine glycosylierte Steroidverbindung der Strukturformel I zu erhalten.

15. Verbindung der Strukturformel: 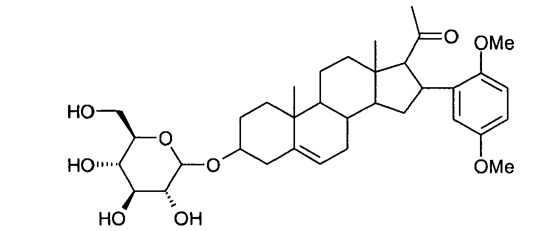 im vorliegenden Schriftstück als Verbindung UBS3268 bezeichnet

16. Verbindung der Strukturformel: 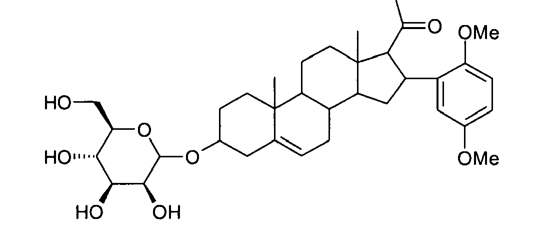 im vorliegenden Schriftstück als Verbindung UBS3270 bezeichnet

17. Verbindung der Strukturformel: 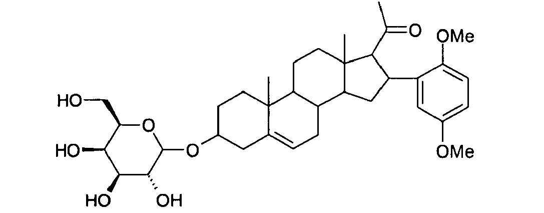 im vorliegenden Schriftstück als Verbindung UBS3285 bezeichnet

18. Verbindung der Strukturformel:

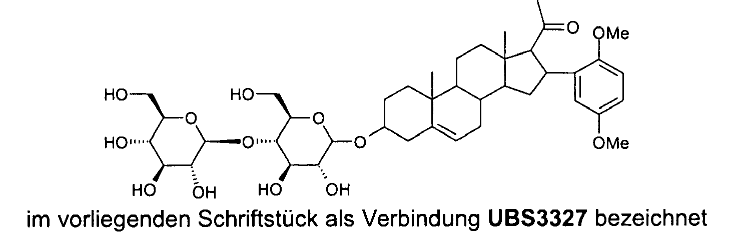

19. Verbindung der Strukturformel:

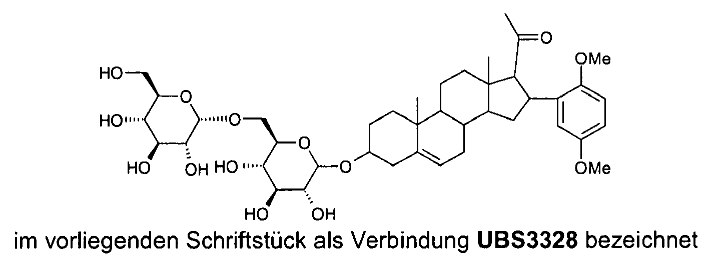

20. Verbindung der Strukturformel:

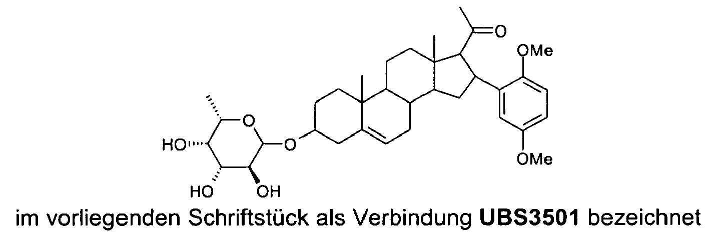

21. Verbindung der Strukturformel:

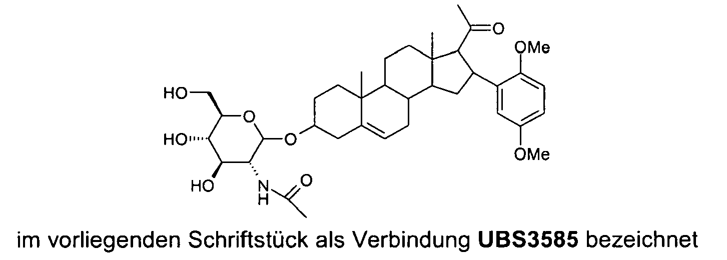

22. Verbindung der Strukturformel:

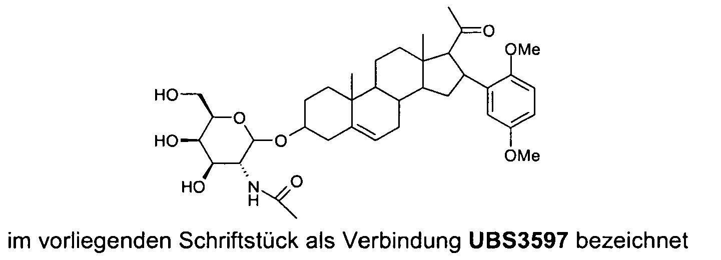

23. Verbindung der Strukturformel:

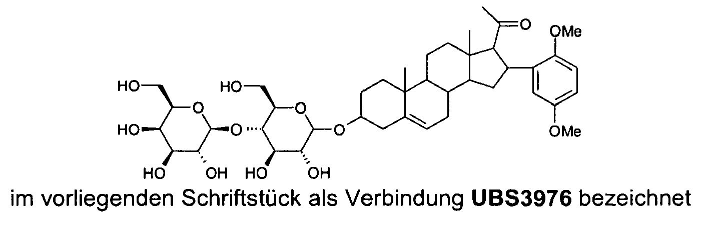

24. Verbindung der Strukturformel:

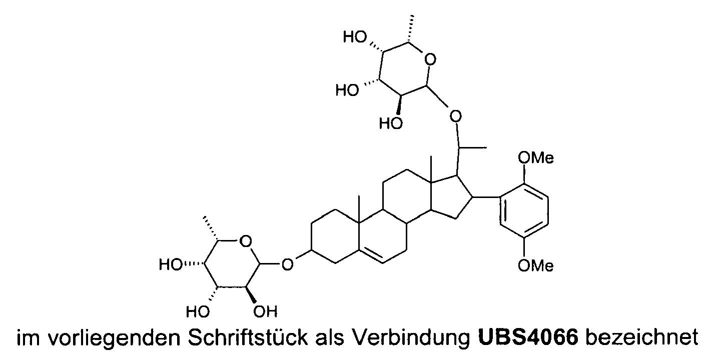

25. Verbindung der Strukturformel:

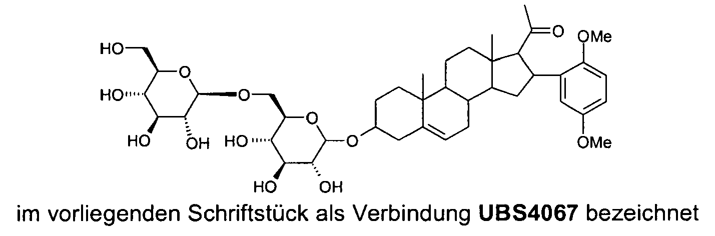

26. Verbindung der Strukturformel:

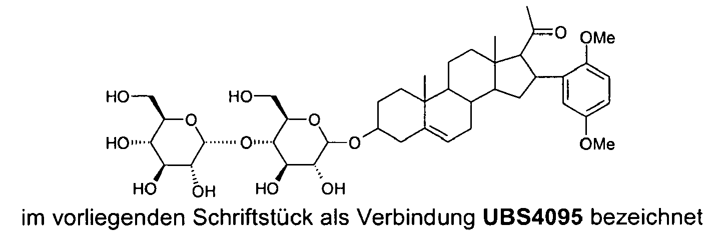

27. Verbindung der Strukturformel:

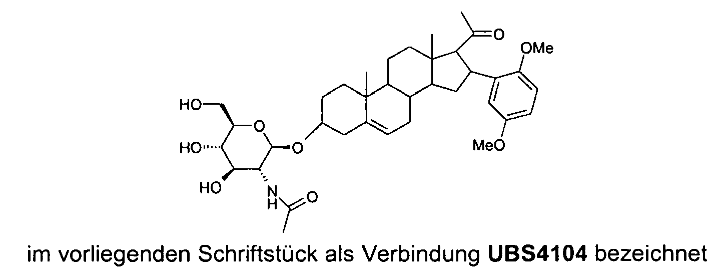

28. Verbindung der Strukturformel:

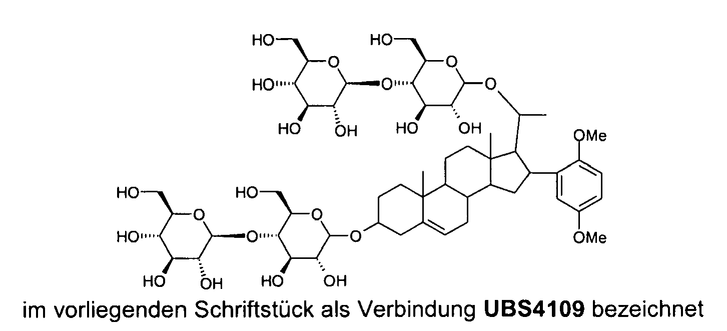

29. Verbindung der Strukturformel:

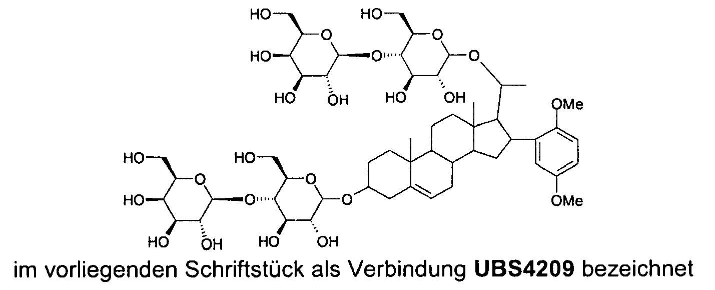

30. Verbindung der Strukturformel:

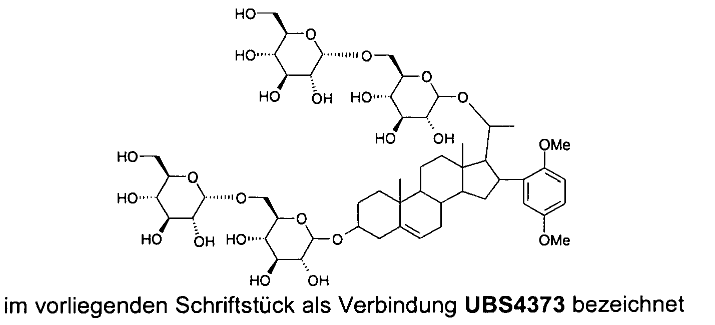

31. Verbindung gemäß einem beliebigen der Ansprüche 1 bis 10 und 14 bis 29 zur Verwendung als Arzneimittel.

32. Verbindung gemäß einem beliebigen der Ansprüche 1 bis 10 und 14 bis 29 zur Verwendung als Antimigrationsmittel.

33. Verwendung einer Verbindung gemäß einem beliebigen der Ansprüche 1 bis 10 und 14 bis 29 zur Herstellung eines Arzneimittels zur Behandlung von Krebs.

34. Pharmazeutische Zusammensetzung, die einen pharmazeutisch unbedenklichen Trägerstoff und eine therapeutisch wirksame Menge einer Verbindung gemäß einem beliebigen der Ansprüche 1 bis 10 und 14 bis 29 enthält.

35. Verwendung einer pharmazeutischen Zusammensetzung gemäß Anspruch 33 zur Herstellung eines Arzneimittels zur Behandlung von Krebs.

## Revendications

1. Composé de formule structurelle I, les stéréoisomères, les tautomères, les racémiques de celui-ci, ou un sel et/ou un solvate pharmaceutiquement acceptable de celui-ci, 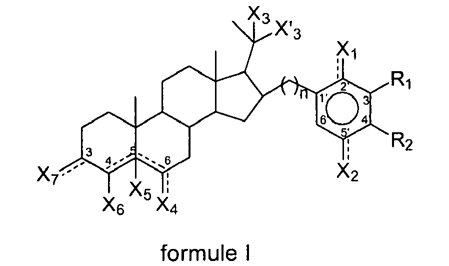 dans lequel X₁, X₂ sont indépendamment -OMe, R₁ et R₂ sont indépendamment un atome d'hydrogène,
dans lequel X₃ fait partie, conjointement à X'₃, d'un groupe fonctionnel oxo, ou
dans lequel X₃ et X'₃ sont indépendamment choisis dans le groupe comprenant un atome d'hydrogène, les groupes hydroxyle, oxyalkyle, oxycarbonyle, glucosyle, fructosyle, galactosyle, mannosyle, ribosyle, ribulosyle, xylulosyle, érythrosyle, érythrulosyle, rhamnosyle, thréosyle, sorbosyle, psicosyle, tagatosyle, fucosyle, arabinosyle, altrosyle, laminaribiosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-amino-2-désoxy-mannosyle, 2-acétamido-2-désoxy-mannosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, une combinaison de ceux-ci, les dérivés désoxy de ceux-ci, les dérivés acétate protégés par un groupe hydroxyle de ceux-ci, les dérivés amino de ceux-ci, les dérivés amido de ceux-ci, les dérivés thio de ceux-ci, un disaccharide de ceux-ci, un trisaccharide de ceux-ci, un oligosaccharide et un polysaccharide de ceux-ci ;
dans lequel X₄ et X₇ sont indépendamment choisis dans le groupe comprenant un atome d'hydrogène, un atome d'oxygène, les groupes oxo, hydroxyle, glucosyle, fructosyle, galactosyle, mannosyle, ribosyle, ribulosyle, xylulosyle, érythrosyle, érythrulosyle, rhamnosyle, thréosyle, sorbosyle, psicosyle, tagatosyle, fucosyle, arabinosyle, altrosyle, laminaribiosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy galactosyle, 2-acétamido-2-désoxy-galactosyle, 2-amino-2-désoxy-mannosyle, 2-acétamido-2-désoxy-mannosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-α-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, une combinaison de ceux-ci, les dérivés désoxy de ceux-ci, les dérivés acétate ou benzoyle protégés par un groupe hydroxyle de ceux-ci, les dérivés amino de ceux-ci, les dérivés amido de ceux-ci, les dérivés thio de ceux-ci, un disaccharide de ceux-ci, un trisaccharide de ceux-ci, un oligosaccharide et un polysaccharide de ceux-ci ;
dans lequel au moins un de X₃, X'₃, X₄ et X₇ est une fraction glycosyle choisie dans le groupe tel qu'indiqué ci-dessus ;
dans lequel X₄ ou X₆ est un atome d'hydrogène et dans lequel X₅ fait partie d'une double liaison entre les atomes de carbone aux positions 4 et 5 ou aux positions 5 et 6, et
dans lequel n vaut 0.

2. Composé selon la revendication 1, dans lequel X₁ et X₂ sont -OMe, dans lequel R₁ et R₂ sont -H, dans lequel n vaut 0, dans lequel X₆ est -H,
et
dans lequel X₃ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X'₃ est choisi dans le groupe comprenant un atome d'hydrogène, un groupe alkyle ou aralkyle, dans lequel X₄ est un atome d'hydrogène, dans lequel X₅ fait partie d'une double liaison entre les atomes de carbone aux positions 5 et 6, et dans lequel X₇ est choisi dans le groupe comprenant un atome d'hydrogène, un atome d'oxygène, un groupe hydroxyle ou oxo ; ou
dans lequel X₃ est choisi dans le groupe comprenant un atome d'hydrogène, un groupe hydroxyle, oxyalkyle ou oxycarbonyle, dans lequel X'₃ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₄ est un atome d'hydrogène, dans lequel X₅ fait partie d'une double liaison entre les atomes de carbone aux positions 5 et 6, et dans lequel X₇ est choisi dans le groupe comprenant un atome d'hydrogène, un atome d'oxygène, un groupe hydroxyle ou oxo ; ou
dans lequel X₃ fait partie, conjointement à X'₃, d'un groupe fonctionnel oxo, dans lequel X₄ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₅ fait partie d'une double liaison entre les atomes de carbone aux positions 4 et 5, dans lequel X₆ est -H, et dans lequel X₇ est choisi dans le groupe comprenant un atome d'hydrogène, un atome d'oxygène, un groupe hydroxyle, alkyloxy ou oxo ; ou
dans lequel X₃ fait partie, conjointement à X'₃, d'un groupe fonctionnel oxo, dans lequel X₄ est un atome d'hydrogène, dans lequel X₅ fait partie d'une double liaison entre les atomes de carbone aux positions 5 et 6, et dans lequel X₇ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci ; ou
dans lequel X₃ ou X'₃ sont indépendamment choisis dans le groupe comprenant un atome d'hydrogène ou un groupe glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3'-fucosyl-D-lactosyle, 3'-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₄ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₅ et X₆ font partie d'une double liaison entre les atomes de carbone aux positions 4 et 5, dans lequel X₇ est choisi dans le groupe comprenant un atome d'hydrogène, un atome d'oxygène, un groupe hydroxyle, alkyloxy ou oxo, et dans lequel au moins un de X₃ et X'₃ est une fraction glycosyle choisie dans le groupe tel qu'indiqué ci-dessus ; ou
dans lequel X₃ ou X'₃ sont indépendamment choisis dans le groupe comprenant un atome d'hydrogène, les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₄ est un atome d'hydrogène, dans lequel X₅ et X₆ font partie d'une double liaison entre les atomes de carbone aux positions 5 et 6, dans lequel X₇ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, et dans lequel au moins un de X₃ et X'₃ est une fraction glycosyle choisie dans le groupe tel qu'indiqué ci-dessus ; ou
dans lequel X₃ fait partie, conjointement avec X'₃, d'un groupe fonctionnel oxo ou ils sont indépendamment choisis dans le groupe comprenant un atome d'hydrogène, les groupes hydroxyle, alkyloxy, dans lequel X₄ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou *β* de ceux-ci, la forme pyranuronique ou furanuronique de ceux-ci, la forme pyranose ou furanose de ceux-ci, un disaccharide ou un trisaccharide de ceux-ci, dans lequel X₅ et X₆ font partie d'une double liaison entre les atomes de carbone aux positions 4 et 5, et dans lequel X₇ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-*β*-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-a-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci ; ou
dans lequel X₃ ou X'₃ sont indépendamment choisis dans le groupe comprenant un atome d'hydrogène, les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-b-D-galactosyl-D-glucosyle, 2-amino-2-désoxy-4-O-b-D-galactosyl-D-glucosyle, 6'-N-acétylglucosaminyllactosyle, 2-acétamido-2-désoxy-3-O-a-L-fucosyl-D-glucosyle, 6-O(2-acétamido-2-désoxy-b-D-glucosyl)-D-galactosyle, 2-acétamido-2-désoxy-3-O-b-D-galactosyl-D-glucosyle, 2'-acétamido-2'-désoxy-3-O-b-D-glucosyl-D-galactosyle, 3-fucosyl-D-lactosyle, 3-fucosyl-2-acétamido-2-désoxy-4-O-b-D-galactosyl-D-glucosyle, les isomères L ou D de ceux-ci, la forme α ou *β* de ceux-ci, la forme pyranuronique ou furanuronique de ceux-ci, la forme pyranose ou furanose de ceux-ci, un disaccharide ou un trisaccharide de ceux-ci, dans lequel X₄ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-b-D-galactosyl-D-glucosyle, 2-amino-2-désoxy-4-O-b-D-galactosyl-D-glucosyle, 6'-N-acétylglucosaminyllactosyle, 2-acétamido-2-désoxy-3-O-a-L-fucosyl-D-glucosyle, 6-O(2-acétamido-2-désoxy-b-D-glucosyl)-D-galactosyle, 2-acétamido-2-désoxy-3-O-b-D-galactosyl-D-glucosyle, 2'-acétamido-2'-désoxy-3-O-b-D-glucosyl-D-galactosyle, 3-fucosyl-D-lactosyle, 3-fucosyl-2-acétamido-2-désoxy-4-O-b-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₅ et X₆ font partie d'une double liaison entre les atomes de carbone aux positions 4 et 5, dans lequel X₇ est choisi dans le groupe comprenant les groupes glucosyle, fructosyle, galactosyle, mannosyle, fucosyle, isomaltosyle, maltosyle, lactosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-b-D-galactosyl-D-glucosyle, 2-amino-2-désoxy-4-O-b-D-galactosyl-D-glucosyle, 6'-N-acétylglucosaminyllactosyle, 2-acétamido-2-désoxy-3-O-a-L-fucosyl-D-glucosyle, 6-O(2-acétamido-2-désoxy-b-D-glucosyl)-D-galactosyle, 2-acétamido-2-désoxy-3-O-b-D-galactosyl-D-glucosyle, 2'-acétamido-2'-désoxy-3-O-b-D-glucosyl-D-galactosyle, 3-fucosyl-D-lactosyle, 3-fucosyl-2-acétamido-2-désoxy-4-O-b-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel au moins un de X₃ et X'₃ est une fraction glycosyle choisie dans le groupe tel qu'indiqué ci-dessus.

3. Composé selon la revendication 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X'₃ est choisi dans le groupe comprenant un atome d'hydrogène, un groupe alkyle ou aralkyle, dans lequel X₄ est un atome d'hydrogène, dans lequel X₅ fait partie d'une double liaison entre les atomes de carbone aux positions 5 et 6, dans lequel X₆ est -H, dans lequel X₇ est choisi dans le groupe comprenant un atome d'hydrogène, un atome d'oxygène, un groupe hydroxyle ou oxo, et dans lequel n vaut 0.

4. Composé selon la revendication 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ est choisi dans le groupe comprenant un atome d'hydrogène, les groupes hydroxyle, oxyalkyle ou oxycarbonyle, dans lequel X'₃ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₄ est un atome d'hydrogène, dans lequel X₅ fait partie d'une double liaison entre les atomes de carbone aux positions 5 et 6, dans lequel X₆ est -H, dans lequel X₇ est choisi dans le groupe comprenant un atome d'hydrogène, un atome d'oxygène, un groupe hydroxyle ou oxo, et dans lequel n vaut 0.

5. Composé selon la revendication 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ fait partie, conjointement à X'₃, d'un groupe fonctionnel oxo, dans lequel X₄ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₅ fait partie d'une double liaison entre les atomes de carbone aux positions 4 et 5, dans lequel X₆ est -H, dans lequel X₇ est choisi dans le groupe comprenant un atome d'hydrogène, un atome d'oxygène, les groupes hydroxyle, alkyloxy ou oxo, et dans lequel n vaut 0.

6. Composé selon la revendication 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ fait partie, conjointement à X'₃, d'un groupe fonctionnel oxo, dans lequel X₄ est un atome d'hydrogène, dans lequel X₅ fait partie d'une double liaison entre les atomes de carbone aux positions 5 et 6, dans lequel X₆ est -H, dans lequel X₇ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci ; et dans lequel n vaut 0.

7. Composé selon l'une quelconque des revendications 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ ou X'₃ sont indépendamment choisis dans le groupe comprenant un atome d'hydrogène ou un groupe glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3'-fucosyl-D-lactosyle, 3'-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₄ est choisi dans le groupe comprenant les groupes glucosyle, fructosyle, galactosyle, mannosyle, fucosyle, isomaltosyle, isomaltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₅ et X₆ font partie d'une double liaison entre les atomes de carbone aux positions 4 et 5, dans lequel X₆ est -H, dans lequel X₇ est choisi dans le groupe comprenant un atome d'hydrogène, un atome d'oxygène, les groupes hydroxyle, alkyloxy ou oxo, dans lequel au moins un de X₃ et X'₃ est une fraction glycosyle choisie dans le groupe tel qu'indiqué ci-dessus et dans lequel n vaut 0.

8. Composé selon la revendication 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ ou X'₃ sont indépendamment choisis dans le groupe comprenant un atome d'hydrogène, les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₄ est un atome d'hydrogène, dans lequel X₅ et X₆ font partie d'une double liaison entre les atomes de carbone aux positions 5 et 6, dans lequel X₆ est -H, dans lequel X₇ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel au moins un de X₃ et X'₃ est une fraction glycosyle choisie dans le groupe tel qu'indiqué ci-dessus et dans lequel n vaut 0.

9. Composé selon la revendication 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ fait partie, conjointement avec X'₃, d'un groupe fonctionnel oxo ou ils sont indépendamment choisis dans le groupe comprenant un atome d'hydrogène, les groupes hydroxyle, alkyloxy, dans lequel X₄ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₅ et X₆ font partie d'une double liaison entre les atomes de carbone aux positions 4 et 5, dans lequel X₆ est -H, dans lequel X₇ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-β-D-galactosyl-D-glucosyle, 2-amino-2-désoxy-4-O-β-D-galactosyl-D-glucosyle, 6'-N-acétylglucosaminyllactosyle, 2-acétamido-2-désoxy-3-O-α-L-fucosyl-D-glucosyle, 6-O(2-acétamido-2-désoxy-β-D-glucosyl)-D-galactosyle, 2-acétamido-2-désoxy-3-O-β-D-galactosyl-D-glucosyle, 2'-acétamido-2'-désoxy-3-O-β-D-glucosyl-D-galactosyle, 3-fucosyl-D-lactosyle, 3-fucosyl-2-acétamido-2-désoxy-4-O-β-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, et dans lequel n vaut 0.

10. Composé selon la revendication 1 ou 2, les stéréoisomères, les tautomères, les racémiques de celui-ci, ou un sel et/ou un solvate pharmaceutiquement acceptable de celui-ci, dans lequel X₁ et X₂ sont -OMe, dans lequel R₁ et R₂ sont -H, dans lequel X₃ ou X'₃ sont indépendamment choisis dans le groupe comprenant un atome d'hydrogène, les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-b-D-galactosyl-D-glucosyle, 2-amino-2-désoxy-4-O-b-D-galactosyl-D-glucosyle, 6'-N-acétylglucosaminyllactosyle, 2-acétamido-2-désoxy-3-O-a-L-fucosyl-D-glucosyle, 6-O(2-acétamido-2-désoxy-b-D-glucosyl)-D-galactosyle, 2-acétamido-2-désoxy-3-O-b-D-galactosyl-D-glucosyle, 2'-acétamido-2'-désoxy-3-O-bD-glucosyl-D-galactosyle, 3-fucosyl-D-lactosyle, 3-fucosyl-2-acétamido-2-désoxy-4-O-b-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₄ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy-glucosyle, 2-acétamido-2-désoxy-glucosyle, 2-amino-2-désoxy-galactosyle, 2-acétamido-2-désoxy-galactosyle, 2-acétamido-2-désoxy-4-O-b-D-galactosyl-D-glucosyle, 2-amino-2-désoxy-4-O-b-D-galactosyl-D-glucosyle, 6'-N-acétylglucosaminyllactosyle, 2-acétamido-2-désoxy-3-O-a-L-fucosyl-D-glucosyle, 6-O(2-acétamido-2-désoxy-b-D-glucosyl)-D-galactosyle, 2-acétamido-2-désoxy-3-O-b-D-galactosyl-D-glucosyle, 2'-acétamido-2'-désoxy-3-O-bD-glucosyl-D-galactosyle, 3-fucosyl-D-lactosyle, 3-fucosyl-2-acétamido-2-désoxy-4-O-b-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel X₅ et X₆ font partie d'une double liaison entre les atomes de carbone aux positions 4 et 5, dans lequel X₆ est -H, dans lequel X₇ est choisi dans le groupe comprenant les groupes glucosyle, fructosyle, galactosyle, mannosyle, fucosyle, isomaltosyle, maltosyle, cellobiosyle, gentiobiosyle, mélibiosyle, palatinosyle, lactulosyle, 3-mannobiosyle, 6-mannobiosyle, 3-galactobiosyle, 4-galactobiosyle, maltotriosyle, maltotétraosyle, 2-amino-2-désoxy glucosyle, 2-acétamido-2-désoxy-glucosyle, 2-amino-2-désoxy-galactosyle, 2-acétamido-2-désoxy-galactosyle, 2-acétamido-2-désoxy-4-O-b-D-galactosyl-D-glucosyle, 2-amino-2-désoxy-4-O-b-D-galactosyl-D-glucosyle, 6'-N-acétylglucosaminyllactosyle, 2-acétamido-2-désoxy-3-O-a-L-fucosyl-D-glucosyle, 6-O(2-acétamido-2-désoxy-b-D-glucosyl)-D-galactosyle, 2-acétamido-2-désoxy-3-O-b-D-galactosyl-D-glucosyle, 2'-acétamido-2'-désoxy-3-O-b-D-glucosyl-D-galactosyle, 3-fucosyl-D-lactosyle, 3-fucosyl-2-acétamido-2-désoxy-4-O-b-D-galactosyl-D-glucosyle, les isomères L ou D de ceux-ci, la forme α ou β de ceux-ci, la forme pyranuronique ou furanuronique de ceux-ci, la forme pyranose ou furanose de ceux-ci, un disaccharide ou un trisaccharide de ceux-ci, dans lequel au moins un de X₃ et X'₃ est une fraction glycosyle choisie dans le groupe tel qu'indiqué ci-dessus et dans lequel n vaut 0.

11. Composé de formule I, les stéréoisomères, les tautomères, les racémiques de celui-ci, ou un sel et/ou un solvate pharmaceutiquement acceptable de celui-ci, dans lequel X₁ et X₂ sont -OMe, R₁ et R₂ sont -H, X₃ fait partie, conjointement à X'₃, d'un groupe fonctionnel oxo, X₄ et X₆ sont -H, et n est 0, X₅ fait partie d'une double liaison entre les atomes de carbone aux positions 5 et 6, et X₇ est choisi parmi les groupes D-glucosyle, D-mannosyle, D-galactosyle, D-cellobiosyle, D-isomaltosyle, L-fucosyle, D-lactosyle, D-gentiobiosyle, D-maltosyle, 2-acétamido-2-désoxy-D-glucosyle, 2-acétamido-2-désoxy-D-galactosyle, xylopyranosyle, N-acétyl-lactosaminyle, 2-acétamido-2-désoxy-4-O-β-D-galactosyl-D-glucosyle, 3-fucosyl-2-acétamido-2-désoxy-4-O-β-D-galactosyl-D-glucosyle, 3-fucosyl-D-lactosyle, D-mélibiosyle, D-maltotrisosyle, D-lactulosyle, ou D-palatinosyle; ou
dans lequel X₁ et X₂ sont -OMe, R₁ et R₂ sont -H, X₃ fait partie, conjointement à X'₃, d'un groupe fonctionnel oxo, X₆ est -H, n vaut 0, X₅ fait partie d'une double liaison entre les atomes de carbone aux positions 4 et 5, et X₄ et X₇ sont identiques et sont choisis parmi les groupes L-fucosyle, D-cellobiosyle, D-isomaltosyle, D-gentiobiosyle, D-maltosyle, D-lactosyle, N-acétyl-lactosaminyle, 2-acétamido-2-désoxy-4-O-β-D-galactosyl-D-glucosyle, 3-fucosyl-2-acétamido-2-désoxy-4-O-β-D-galactosyl-D-glucosyle, 3-fucosyl-D-lactosyle, D-mélibiosyle, D-maltotrisosyle, D-lactulosyle, ou D-palatinosyle ; ou
dans lequel X₁ et X₂ sont -OMe, R₁ et R₂ sont -H, X'₃ est H, et X₆ est -H, et n est 0, X₅ fait partie d'une double liaison entre les atomes de carbone aux positions 4 et 5, X₃, X₄ et X₇ sont identiques et sont choisis parmi les groupes L-fucosyle, D-cellobiosyle, D-isomaltosyle, D-gentiobiosyle, D-maltosyle, D-lactosyle, N-acétyl-lactosaminyle, 2-acétamido-2-désoxy-4-O-β-D-galactosyl-D-glucosyle, 3-fucosyl-2-acétamido-2-désoxy-4-O-β-D-galactosyl-D-glucosyle, 3-fucosyl-D-lactosyle, D-mélibiosyle, D-maltotrisosyle, D-lactulosyle, ou D-palatinosyle; ou
dans lequel X₁ et X₂ sont -OMe, R₁ et R₂ sont -H, X'₃ est H, et X₆ est -H, et n est 0, X₅ fait partie d'une double liaison entre les atomes de carbone aux positions 4 et 5, X₇ est un groupe hydroxyle, X₃ et X₄ sont identiques et sont choisis parmi les groupes L-fucosyle, D-cellobiosyle, D-isomaltosyle, D-gentiobiosyle, D-maltosyle, D-lactosyle, N-acétyl-lactosaminyle, 2-acétamido-2-désoxy-4-O-β-D-galactosyl-D-glucosyle, 3-fucosyl-2-acétamido-2-désoxy-4-O-β-D-galactosyl-D-glucosyle, 3-fucosyl-D-lactosyle, D-mélibiosyle, D-maltotrisosyle, D-lactulosyle, ou D-palatinosyle; ou
dans lequel X₁ et X₂ sont -OMe, R₁ et R₂ sont -H, X'₃ est H, et X₆ est -H, et n est 0, X₅ fait partie d'une double liaison entre les atomes de carbone aux positions 5 et 6, X₄ est -H, X₃ et X₇ sont identiques et sont choisis parmi les groupes L-fucosyle, D-cellobiosyle, D-isomaltosyle, D-gentiobiosyle, D-maltosyle, D-lactosyle, N-acétyl-lactosaminyle, 2-acétamido-2-désoxy-4-O-β-D-galactosyl-D-glucosyle, 3-fucosyl-2-acétamido-2-désoxy-4-O-β-D-galactosyl-D-glucosyle, 3-fucosyl-D-lactosyle, D-mélibiosyle, D-maltotrisosyle, D-lactulosyle, ou D-palatinosyle; ou
dans lequel X₁ et X₂ sont -OMe, R₁ et R₂ sont -H, X₃ fait partie, conjointement à X'₃, d'un groupe fonctionnel oxo, X₇ est un groupe oxo et X₆ est -H, et n est 0, X₅ fait partie d'une double liaison entre les atomes de carbone aux positions 4 et 5, et X₄ est choisi parmi les groupes β-D-glucopyranosyle, galactopyranosyle, mannopyranosyle, xylopyranosyle, cellobiosyle, lactosyle, glucofuranosyle, maltosyle, ou gentiobiosyle ; ou
dans lequel X₁ et X₂ sont -OMe, R₁ et R₂ sont -H, X₃ est un groupe L-fucosyle, X'₃ est H, et X₆ est -H, et n est 0, X₅ fait partie d'une double liaison entre les atomes de carbone aux positions 5 et 6, X₄ est H, et X₇ est choisi parmi les groupes L-fucosyle, D-cellobiosyle, D-isomaltosyle, D-gentiobiosyle, D-maltosyle, ou 2-acétamido-2-désoxy-4-O-β-D-galactosyl-D-glucosyle ; ou
dans lequel X₁ et X₂ sont -OMe, R₁ et R₂ sont -H, X₃ est un groupe D-cellobiosyle, X'₃ est H, et X₆ est -H, et n est 0, X₅ fait partie d'une double liaison entre les atomes de carbone aux positions 5 et 6, X₄ est H, et X₇ est choisi parmi les groupes D-cellobiosyle, D-lactosyle, D-isomaltosyle, L-fucosyle, D-gentiobiosyle, D-maltosyle, ou 2-acétamido-2-désoxy-4-O-β-D-galactosyl-D-glucosyle ; ou
dans lequel X₁ et X₂ sont -OMe, R₁ et R₂ sont -H, X₃ est un groupe D-gentiobiosyle, X'₃ est H, et X₆ est -H, et n est 0, X₅ fait partie d'une double liaison entre les atomes de carbone aux positions 5 et 6, X₄ est H, et X₇ est choisi parmi les groupes D-gentiobiosyle, D-lactosyle, D-cellobiosyle, D-isomaltosyle, L-fucosyle, D-maltosyle, ou 2-acétamido-2-désoxy-4-O-β-D-galactosyl-D-glucosyle ; ou
dans lequel X₁ et X₂ sont -OMe, R₁ et R₂ sont -H, X₃ est un groupe D-lactosyle, X'₃ est H, et X₆ est -H, et n est 0, X₅ fait partie d'une double liaison entre les atomes de carbone aux positions 5 et 6, X₄ est H, et X₇ est choisi parmi les groupes hydroxyle, D-lactosyle, D-cellobiosyle, D-isomaltosyle, L-fucosyle, D-gentiobiosyle, D-maltosyle, ou 2-acétamido-2-désoxy-4-O-β-D-galactosyl-D-glucosyle ; ou
dans lequel X₁ et X₂ sont -OMe, R₁ et R₂ sont -H, X₃ est un groupe D-isomaltosyle, X'₃ est H, et X₆ est -H, et n est 0, X₅ fait partie d'une double liaison entre les atomes de carbone aux positions 5 et 6, X₄ est H, et X₇ est choisi parmi les groupes D-isomaltosyle, D-lactosyle, D-cellobiosyle, L-fucosyle, D-gentiobiosyle, D-maltosyle, ou 2-acétamido-2-désoxy-4-O-β-D-galactosyl-D-glucosyle ; ou
dans lequel X₁ et X₂ sont -OMe, R₁ et R₂ sont -H, X₃ est un groupe D-maltosyle, X'₃ est H, et X₆ est -H, et n est 0, X₅ fait partie d'une double liaison entre les atomes de carbone aux positions 5 et 6, X₄ est H, et X₇ est choisi parmi les groupes D-maltosyle, D-lactosyle, D-cellobiosyle, D-isomaltosyle, L-fucosyle, D-gentiobiosyle, ou 2-acétamido-2-désoxy-4-O-β-D-galactosyl-D-glucosyle ; ou
dans lequel X₁ et X₂ sont -OMe, R₁ et R₂ sont -H, X₃ est un groupe D-2-acétamido-2-désoxy-4-O-b-D-galactosyl-D-glucosyle, X'₃ est H, et X₆ est -H, et n est 0, X₅ fait partie d'une double liaison entre les atomes de carbone aux positions 5 et 6, X₄ est H, et X₇ est choisi parmi les groupes 2-acétamido-2-désoxy-4-O-β-D-galactosyl-D-glucosyle, D-maltosyle, D-lactosyle, D-cellobiosyle, D-isomaltosyle, L-fucosyle, ou D-gentiobiosyle.

12. Procédé de synthèse d'un composé de formule structurelle 1 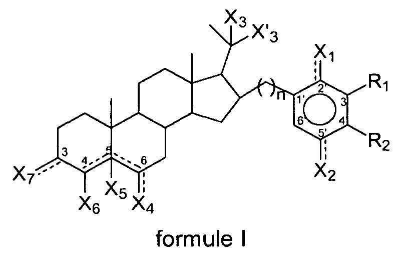 dans lequel X₁, X₂, X₃, X₄, X₅, X₆, X₇, R₁, R₂ et n sont indépendamment choisis dans le groupe tel qu'indiqué dans l'une quelconque des revendications 1 à 10, ledit procédé comprenant les étapes consistant à
a) fournir un matériau de départ de formule structurelle IV, 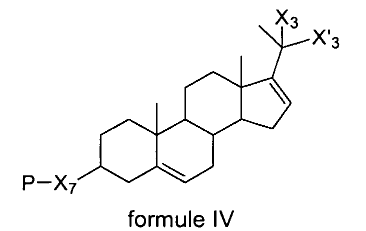 dans lequel X₃ fait partie, conjointement à X'₃, d'un groupe fonctionnel oxo, ou dans lequel X₃ et X'₃ sont indépendamment choisis dans le groupe comprenant un atome d'hydrogène, un groupe hydroxyle, un atome de soufre, les groupes oxyalkyle, oxycarbonyle, alkyle, Het¹alkyle, alkyloxycarbonyle, alcényle, alcynyle, aminoalkyle, aminoacyle, alkylcarbonylamino, alkylthiocarbonylamino, Het¹, facultativement substitués par un ou plusieurs substituants indépendamment choisis dans le groupe comprenant les groupes alkyle, aralkyle, aryle, Het¹, Het², cycloalkyle, alkyloxy, alkyloxycarbonyle, carboxyle, aminocarbonyle, mono- ou di(alkyl)aminocarbonyle, aminosulfonyle, alkylS(=O)t, hydroxy, cyano, un atome d'halogène ou un groupe amino mono- ou disubstitué dans lequel les substituants sont choisis dans le groupe comprenant les groupes alkyle, aryle, aralkyle, aryloxy, arylamino, arylthio, aryloxyalkyle, arylaminoalkyle, aralcoxy, alkylthio, alcoxy, aryloxyalcoxy, arylaminoalcoxy, aralkylamino, aryloxyalkylamino, arylaminoalkylamino, arylthioalcoxy, arylthioalkylamino, aralkylthio, aryloxyalkylthio, arylaminoalkylthio, arylthioalkylthio, alkylamino, cycloalkyle et cycloalkylalkyle ;
dans lequel X₇ est choisi dans le groupe comprenant un atome d'hydrogène, un atome d'oxygène, un atome d'halogène, les groupes oxo, carbonyle, thiocarbonyle, hydroxyle, alkyle, aryle, Het¹, Het¹alkyle, Het¹aryle, alcényle, alcynyle, hydroxyalkyle, hydroxycarbonyle, hydroxycarbonylalkyle, hydroxycarbonylaryle, hydroxycarbonyloxyalkyle facultativement substitués par un ou plusieurs substituants indépendamment choisis dans le groupe comprenant les groupes alkyle, aralkyle, aryle, Het¹, Het², cycloalkyle, alkyloxy, alkyloxycarbonyle, carboxyle, aminocarbonyle, mono- ou di(alkyl)aminocarbonyle, aminosulfonyle, alkylS(=O)t, hydroxy, cyano, un atome d'halogène ou un groupe amino mono- ou disubstitué dans lequel les substituants sont choisis dans le groupe comprenant les groupes alkyle, aryle, aralkyle, aryloxy, arylamino, arylthio, aryloxyalkyle, arylaminoalkyle, aralcoxy, alkylthio, alcoxy, aryloxyalcoxy, arylaminoalcoxy, aralkylamino, aryloxyalkylamino, arylaminoalkylamino, arylthioalcoxy, arylthioalkylamino, aralkylthio, aryloxyalkylthio, arylaminoalkylthio, arylthioalkylthio, alkylamino, cycloalkyle et cycloalkylalkyle ; et dans lequel P est un groupe de protection,
b) réaliser la réaction entre le composé de l'étape a) et un composé organométallique de formule structure V 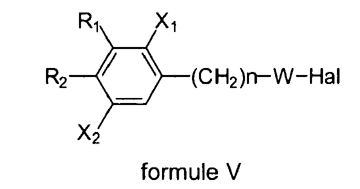 dans lequel X₁, X₂, R₁, R₂ et n sont indépendamment choisis dans le groupe tel qu'indiqué dans l'une quelconque des revendications 1 à 10, dans lequel W est un métal ou une combinaison de métaux et dans lequel Hal est un atome d'halogène,
pour résulter en un intermédiaire de formule structurelle III' 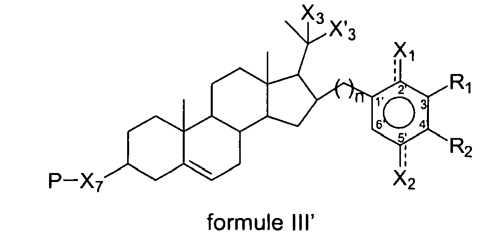 dans lequel X₁, X₂, R₁, R₂ et n sont indépendamment choisis dans le groupe tel qu'indiqué dans l'une quelconque des revendications 1 à 10, dans lequel X₃, X'₃, X₇ sont indépendamment choisis dans le groupe tel qu'indiqué dans l'étape a) et dans lequel P est un groupe de protection,
c) réaliser la réaction entre le composé de l'étape b) et un composé organométallique de formule structure VI
Hal-W-X'₃ formule VI
dans lequel X'₃ est choisi dans le groupe tel qu'indiqué dans l'étape a), dans lequel W est un métal ou une combinaison de métaux, et dans lequel Hal est un atome d'halogène,
pour résulter en un intermédiaire de formule structurelle III 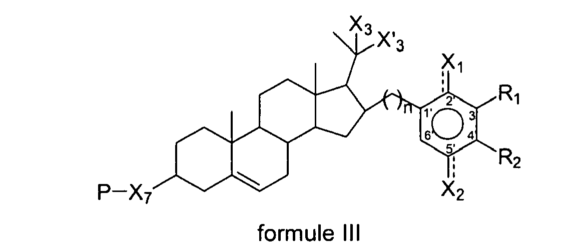 dans lequel X₁, X₂, R_{1'} R₂ et n sont indépendamment choisis dans le groupe tel qu'indiqué dans l'une quelconque des revendications 1 à 10, dans lequel X₃, X'₃, X₇ sont indépendamment choisis dans le groupe tel qu'indiqué dans l'étape a), dans lequel P est un groupe de protection,
d) protéger le groupe X₇ du composé obtenu dans l'étape c) pour former un composé de formule structurelle II, 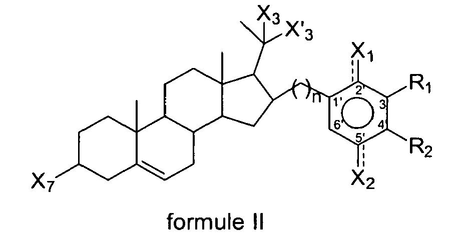 dans lequel X₁, X₂, R_{1'} R₂ et n sont indépendamment choisis dans le groupe tel qu'indiqué dans l'une quelconque des revendications 1 à 10, dans lequel X₃, X'₃, X₇ sont indépendamment choisis dans le groupe tel qu'indiqué dans l'étape a), et
e) coupler un groupe glycosyle O-protégé ou un groupe glycosyle non protégé pour former un composé de formule IIB dans lequel X₁, X₂, R₁, R₂ et n sont indépendamment choisis dans le groupe tel qu'indiqué dans l'une quelconque des revendications 1 à 10, dans lequel X₃ et X'₃ sont indépendamment choisis dans le groupe tel qu'indiqué dans l'étape a), et X₇ est un groupe glycosyle O-protégé ou un groupe glycosyle non protégé, et
f) protéger les groupes O-protégés du groupe glycosyle pour former un composé de formule IB dans lequel X₁, X₂, X₄, X₅, X₆, R₁, R₂ et n sont indépendamment choisis dans le groupe tel qu'indiqué dans l'une quelconque des revendications 1 à 10, dans lequel X₃, X'₃ sont indépendamment choisis dans le groupe tel qu'indiqué dans l'étape a), et X₇ est choisi dans le groupe comprenant un groupe glycosyle, les dérivés thio de celui-ci, les dérivés amino de celui-ci, les dérivés amido de celui-ci, les dérivés protégés par un groupe hydroxyle de celui-ci.

13. Procédé selon la revendication 11, dans lequel l'étape e) consiste à faire réagir le composé de l'étape d) avec un réactif d'oxydation pour former un intermédiaire et à réduire ledit intermédiaire avec un agent de réduction pour résulter en un autre intermédiaire de formule structurelle I, dans lequel X₁, X₂, R₁, R₂ et n sont indépendamment choisis dans le groupe tel qu'indiqué dans l'une quelconque des revendications 1 à 10, et X₃ ou X'₃, et X₄ et X₇ sont des groupes hydroxyles et à poursuivre la réaction avec les étapes e) et f) selon la revendication 11 pour former un composé stéroïde glycosylé de formule structurelle 1.

14. Procédé selon la revendication 11, dans lequel l'étape c) consiste à faire réagir le composé de l'étape b) avec un groupe glycosyle O-protégé ou un groupe glycosyle non protégé pour résulter en un intermédiaire de formule structurelle III, dans lequel X₁, X₂, R₁, R₂ et n sont indépendamment choisis dans le groupe tel qu'indiqué dans l'une quelconque des revendications 1 à 10, dans lequel X₃, X₇ sont indépendamment choisis dans le groupe tel qu'indiqué dans l'étape a) de la revendication 11, et dans lequel P est un groupe de protection, et dans lequel X₃ ou X'₃ est un groupe glycosyl O-protégé ou un groupe glycosyl non protégé et à poursuivre la réaction avec les étapes d), e) et f) selon la revendication 11 pour former un composé stéroïde glycosylé de formule structurelle I.

15. Composé de formule structurelle :

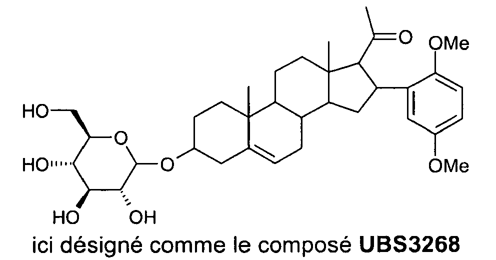

16. Composé de formule structurelle :

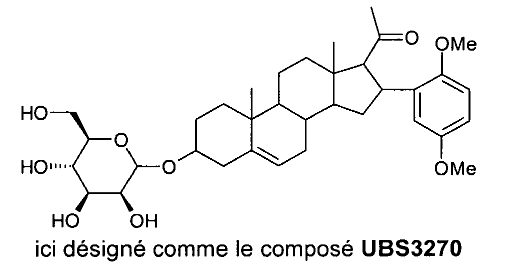

17. Composé de formule structurelle :

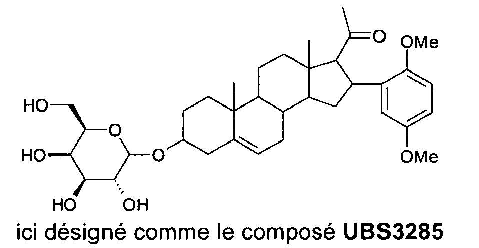

18. Composé de formule structurelle :

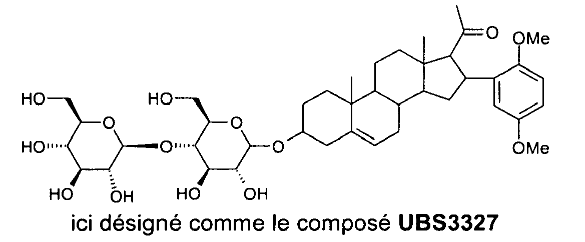

19. Composé de formule structurelle :

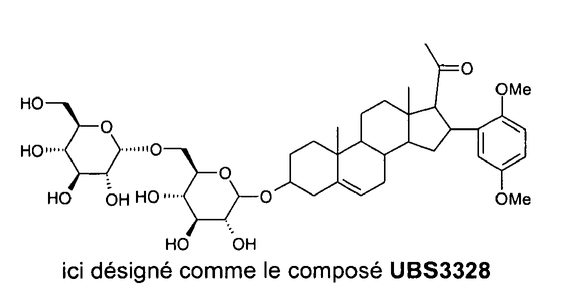

20. Composé de formule structurelle :

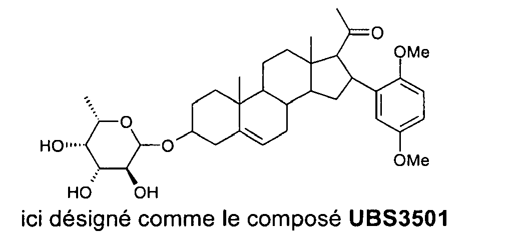

21. Composé de formule structurelle :

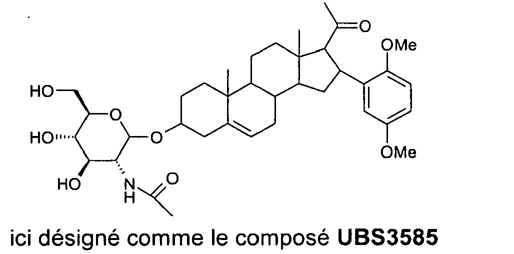

22. Composé de formule structurelle :

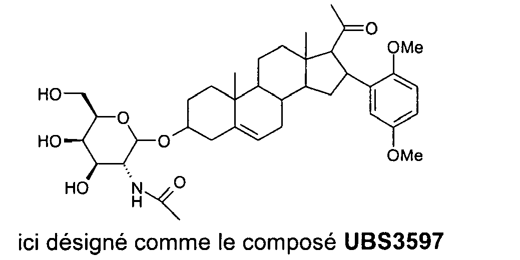

23. Composé de formule structurelle :

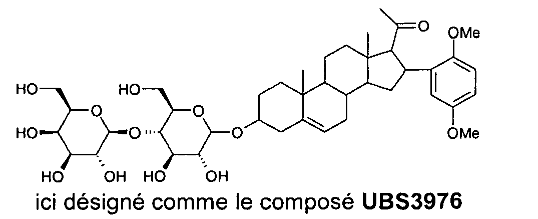

24. Composé de formule structurelle :

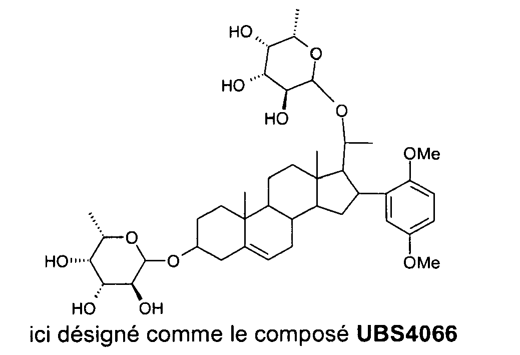

25. Composé de formule structurelle :

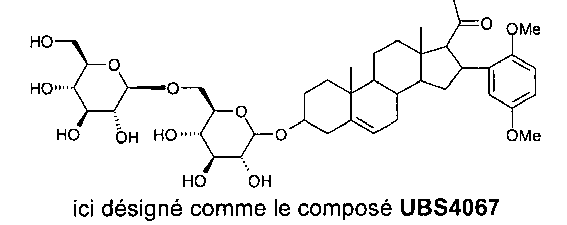

26. Composé de formule structurelle :

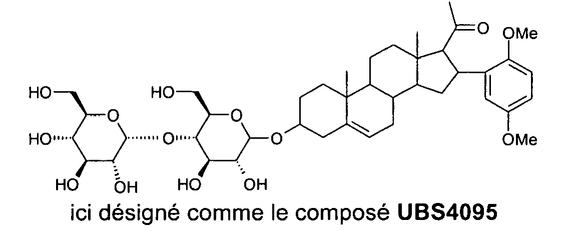

27. Composé de formule structurelle :

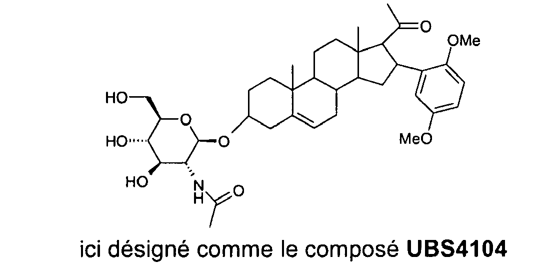

28. Composé de formule structurelle :

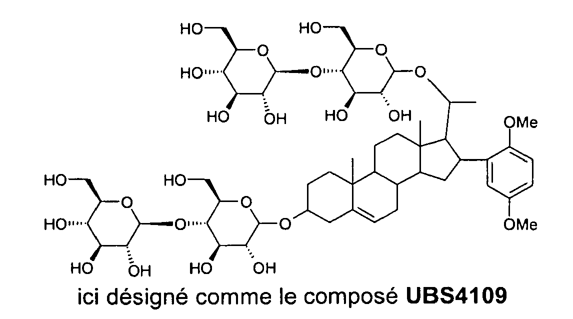

29. Composé de formule structurelle :

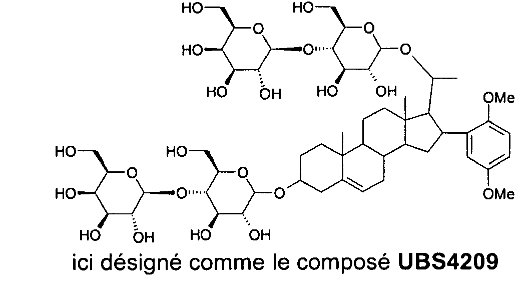

30. Composé de formule structurelle :

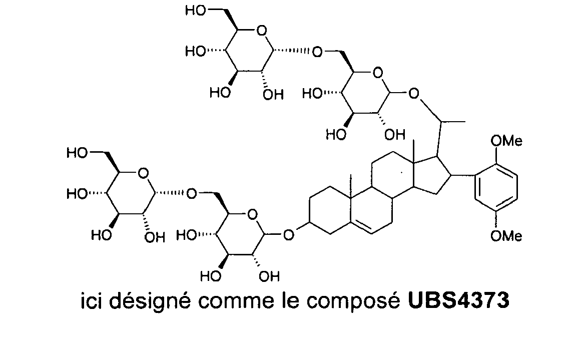

31. Composé selon l'une quelconque des revendications 1 à 10 et 14 à 29, destiné à être utilisé en tant qu'un médicament.

32. Composé selon l'une quelconque des revendications 1 à 10 et 14 à 29, destiné à être utilisé en tant qu'un agent anti-migratoire.

33. Utilisation d'un composé selon l'une quelconque des revendications 1 à 10 et 14 à 29, pour la préparation d'un médicament destiné au traitement du cancer.

34. Composition pharmaceutique comprenant un excipient pharmaceutiquement acceptable et une quantité thérapeutiquement efficace d'un composé selon l'une quelconque des revendications 1 à 10 et 14 to 29.

35. Utilisation d'une composition pharmaceutique selon la revendication 33, pour la préparation d'un médicament destiné au traitement du cancer.
